# EUROPEAN PATENT APPLICATION

(11) **EP 4 666 864 A1**
(43) Date of publication of application: **24.12.2025**
(21) Application number: 24756983.3
(22) Date of filing: 15.02.2024
(51) Int. Cl.: A23J 3/34, A23J 3/22, A23L 5/00, A23L 13/00, A23L 13/74, C12N 9/16

(54) **METHOD FOR PRODUCING MODIFIED PROTEIN-CONTAINING FOOD**

(30) Priority: 15.02.2023 JP 2023022040
(71) Applicant: AJINOMOTO CO., INC., Chuo-ku Tokyo 104-8315 (JP)
(72) Inventor: ABE, Takaaki, Kawasaki-shi, Kanagawa 210-8681 (JP); HORI, Tetsuo, Kawasaki-shi, Kanagawa 210-8681 (JP); ONUKI, Takuya, Kawasaki-shi, Kanagawa 210-8681 (JP); SOGA FUJIMURA, Yuko, Kawasaki-shi, Kanagawa 210-8681 (JP); MIYAZAKI, Natsuki, Kawasaki-shi, Kanagawa 210-8681 (JP); KANEKO, Tomoko, Kawasaki-shi, Kanagawa 210-8681 (JP)
(74) Representative: Strehl & Partner mbB
(86) International application number: PCT/JP2024/005400
(87) International publication number: WO 2024/172143

(57) **Abstract**

The present invention aims to provide a method for producing a protein-containing food in which an unpleasant texture is improved, and the like.

A method for producing a modified protein-containing food, including treating a food ingredient containing a protein with phospholipase D. An enzyme preparation for modifying a protein-containing food, which preparation contains phospholipase D. A pickling liquid for meat processing, which contains phospholipase D, for modifying a protein in a processed meat food. A method for modifying a protein-containing food, including treating a food ingredient containing a protein with phospholipase D.

## Description

### [Technical Field]

The present invention relates to a method for producing a modified protein-containing food, an enzyme preparation for modifying a protein-containing food, a pickling liquid for processing meat to modify the protein in processed meat products, and a method for modifying a protein-containing food.

### [Background Art]

Conventionally, various protein ingredients such as soy protein and milk protein have been widely used in various processed foods such as processed meat product, processed seafood product, and the like, in order to reduce costs, add high values, and improve texture. In particular, due to the recent uncertainty about the supply of raw materials and the soaring prices of raw materials, companies have a strong demand for cost reduction, and studies are underway to maintain quality while reducing costs by adding inexpensive proteins. In addition, the demand for high-protein products is increasing due to the recent health boom, and products containing relatively large amounts of protein are desired. However, the addition of protein ingredients results in unpleasant textures such as "roughness" and "grittiness", thus causing problems.

Therefore, a technique to improve the unpleasant texture derived from proteins in foods has been desired.

Patent Literature 1 discloses a method for producing a meat taste only product without the addition of egg white-derived ingredients, which is characterized by using transglutaminase and carrageenan.

Patent Literature 2 discloses a texture-improving composition containing swelling-suppressed starch and wheat protein, with the aim of providing a texture-improving composition that can impart a good egg white-like texture when used as a food ingredient.

Patent Literature 3 discloses a method for producing processed livestock product-like foods such as sausages and hams that can be produced using only vegetable ingredients without adding egg white, in which method transglutaminase is used.

None of these documents describe the use of phospholipase D to improve the texture of protein-containing foods.

### [Citation List]

### [Patent Literature]

[Patent Literature 1]
   WO 2007/029867
[Patent Literature 2]
   JP 2016-67336 A
[Patent Literature 3]
   JP 2021-132587 A

### [Summary of Invention]

### [Technical Problem]

The object of the present invention is to provide a method for producing a protein-containing food (particularly a food containing a relatively large amount of protein ingredients) improved in an unpleasant texture, and the like.

### [Solution to Problem]

The present inventors have conducted intensive studies in an attempt to solve the above-mentioned problems and found that a smooth texture can be imparted without imparting an unpleasant texture such as "roughness" or "grittiness" derived from protein, by adding a protein ingredient and phospholipase D (sometimes referred to as "PLD" in the present specification) in the production steps of processed foods and the like. In addition, they have found that by using enzymes that contribute to the formation of a cross-linked structure, such as transglutaminase (sometimes referred to as "TG" in the present specification), ascorbic acid oxidase (sometimes referred to as "ASO" in the present specification), glucose oxidase (sometimes referred to as "GO" in the present specification), and the like, in combination with PLD, hardness and elasticity can be imparted without affecting smoothness, which is applicable to various products. Based on the finding, the present inventors conducted further studies and completed the present invention.

That is, the present invention provides the following.
[1] A method for producing a modified protein-containing food, comprising treating a food ingredient containing a protein with phospholipase D.
[2] The method of the above-mentioned [1], wherein the aforementioned food ingredients comprises at least one selected from the group consisting of the following (A) to (I) :
   (A) alkali salt
   (B) calcium salt or calcium oxide
   (C) magnesium salt or magnesium oxide
   (D) reducing agent
   (E) metal ion
   (F) non-polar amino acid or non-polar amino acid salt
   (G) uncharged amino acid or uncharged amino acid salt
   (H) basic amino acid or basic amino acid salt
   (I) acidic amino acid or acidic amino acid salt.
[3] The method of the above-mentioned [2], wherein the (A) alkali salt is at least one selected from the group consisting of sodium carbonate, trisodium phosphate, tripotassium phosphate, and trisodium citrate.
[4] The method of the above-mentioned [2], wherein the (B) calcium salt or calcium oxide is at least one selected from the group consisting of calcium chloride, calcinated shell calcium, calcium lactate, and calcium carbonate.
[5] The method of the above-mentioned [2], wherein the (C) magnesium salt or magnesium oxide is at least one selected from the group consisting of magnesium chloride and magnesium glutamate.
[6] The method of the above-mentioned [2], wherein the (D) reducing agent is at least one selected from the group consisting of a glutathione-containing yeast extract and a cysteine-containing yeast extract.
[7] The method of the above-mentioned [2], wherein the (E) metal ion is at least one selected from the group consisting of an iron-containing yeast, a copper-containing yeast, and a manganese-containing yeast.
[8] The method of the above-mentioned [2], wherein the (F) non-polar amino acid or non-polar amino acid salt is at least one selected from the group consisting of glycine, cystine, alanine, valine, leucine, isoleucine, phenylalanine, proline, and methionine.
[9] The method of the above-mentioned [2], wherein the (G) uncharged amino acid or uncharged amino acid salt is at least one selected from the group consisting of threonine, serine, glutamine, tyrosine, cysteine, and cysteine hydrochloride.
[10] The method of the above-mentioned [2], wherein the (H) basic amino acid or basic amino acid salt is at least one selected from the group consisting of arginine, histidine, and lysine hydrochloride.
[11] The method of the above-mentioned [2], wherein the (I) acidic amino acid or acidic amino acid salt is at least one selected from the group consisting of sodium aspartate and sodium glutamate.
[12] The production method of any of the above-mentioned [1] to [11], further comprising treating with an enzyme that contributes to the formation of a cross-linked structure.
[12-1] The production method of the above-mentioned [12], wherein the enzyme that contributes to the formation of a cross-linked structure is transglutaminase.
[12-2] The production method of the above-mentioned [12], wherein the enzyme that contributes to the formation of a cross-linked structure is ascorbic acid oxidase, and the aforementioned food ingredient comprises L-ASCORBIC ACID.
[12-3] The production method of the above-mentioned [12], wherein the enzyme that contributes to the formation of a cross-linked structure is glucose oxidase, and the aforementioned food ingredient comprises glucose.
[13] The production method of any of the above-mentioned [1] to [12], wherein the protein-containing food is selected from the group consisting of a processed meat food, a processed rice food, a processed soybean food, a processed wheat food, a processed egg food, a processed milk food, a processed seafood, and a plant-based food.
[14] An enzyme preparation for modifying a protein-containing food, which preparation comprises phospholipase D.
[15] The enzyme preparation of the above-mentioned [14], further comprising an enzyme that contributes to the formation of a cross-linked structure.
[15-1] The enzyme preparation of the above-mentioned [15], wherein the enzyme that contributes to the formation of a cross-linked structure is transglutaminase.
[15-2] The enzyme preparation of the above-mentioned [15], wherein the enzyme that contributes to the formation of a cross-linked structure is ascorbic acid oxidase, and the aforementioned food comprises L-ASCORBIC ACID.
[15-3] The enzyme preparation of the above-mentioned [15], wherein the enzyme that contributes to the formation of a cross-linked structure is glucose oxidase, and the aforementioned food comprises glucose.
[16] The enzyme preparation of the above-mentioned [14] or [15], wherein the protein-containing food is selected from the group consisting of a processed meat food, a processed rice food, a processed soybean food, a processed wheat food, a processed egg food, a processed milk food, a processed seafood, and a plant-based food.
[17] A pickling liquid for meat processing, which comprises phospholipase D, for modifying a protein in a processed meat food.
[18] The pickling liquid of the above-mentioned [17], further comprising an enzyme that contributes to the formation of a cross-linked structure.
[18-1] The pickling liquid of the above-mentioned [18], wherein the enzyme that contributes to the formation of a cross-linked structure is transglutaminase.
[18-2] The pickling liquid of the above-mentioned [18], wherein the enzyme that contributes to the formation of a cross-linked structure is ascorbic acid oxidase, and the aforementioned food comprises L-ASCORBIC ACID.
[18-3] The pickling liquid of the above-mentioned [18], wherein the enzyme that contributes to the formation of a cross-linked structure is glucose oxidase, and the aforementioned food comprises glucose.
[19] A method for modifying a protein-containing food, comprising treating a food ingredient containing a protein with phospholipase D.
[20] The modification method of the above-mentioned [19], further comprising treating with an enzyme that contributes to the formation of a cross-linked structure.
[20-1] The modification method of the above-mentioned [20], wherein the enzyme that contributes to the formation of a cross-linked structure is transglutaminase.
[20-2] The modification method of the above-mentioned [20], wherein the enzyme that contributes to the formation of a cross-linked structure is ascorbic acid oxidase, and the aforementioned food ingredient comprises L-ASCORBIC ACID.
[20-3] The modification method of the above-mentioned [20], wherein the enzyme that contributes to the formation of a cross-linked structure is glucose oxidase, and the aforementioned food ingredient comprises glucose.
[21] The modification method of the above-mentioned [19] or [20], wherein the protein-containing food is selected from the group consisting of a processed meat food, a processed rice food, a processed soybean food, a processed wheat food, a processed egg food, a processed milk food, a processed seafood, and a plant-based food.

### [Advantageous Effects of Invention]

According to the present invention, a protein-containing food, in which the protein-derived unpleasant texture is improved, can be provided.

According to the present invention, a food in which the protein-derived unpleasant texture is suppressed can be provided, even when a relatively large amount of protein ingredient is added.

The present invention is applicable to a wide range of protein-containing foods, such as processed meat products, processed seafood products, plant-based foods, and the like.

### [Brief Description of Drawings]

[Fig. 1]

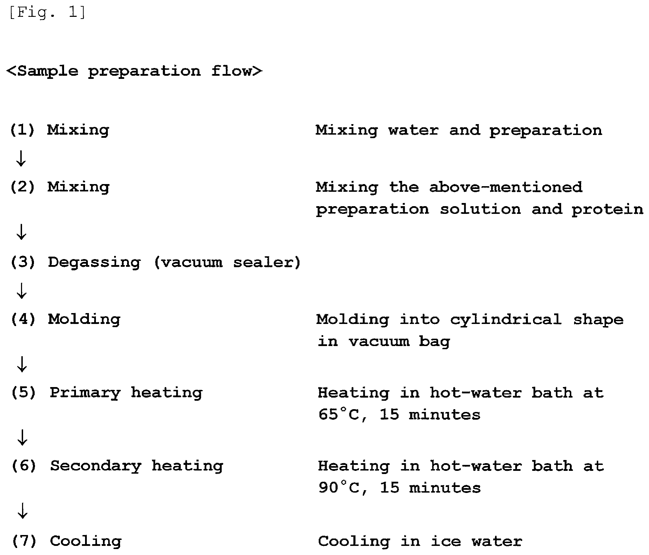

   Fig. 1 shows the sample preparation flow in Experimental Example 1.
[Fig. 2]

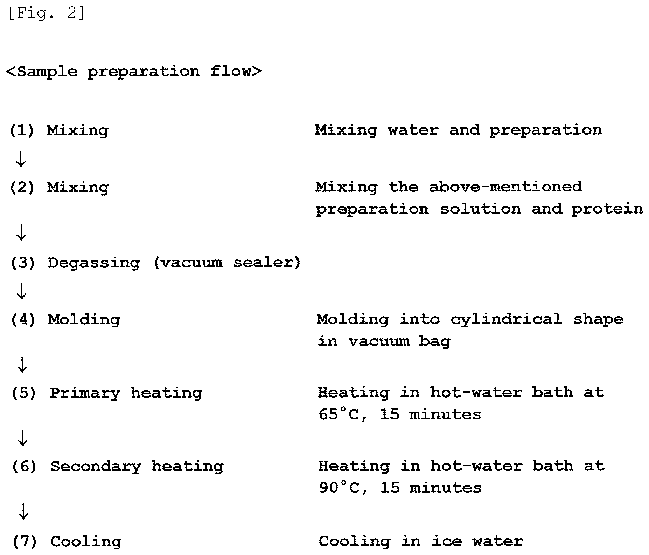

   Fig. 2 shows the sample preparation flow in Experimental Example 2.
[Fig. 3]

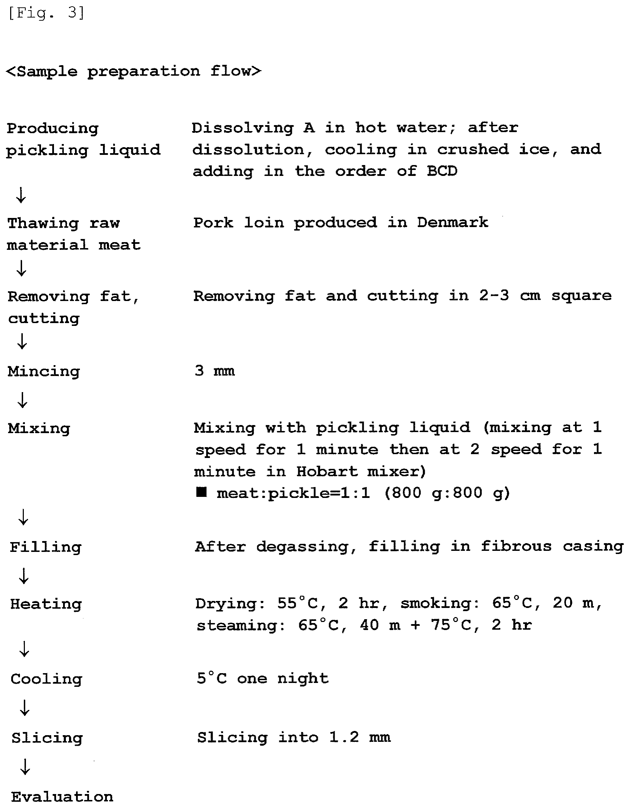

   Fig. 3 shows the sample preparation flow in Experimental Example 3.
[Fig. 4]

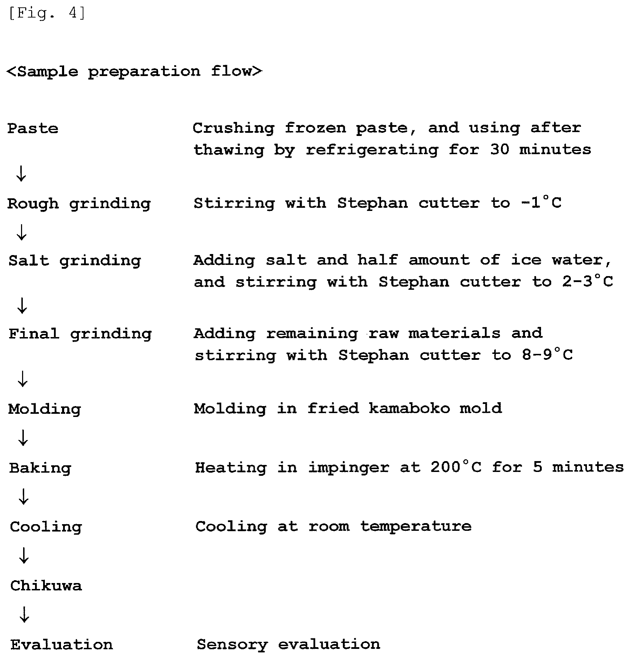

   Fig. 4 shows the sample preparation flow in Experimental Example 4.
[Fig. 5]

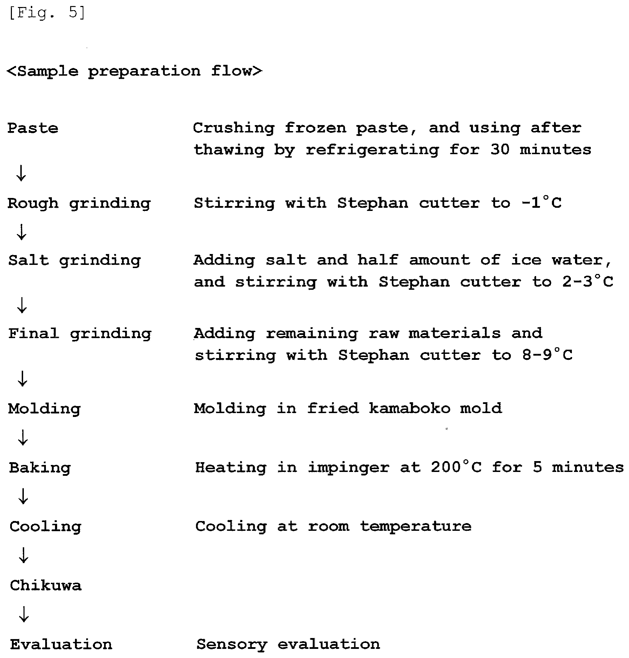

   Fig. 5 shows the sample preparation flow in Experimental Example 5.
[Fig. 6]

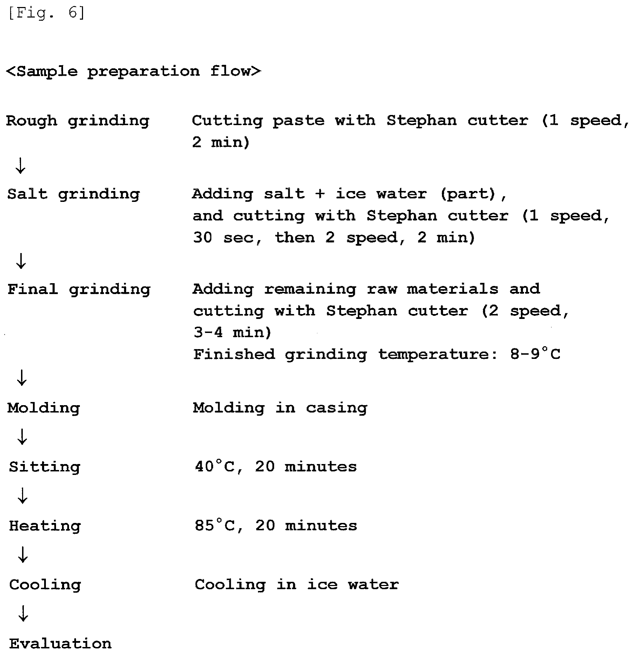

   Fig. 6 shows the sample preparation flow in Experimental Example 6.
[Fig. 7]

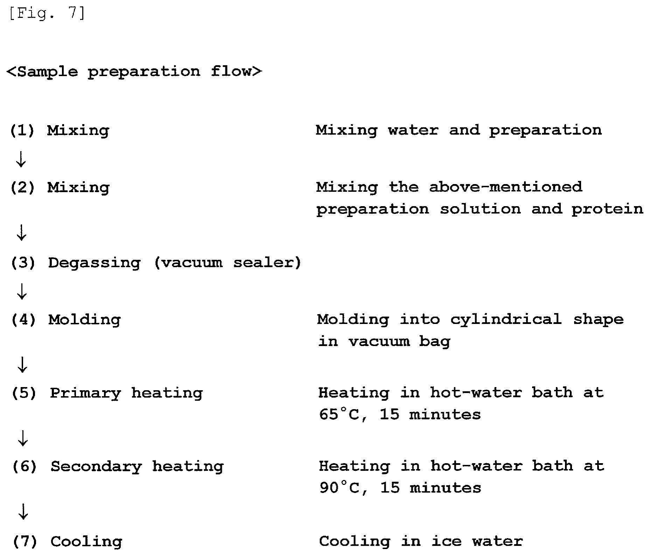

   Fig. 7 shows the sample preparation flow in Experimental Example 7.
[Fig. 8]

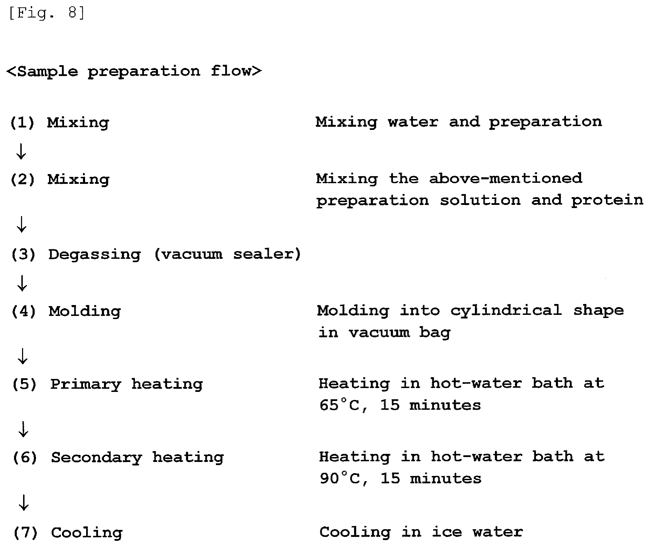

   Fig. 8 shows the sample preparation flow in Experimental Example 8.
[Fig. 9]

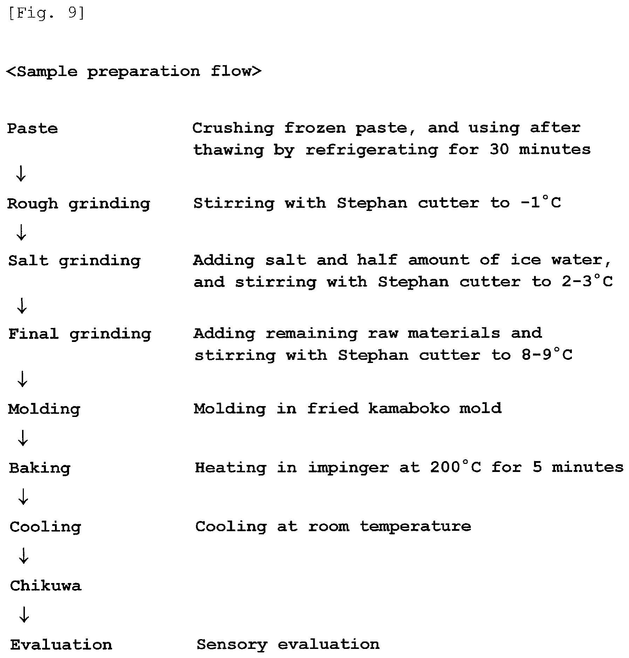

   Fig. 9 shows the sample preparation flow in Experimental Example 9.
[Fig. 10]

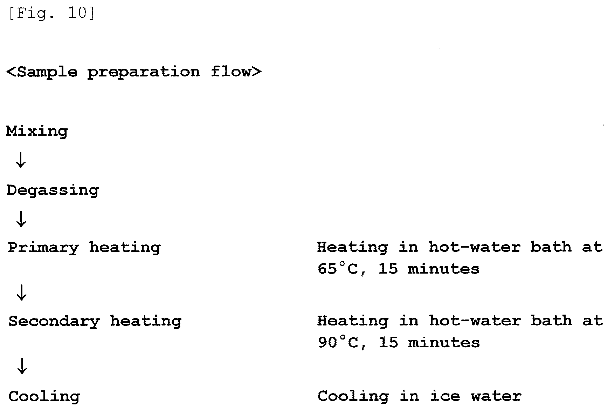

   Fig. 10 shows the sample preparation flows in Experimental Examples 12, 13, 14, 15, 16, and 19.
[Fig. 11]

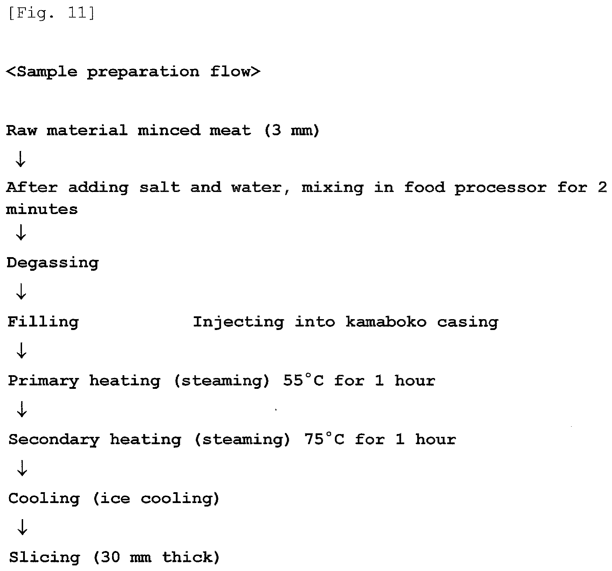

   Fig. 11 shows the sample preparation flow in Experimental Example 17.
[Fig. 12]

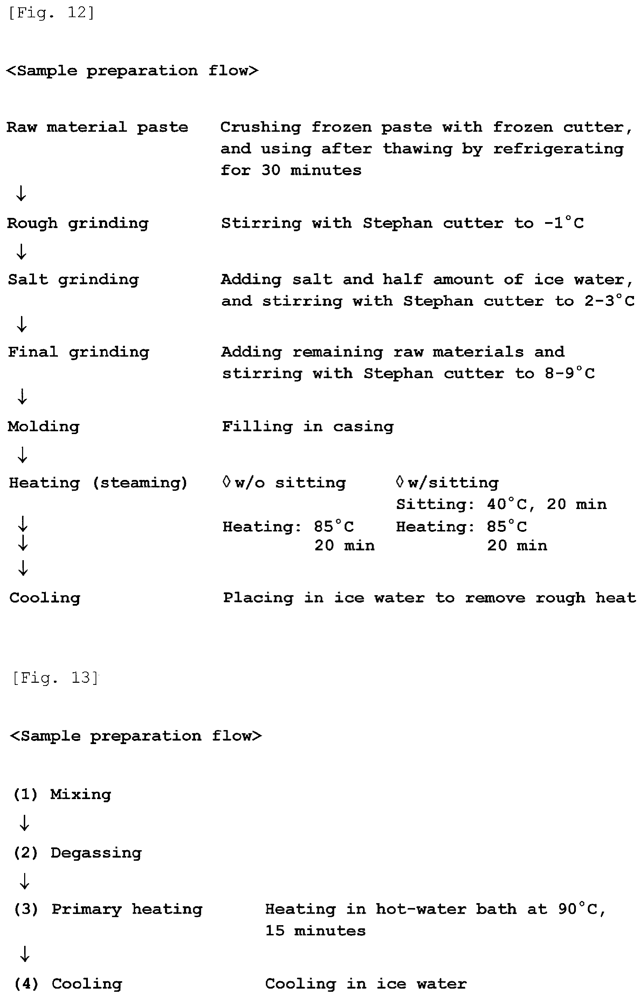

   Fig. 12 shows the sample preparation flow in Experimental Example 18.
[Fig. 13]
   Fig. 13 shows the sample preparation flow in Experimental Example 20.
[Fig. 14]

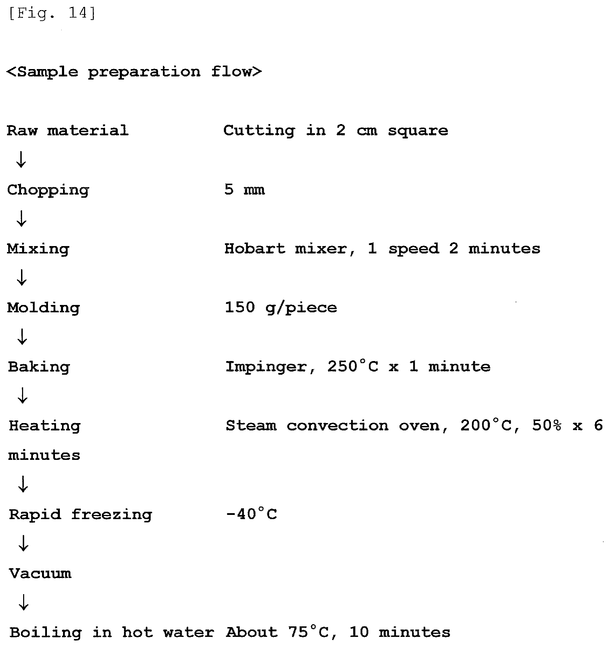

   Fig. 14 shows the sample preparation flow in Experimental Example 21, 22.
[Fig. 15]

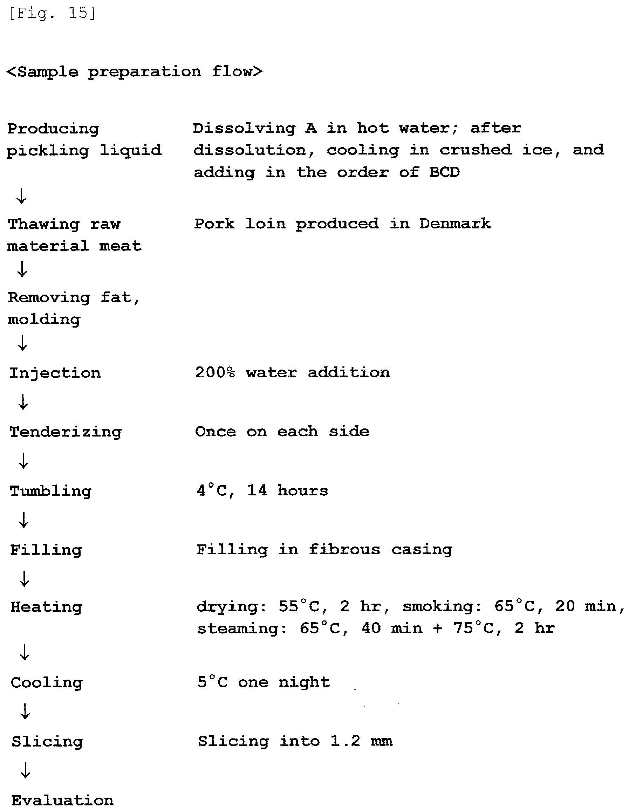

   Fig. 15 shows the sample preparation flow in Experimental Example 23.
[Fig. 16]

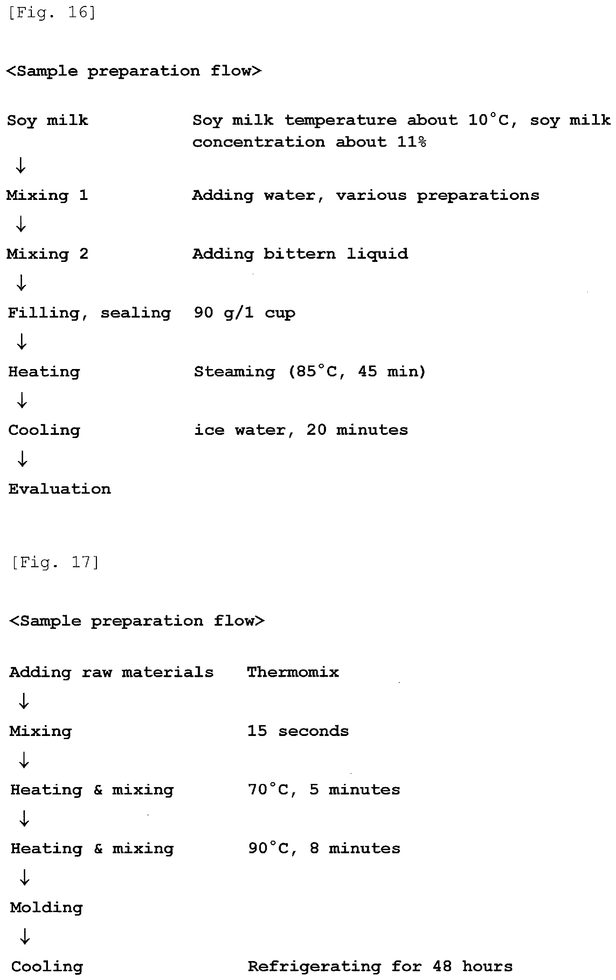

   Fig. 16 shows the sample preparation flow in Experimental Example 24.
[Fig. 17]
   Fig. 17 shows the sample preparation flow in Experimental Example 25.
[Fig. 18]

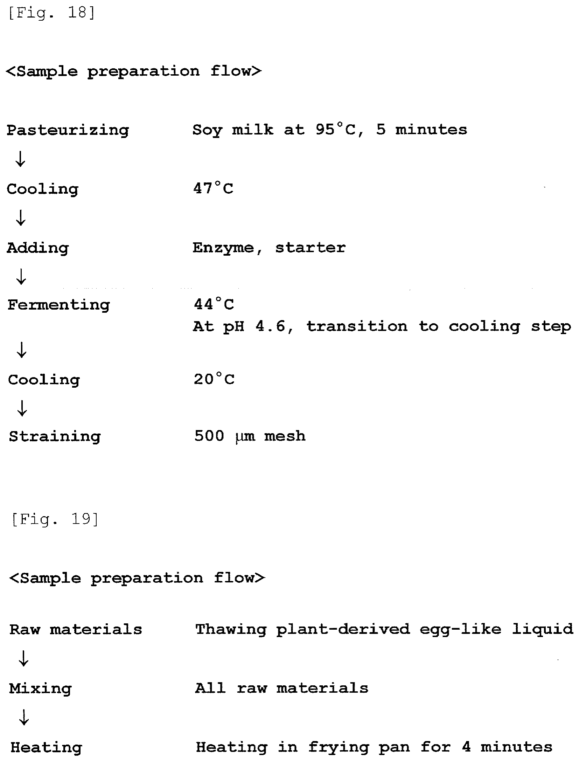

   Fig. 18 shows the sample preparation flow in Experimental Example 26.
[Fig. 19]
   Fig. 19 shows the sample preparation flow in Experimental Example 27.
[Fig. 20]

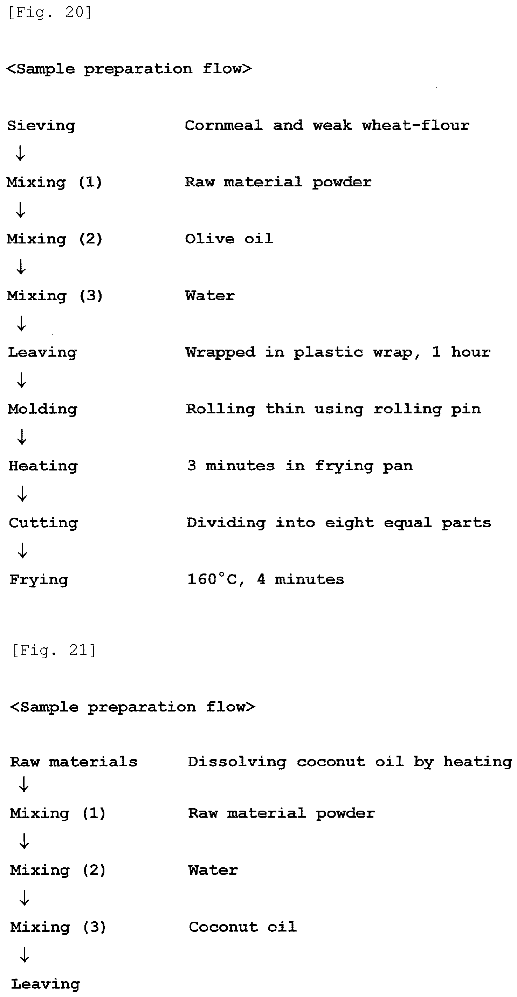

   Fig. 20 shows the sample preparation flow in Experimental Example 28.
[Fig. 21]
   Fig. 21 shows the sample preparation flow in Experimental Example 29.
[Fig. 22-1]

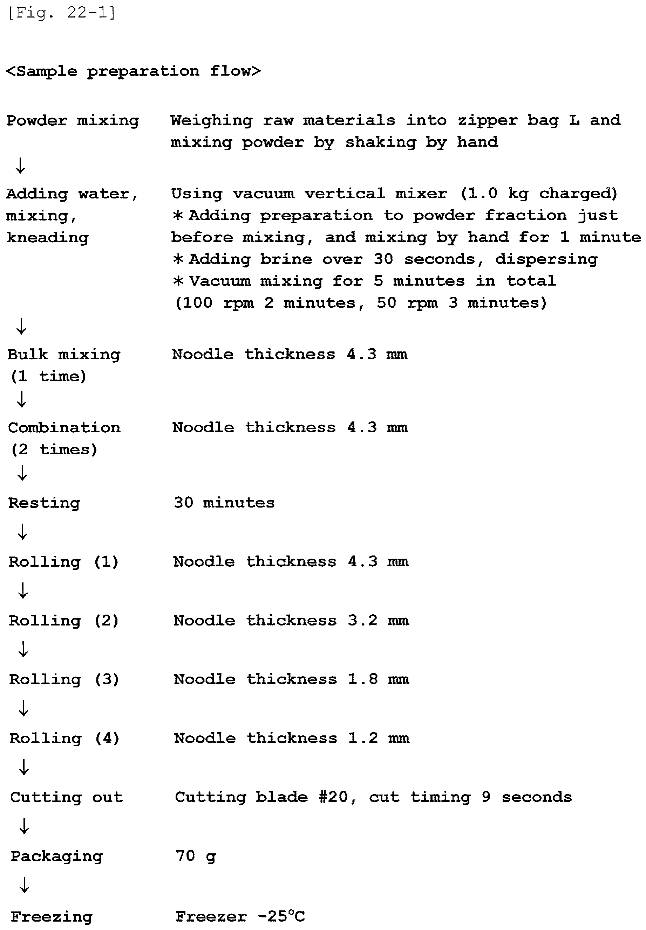

   Fig. 22-1 shows the sample preparation flow in Experimental Examples 30, 31, 32.
[Fig. 22-2]

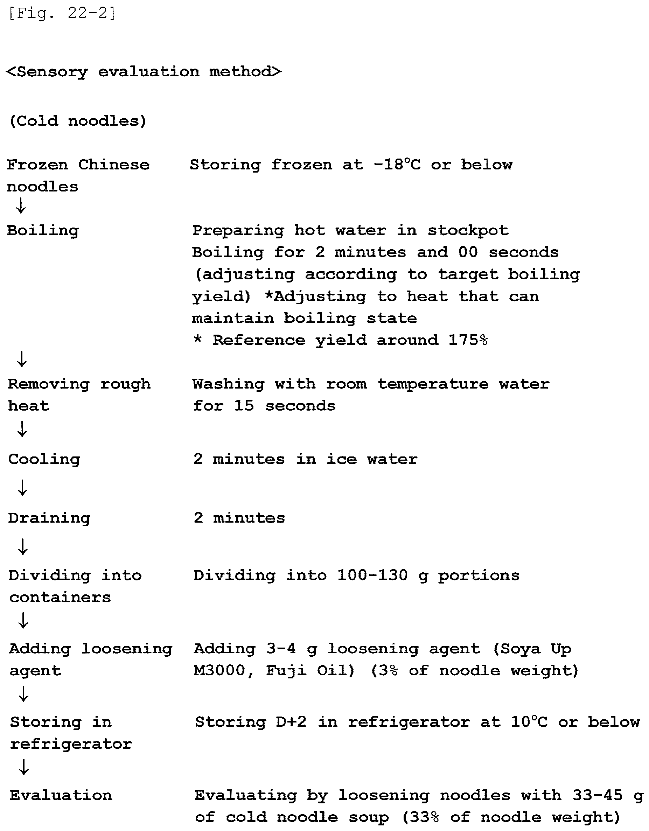

   Fig. 22-2 shows the sensory evaluation methods in Experimental Examples 30, 31, 32.

### [Description of Embodiments]

### 1. Production method of modified protein-containing food

The present invention relates to a method for producing a modified protein-containing food.

The production method of the modified protein-containing food of the present invention (hereinafter also to be simply referred to as the production method of the present invention) includes treating a food ingredient containing a protein with phospholipase D.

In the present invention, the protein-containing foods include processed foods produced from food ingredients containing protein (hereinafter also to be simply referred to as "food ingredients"). Examples of the food ingredient containing protein include meats such as beef, pork, and chicken; fish such as Alaska pollock, hairtail, and threadfin bream; seafood (marine products) such as shellfish, shrimp, crab, octopus, and squid; grains such as rice and wheat; milk, egg, and proteins derived from plants or animals (for example, vegetable proteins such as soy protein, wheat protein, oat protein, pea protein, broad bean protein, mung bean protein, rice protein, chickpea protein, rapeseed protein, corn powder, Navy bean powder, almond protein, peanut powder, spirulina, soy milk, oat milk, and coconut milk; animal proteins such as egg white, egg white (powder), milk protein, skim milk powder, whey powder, casein or a salt thereof (for example, casein Na), cricket powder (Cricket, Big Cricket Protein), and Silkworm Powder); and the like.

Specific examples of the protein-containing food include processed meat foods such as ham, sausage, hamburger steak, and fried chicken; processed egg foods such as omelet; processed rice foods such as cooked rice, rice flour bread, and rice vermicelli; processed soybean foods such as tofu; processed wheat foods such as bread, noodles (e.g., Chinese noodles, Japanese wheat noodles), sweets (snacks), gyoza (dumplings) and burrito (dumplings); processed egg foods such as mayonnaise; processed dairy foods such as ice cream, yogurt, and cheese; processed seafoods such as chikuwa (tube-shaped fish sausage) and kamaboko (fish cake); plant-based foods in which the animal-derived protein of the above-mentioned foods is replaced with plant-derived protein (plant-based (PB) cheese (also called cheese analog), plant-based yogurt, plant-based eggs, plant-based snacks (formed and bar)); and semi-solid liquid diets of the above-mentioned protein-containing foods.

The "processed seafood" refers to foods made from marine products such as fish, shellfish, shrimp, crab, octopus, and squid. The embodiment of provision of the protein-containing foods is not particularly limited. That is, the protein-containing foods may be provided in any form, such as raw food, heated product, frozen product, aseptically packaged product, retort product, dried product, canned product, and the like.

The present invention is particularly advantageously used for solid or semi-solid protein-containing foods that require not only smoothness but also preferable hardness and elasticity at the time of eating.

In the present invention, a solid food containing protein (protein-containing solid food) refers to a food that contains protein and is in a solid state (in other words, a state with no fluidity, a gelled state, and one that maintains the shape thereof against gravity).

Examples of the protein-containing solid food in the present invention include ham, sausage, hamburger steak, fried chicken, cooked rice, rice flour bread, rice vermicelli, bread, noodles (e.g. Chinese noodles, Japanese wheat noodles), sweets, gyoza (dumpling), burrito (dumpling), cheese, chikuwa, kamaboko, and plant-based foods in which the animal protein in these foods is replaced with plant protein.

In the present invention, a semi-solid food containing protein (protein-containing semi-solid food) refers to a food that contains protein and has the properties of both a liquid and a solid, and in a semi-liquid state that is closer to solid than liquid.

Examples of the protein-containing semi-solid food in the present invention include tofu, mayonnaise, ice cream, yogurt, semi-solid liquid foods, and plant-based foods in which the animal protein of these foods is replaced with plant protein.

In the present invention, even when a vegetable or animal-derived protein (e.g., vegetable protein such as soy protein or wheat protein; animal protein such as egg white, milk protein, casein or a salt thereof (e.g., casein Na), cricket powder, etc.) is contained in an amount of, for example, 0.1 wt% or more of the entire product, a product suppressed in the unpleasant texture derived from protein can be provided.

Phospholipase is an enzyme having the activity of hydrolyzing phospholipids.

In the present specification, the activity unit of phospholipase D is measured and defined as follows.

An enzyme solution (0. 1 mL) is mixed with 0.9 mL of a substrate solution containing phosphatidylcholine, and reacted at 37°C for 30 min. After discontinuation of the reaction, 50 µL of the reaction solution is added to 1 mL of color-developing solution containing choline oxidase, peroxidase, and the like, and reacted for 5 min. After discontinuation of the reaction, the amount of pigment produced from choline is measured. The amount of enzyme that liberates 1 µmol of choline per minute at 37°C using phosphatidylcholine as a substrate is defined as 1 U (unit).

In the present invention, the amount of phospholipase D to be added is preferably 0.000000065 U or more, more preferably 0.00000065 U or more, further preferably 0.000065 or more, in terms of enzyme activity per 1 g of protein.

In the present invention, the amount of phospholipase D to be added is preferably 30000 U or less, more preferably 15000 U or less, and further preferably 6494 or less, in terms of enzyme activity per 1 g of protein.

In the present invention, the amount of phospholipase D to be added is preferably 0.000000065 to 300000 U, more preferably 0.00000065 to 150000 U, further preferably 0.000065 to 6494 U, in terms of enzyme activity per 1 g of protein.

In addition, in the present invention, the amount of phospholipase D to be added is preferably 0.1 U or more, more preferably 1.2 to 10000.0 U, further preferably 12.0 to 5000.0 U, in terms of enzyme activity per 1 g of protein.

The action time (reaction time) of phospholipase D is not particularly limited as long as the enzyme can act on the phospholipid as a substrate substance. For example, it is 0 min or more, 1 min or more, 3 min or more, 5 min or more, 10 min or more, 20 min or more, or 30 min or more. In addition, for example, it is 168 hr or less, 72 hr or less, 48 hr or less, 24 hr or less, 12 hr or less, 6 hr or less, 3 hr or less, 2 hr or less, or 1 hr or less. A practical action time is preferably 0 to 148 hr, more preferably 30 min to 148 hr. In addition, the action temperature (reaction temperature) is also not particularly limited as long as the enzyme maintains its activity. A action at 0 to 60°C is practically preferred. The enzyme reaction can be terminated by, for example, heating at 70 to 75°C for 5 to 10 min.

In the production method of the present invention, it is preferable to further add, in addition to the above-mentioned phospholipase D, an enzyme that contributes to the formation of a cross-linked structure to the food ingredients and allow the enzyme to act.

In the present invention, an enzyme that contributes to the formation of a cross-linked structure is an enzyme that acts directly or indirectly on a protein and has the activity of forming a cross-linked structure in the protein.

In the present invention, examples of the enzyme that contributes to the formation of a cross-linked structure include transglutaminase, ascorbic acid oxidase and glucose oxidase.

In the present invention, when ascorbic acid oxidase is used as the enzyme that contributes to the formation of a cross-linked structure, the food material to which the enzyme is added contains L-ASCORBIC ACID to be the substrate. The L-ASCORBIC ACID means ascorbic acid, ascorbate salt, or ascorbic acid with modified skeleton; examples include salts with alkali metal or alkaline earth metal (e.g., sodium ascorbate, calcium ascorbate, etc.), provitamin ascorbic acid 2-glucoside, ascorbic acid esters (e.g., ascorbyl palmitate, ascorbyl stearate, etc.), materials containing a lot of ascorbic acid, and the like. Among these, ascorbic acid and sodium ascorbate are preferred. Examples of the food material containing a lot of ascorbic acid include acerola powder and the like.

In the present invention, when ascorbic acid oxidase is used as the enzyme that contributes to the formation of a cross-linked structure, the amount of the L-ASCORBIC ACID in the food material to which the enzyme is added is, for example, 0.000000000001 to 50.0 weight, preferably 0.00000000001 to 30.0 wt%, more preferably 0.0000000001 to 10.0 wt%, further preferably 0.000000001 to 6.0 wt%, per gram of protein to which the enzyme is added.

In the present invention, when ascorbic acid oxidase is used as the enzyme that contributes to the formation of a cross-linked structure, the amount of the L-ASCORBIC ACID in the food material to which the enzyme is added is, for example, 0.1 to 99 wt%, preferably 1 to 95 wt%, more preferably 5 to 90 wt%, further preferably 10 to 80 wt%, calculated as ascorbic acid, relative to the agent of the present invention.

In the present invention, when glucose oxidase is used as the enzyme that contributes to the formation of a cross-linked structure, the food material to which the enzyme is added contains glucose to be the substrate.

In the present invention, when glucose oxidase is used as the enzyme that contributes to the formation of a cross-linked structure, the amount of the glucose in the food material to which the enzyme is added is 0.0000000001 to 10.0 weight, preferably 0.000000001 to 5.0 wt%, more preferably 0.00000001 to 1.0 wt%, further preferably 0.0000001 to 0.1 wt%, per gram of protein to which the enzyme is added.

In the present invention, when ascorbic acid oxidase is used as the enzyme that contributes to the formation of a cross-linked structure, the amount of the glucose in the food material to which the enzyme is added is, for example, 0.1 to 99 wt%, preferably 0.2 to 95 wt%, more preferably 0.5 to 90 wt%, further preferably 1 to 80 wt%, relative to the agent of the present invention.

When multiple enzymes are added, the order of addition may be any, and they may be added all at once or in sequence with a time lag. From the aspect of convenience, they are desirably added all at once.

When an enzyme that contributes to the formation of a cross-linked structure is further allowed to act on the food ingredients, the action time, action temperature, and method of terminating the enzyme reaction are the same as the action time, action temperature, and method of terminating the enzyme reaction for the above-mentioned phospholipase D.

In the production method of the present invention, the enzymes that act on the food ingredients include the following (I) to (VII). In the following, (I) to (VII) are also collectively referred to as "the enzyme in the present invention".
(I) phospholipase D
(II) phospholipase D and transglutaminase
(III) phospholipase D and ascorbic acid oxidase
(IV) phospholipase D, transglutaminase and ascorbic acid oxidase
(V) phospholipase D and glucose oxidase
(VI) phospholipase D, transglutaminase and glucose oxidase
(VII) phospholipase D, transglutaminase, ascorbic acid oxidase and glucose oxidase

The transglutaminase used in the present invention is an enzyme that has the activity of catalyzing an acyl transfer reaction in which a glutamine residue in a protein or peptide is used as a donor and a lysine residue is used as an acceptor. Transglutaminases of various origins are known, for example, one derived from mammal, one derived from fish, one derived from microorganism, and the like. The transglutaminase used in the present invention is not particularly limited in origin as long as it has the aforementioned activity, and transglutaminase of any origin can be used, and a recombinant enzyme may also be used. The transglutaminase used in the present invention may be a commercially available product. As a specific example, microorganism-derived transglutaminase commercially available from Ajinomoto Co., Inc. under the product name "Activa" TG can be used alone or in combination.

In the present specification, the enzyme activity of transglutaminase is determined by reacting transglutaminase in a Tris buffer solution at 37°C, pH 6.0 in a reaction system using benzyloxycarbonyl-L-glutamylglycine and hydroxylamine as substrates, forming an iron complex by using the resulting hydroxamic acid in the presence of trichloroacetic acid, measuring the absorbance at 525 nm, and obtaining the amount of hydroxamic acid using a calibration curve. The amount of enzyme that produces 1 µmol of hydroxamic acid per minute is defined as 1 unit (1 U) (see JP 64-27471 A).

In the production method of the present invention, when transglutaminase is used, the amount of the transglutaminase to be added is, for example, 0.01 to 25.6 U, preferably 0.06 to 12.8 U, more preferably 0.3 to 6.4 U, further preferably 0.6 to 3.2 U, in terms of enzyme activity per 1 g of protein.

In addition, in the production method of the present invention, when transglutaminase is used, the amount of the transglutaminase to be added is, for example, 0.01 to 22.0 U, preferably 0.1 to 11.0 U, more preferably 0.2 to 5.5 U, further preferably 0.5 to 3.5 U, in terms of enzyme activity per 1 g of protein.

The ascorbic acid oxidase (enzyme number EC1.10.3.3) used in the present invention is one of the ascorbic acid and aldaric acid metabolic enzymes, and is an oxidoreductase that catalyzes a chemical reaction that produces dehydroascorbic acid and water, using ascorbic acid and oxygen as substrates. Conventionally, ascorbic acid oxidase derived from Cucurbitaceae plants such as pumpkin, cucumber, and zucchini has been frequently used industrially. The origin of the ascorbic acid oxidase used in the present invention is not particularly limited as long as it has the above-mentioned activity, and may be derived from, for example, plant, microorganism, animal, or the like. In addition, the ascorbic acid oxidase to be used in the present invention may be a recombinant enzyme.

The method for producing the ascorbic acid oxidase to be used in the present invention is not particularly limited, and ascorbic acid oxidase produced by a method known per se or a method analogous thereto may be used. Commercially available ascorbic acid oxidase may also be used.

In the present invention, one type of ascorbic acid oxidase may be used alone, or two or more types may be used in combination.

In the present invention, as the activity unit of ascorbic acid oxidase, the amount of enzyme that oxidizes 1 µmol of ascorbic acid per minute under conditions of 30°C, pH 5.6 is defined as 1 U (unit).

Specifically, in the present invention, the activity of ascorbic acid oxidase is measured by the following procedures (1) to (3).
(1) 1 mL of 0.001 mol/L ascorbic acid solution and 1 mL of 0.01 mol/L disodium hydrogen phosphate are placed in a test tube, and preheated in a constant temperature water tank at 30°C for 5 min. To this mixture (pH 5.6) is added 0.2 mL of the diluted test enzyme solution and the mixture is immediately stirred to allow for reaction. After reacting for exactly 5 min, 6 mL of 0.2 mol/L hydrochloric acid is added and the mixture is immediately stirred to discontinue the reaction. The absorbance (Abs1) of this solution at a wavelength of 245 nm is measured.
(2) As a blank (blind test), 1 mL of 0.001 mol/L ascorbic acid solution and 1 mL of 0.01 mol/L disodium hydrogen phosphate are placed in a test tube and preheated for 5 min in a constant temperature water tank at 30°C. To this mixture (pH 5.6) is added 6 mL of 0.2 mol/L hydrochloric acid and the mixture is immediately stirred. After 5 min, 0.2 mL of the diluted test enzyme solution is added and the mixture is immediately stirred. The absorbance (Abs2) of this solution at a wavelength of 245 nm is measured.
(3) The difference in the absorbances measured in the aforementioned (1) and (2), ΔAbs (=Abs2-Abs1), is determined, and the activity of ascorbic acid oxidase (U/mg) is calculated according to the following formula: Activity of ascorbic acid oxidase (U/mL)=(ΔAbs×8.2×[dilution ratio of test enzyme solution])/(10.0×1.0×5×0.2)
   10.0: millimolar absorption coefficient of ascorbic acid at pH 1.0 (cm²/µmol)
   1.0: optical path length (cm)
   5: reaction time (min)
   8.2: total volume of reaction solution (mL)
   0.2: volume of test enzyme solution (mL)

In the production method of the present invention, when ascorbic acid oxidase is used, the amount of the ascorbic acid oxidase to be added is, for example, 0.00000012 to 12000000000000 U, preferably 0.0000012 to 1200000000000 U, more preferably 0.000012 to 120000000000 U, further preferably 0.00012 to 12000000000 U, in terms of enzyme activity per 1 g of the content of the substrate of the enzyme (calculated as L-ascorbic acid).

In addition, in the production method of the present invention, when ascorbic acid oxidase is used, the amount of the ascorbic acid oxidase to be added is, for example, 0.5 to 500 U, preferably 1 to 350 U, more preferably 3 to 200 U, further preferably 5 to 100 U, in terms of enzyme activity per 1 g of the content of the substrate of the enzyme (calculated as L-ascorbic acid).

The glucose oxidase (EC1.1.3.4) to be used in the present invention is an enzyme that catalyzes a reaction in which glucose and oxygen are used as substrates to produce gluconolactone (gluconolactone is non-enzymatically hydrolyzed to gluconic acid) and hydrogen peroxide. The hydrogen peroxide produced by this reaction oxidizes the SH groups in proteins to promote the production of SS bond (disulfide bond) and form a cross-linked structure in protein. Glucose oxidases of various origins are known, including those derived from microorganisms such as aspergillus oryzae and those derived from plants. Any of those glucose oxidases may be used, and the origin thereof is not limited. It may also be a recombinant enzyme. A specific example of glucose oxidase is the glucose oxidase derived from microorganism which is commercially available under the product name of "Sumizyme PGO" from Shin Nihon Chemical Co., Ltd.

As the activity unit of glucose oxidase in the present invention, the amount of enzyme that oxidizes 1 µmol of glucose per minute at 37°C and pH 7.0 is defined as 1 U (unit).

For the activity of glucose oxidase in the present invention, the following method can be exemplified. Using glucose as a substrate, hydrogen peroxide is produced by the action of glucose oxidase in the presence of oxygen. The produced hydrogen peroxide is reacted with peroxidase in the presence of aminoantipyrine and phenol to produce quinoneimine dye. The produced quinoneimine dye is measured at a wavelength of 500 nm. Specifically, it is as follows. Glucose oxidase is stirred and dissolved in 0.1 mol/L phosphate buffer (adjusted to pH 7.0 with potassium dihydrogen phosphate and sodium hydroxide aqueous solution), and then diluted 50-fold with 0.1 mol/L phosphate buffer to obtain a GO solution. A phenol-containing buffer solution (2.0 mL) (obtained by mixing Milli-Q, 1.36 g of potassium dihydrogen phosphate, 3 mL of 5% phenol test solution, and 3 mL of 5% Triton X-100 solution and adjusted to pH 7.0, 100 mL with sodium hydroxide aqueous solution), 500 µL of 10% glucose solution, 500 µL of 0.01% peroxidase solution (using PO "amano" 3 (1250 U±250 U)), and 100 µL of 0.4% 4-aminoantipyrine solution are added in this order to an analysis cell, mixed by inversion, and retained at 37±0.1°C for 10 min. The GO solution (100 µL) is placed in the above-mentioned analysis cell, 11 points are automatically measured every 30 seconds for 5 min, and the GO activity value is measured from the increment (slope) between 120 seconds and 300 seconds. For the blank plot, the value measured by adding 0.1 mol/L phosphate buffer instead of the GO solution was used and subtracted from the value measured for the GO test plot. For oxidoreductases other than glucose oxidase, the amount of enzyme required to oxidize or reduce 1 µmol of substrate per minute is defined as 1 U (unit).

In the production method of the present invention, when glucose oxidase is used, the amount of the glucose oxidase to be added is, for example, 0.0000000022 to 215000000000 U, preferably 0.000000022 to 21500000000 U, more preferably 0.00000022 to 2150000000 U, further preferably 0.0000022 to 215000000 U, in terms of enzyme activity per 1 g of the substrate of the enzyme (glucose).

In addition, in the production method of the present invention, when glucose oxidase is used, the amount of the glucose oxidase to be added is, for example, 0.01 to 10000 U, preferably 0.1 to 5000 U, more preferably 0.5 to 3000 U, further preferably 1.0 to 2000 U, in terms of enzyme activity per 1 g of the substrate of the enzyme (glucose).

In the production method of the present invention, it is preferable to contain an auxiliary material selected from the following (A) to (N) in the food ingredients to which the enzyme is added. These auxiliary materials may be contained alone or in combination of two or more. In the production method of the present invention, by containing these auxiliary materials in the food ingredients to which the enzyme is added, a further improvement in smoothness (improvement of discomfort) can be expected compared to when they are not contained.
(A) alkali salt (e.g., sodium carbonate, trisodium phosphate, tripotassium phosphate, trisodium citrate),
(B) calcium salt, calcium oxide (e.g., calcium chloride, calcinated shell calcium, calcium lactate, calcium carbonate),
(C) magnesium salt, magnesium oxide (e.g., magnesium chloride, magnesium glutamate),
(D) reducing agent (e.g., glutathione-containing yeast extract, cysteine-containing yeast extract),
(E) metal ion (e.g., iron-containing yeast, copper-containing yeast, manganese-containing yeast),
(F) non-polar amino acid and non-polar amino acid salt (e.g., glycine, cystine, alanine, valine, leucine, isoleucine, phenylalanine, proline, methionine),
(G) uncharged amino acid and uncharged amino acid salt (e.g., threonine, serine, glutamine, tyrosine, cysteine, cysteine hydrochloride),
(H) basic amino acid and basic amino acid salt (e.g., arginine, histidine, lysine hydrochloride),
(I) acidic amino acid and acidic amino acid salt (e.g., sodium aspartate, sodium glutamate).

In the production method of the present invention, when an alkali salt is used as an auxiliary material, the amount of the alkali salt in the food material to which the enzyme is added is, for example, 0.0000000001 to 1.0 wt%, preferably 0.000000001 to 0.1 wt%, more preferably 0.00000001 to 0.06 wt%, further preferably 0.0000001 to 0.01 wt%, per gram of protein.

In the production method of the present invention, when a calcium salt or calcium oxide is used as an auxiliary material, the amount of the calcium salt or calcium oxide in the food material to which the enzyme is added is, for example, 0.0000000001 to 1.0 wt%, preferably 0.000000001 to 0.1 wt%, more preferably 0.00000001 to 0.06 wt%, further preferably 0.0000001 to 0.01 wt%, per gram of protein.

In the production method of the present invention, when a magnesium salt or magnesium oxide is used as an auxiliary material, the amount of the magnesium salt or magnesium oxide in the food material to which the enzyme is added is, for example, 0.0000000001 to 0.1 wt%, preferably 0.000000001 to 0.05 wt%, more preferably 0.00000001 to 0.01 wt%, further preferably 0.0000001 to 0.001 wt%, per gram of protein.

In the production method of the present invention, when a reducing agent is used as an auxiliary material, the amount of the reducing agent in the food material to which the enzyme is added is, for example, 0.000000000001 to 1.0 wt%, preferably 0.00000000001 to 0.5 wt%, more preferably 0.0000000001 to 0.1 wt%, further preferably 0.000000001 to 0.06 wt%, per gram of protein.

In the production method of the present invention, when a metal ion is used as an auxiliary material, the amount of the metal ion in the food material to which the enzyme is added is, for example, 0.0000000001 to 1.0 wt%, preferably 0.000000001 to 0.5 wt%, more preferably 0.00000001 to 0.1 wt%, further preferably 0.0000001 to 0.06 wt%, per gram of protein.

In the production method of the present invention, when a non-polar amino acid or non-polar amino acid salt is used as an auxiliary material, the amount of the non-polar amino acid or non-polar amino acid salt in the food material to which the enzyme is added is, for example, 0.000000000001 to 1.0 wt%, preferably 0.00000000001 to 0.5 wt%, more preferably 0.0000000001 to 0.1 wt%, further preferably 0.000000001 to 0.06 wt%, per gram of protein.

In the production method of the present invention, when an uncharged amino acid or uncharged amino acid salt is used as an auxiliary material, the amount of the uncharged amino acid or uncharged amino acid salt in the food material to which the enzyme is added is, for example, 0.00000000000001 to 1.0 wt%, preferably 0.0000000000001 to 0.1 wt%, more preferably 0.000000000001 to 0.06 wt%, further preferably 0.00000000001 to 0.01 wt%, per gram of protein.

In the production method of the present invention, when a basic amino acid or basic amino acid salt is used as an auxiliary material, the amount of the basic amino acid or basic amino acid salt in the food material to which the enzyme is added is, for example, 0.0000000001 to 0.1 wt%, preferably 0.000000001 to 0.05 wt%, more preferably 0.00000001 to 0.01 wt%, further preferably 0.0000001 to 0.001 wt%, per gram of protein.

In the production method of the present invention, when an acidic amino acid or acidic amino acid salt is used as an auxiliary material, the amount of the acidic amino acid or acidic amino acid salt in the food material to which the enzyme is added is, for example, 0.0000000001 to 0.1 wt%, preferably 0.000000001 to 0.05 wt%, more preferably 0.00000001 to 0.01 wt%, further preferably 0.0000001 to 0.001 wt%, per gram of protein.

The production method of the present invention can produce a protein-containing food by using ingredients and methods similar to those used for general protein-containing foods, except that a treatment with the enzyme in the present invention is performed (when ascorbic acid oxidase or glucose oxidase is used, the ASCORBIC ACID or glucose to be the substrate is added to the ingredients), and/or preferably, the auxiliary materials described above are used. The enzyme in the present invention may be allowed to act on the food ingredients at any stage of the production step of the protein-containing food. It may also be added and allowed to act during the step of producing protein ingredients. The enzyme in the present invention can be allowed to act on the food ingredients either as is, or by preparing an appropriate solution or the like and placing same in coexistence with the food ingredients. For example, the enzyme in the present invention may be added to the food ingredients, or the food ingredients may be immersed in a treatment solution containing the enzyme in the present invention. In the following, such operation to place the enzyme in the present invention in coexistence with the food ingredients is also to be collectively referred to as "addition" of the enzyme in the present invention. The order of reaction of the enzymes in the present invention with food ingredients is not particularly limited. The enzymes in the present invention may be added to and allowed to act on food ingredients all at the same time, or each may be added to and allowed to act on food ingredients separately or in any combination. The below-mentioned treatments with the enzyme preparation of the present invention can also be performed similarly.

The production method of the present invention can produce a modified, protein-containing food.

In the present specification, "modification" refers to imparting or enhancing a favorable texture (smoothness, hardness, elasticity). In addition, "modification" also includes suppression of off-taste or off-flavor, or suppression of unpleasantness through modification.

The presence or absence of modification can be evaluated according to the sensory evaluation in the below-mentioned Experimental Examples.

### 2. Enzyme preparation for modifying protein-containing food

The present invention also relates to an enzyme preparation for modifying protein-containing food (hereinafter also to be simply referred to as the enzyme preparation of the present invention) containing phospholipase D.

In the enzyme preparation of the present invention, the definition and examples of protein-containing food, examples of protein-containing food ingredients, examples of auxiliary materials, the amount of auxiliary materials in the food ingredients, and the definition, amount to be added, and method of addition (action time, action temperature, method of terminating the enzyme reaction) of phospholipase D are the same as the definition and examples of protein-containing food, examples of protein-containing food ingredients, examples of auxiliary materials, the amount of auxiliary materials in the food ingredients, and the definition, amount to be added, and method of addition (action time, action temperature, method of terminating the enzyme reaction) of phospholipase D in the production method of the present invention.

In the enzyme preparation of the present invention, it is preferable to further contain, in addition to the above-mentioned phospholipase D, an enzyme that contributes to the formation of a cross-linked structure. In the enzyme preparation of the present invention, the definition, examples, amount to be added, and method of addition of the enzyme that contributes to the formation of a cross-linked structure are the same as the definition, examples, amount to be added, and method of addition of the enzyme that contributes to the formation of a cross-linked structure in the production method of the present invention.

The enzyme preparation of the present invention can be added to a food material containing protein (preferably a food material further containing the above-mentioned auxiliary material) and reacted according to the method and amount of addition of phospholipase D (or phospholipase D and an enzyme that contributes to the formation of a cross-linked structure), explained in the above-mentioned production method of the present invention, to produce a modified protein-containing food.

The enzyme preparation of the present invention can also be used as a pickling liquid for meat processing, for example, to modify proteins in the processed meat foods shown below.

### 3. Pickling liquid for meat processing

The present invention also relates to a pickling liquid for meat processing (hereinafter also to be simply referred to as the pickling liquid of the present invention), which contains phospholipase D, to modify proteins in processed meat foods.

Examples of the processed meat food include ham, sausage, hamburger steak, and fried chicken.

In the pickling liquid of the present invention, the definition, amount to be added, and method of addition (action time, action temperature, method of terminating the enzyme reaction) of phospholipase D are the same as the definition, amount to be added, and method of addition (action time, action temperature, method of terminating the enzyme reaction) of phospholipase D in the production method of the present invention.

In the pickling liquid of the present invention, it is preferable to further contain, in addition to the above-mentioned phospholipase D, an enzyme that contributes to the formation of a cross-linked structure. In the pickling liquid of the present invention, the definition, examples, amount to be added, and method of addition of the enzyme that contributes to the formation of a cross-linked structure are the same as the definition, examples, amount to be added, and method of addition of the enzyme that contributes to the formation of a cross-linked structure in the production method of the present invention.

Additives generally used in pickling liquids (e.g., salt, sugars (reduced starch syrup), polymerized phosphate, nitrite, sodium ascorbate, gelling agents (e.g., carrageenan), heterogeneous proteins (e.g., egg white, soy protein, milk protein, sodium caseinate), seasonings (e.g., Ajinomoto (trade name)), and coloring agents) may be added to the pickling liquid of the present invention. The amount of additives can be appropriately selected from the known amounts used in pickling liquids.

The amount of the pickling liquid of the present invention may be appropriately selected so that the enzyme in the present invention can be present in the above-mentioned preferred amount relative to the food ingredients of the processed meat food to be treated.

The pickling liquid of the present invention, or food ingredient of the processed meat food to be treated preferably contains an auxiliary material selected from the above-mentioned (A) to (I). These auxiliary materials may be contained alone or in combination of two or more. Examples of auxiliary materials and the amount of auxiliary materials in the food ingredients are the same as the examples of auxiliary materials and the amount of auxiliary materials in the food ingredients in the production method of the present invention.

### 4. Method for modifying protein-containing food

The present invention also relates to a method for modifying protein-containing food (hereinafter also to be simply referred to as the modification method of the present invention), which includes treating a food ingredient containing a protein with phospholipase D.

In the modification method of the present invention, the definition and examples of protein-containing food, examples of protein-containing food ingredients, examples of auxiliary materials, the amount of auxiliary materials in the food ingredients, and the definition, amount to be added, and method of addition (action time, action temperature, method of terminating the enzyme reaction) of phospholipase are the same as the definition and examples of protein-containing food, examples of protein-containing food ingredients, examples of auxiliary materials, the amount of auxiliary materials in the food ingredients, and the definition, amount to be added, and method of addition (action time, action temperature, method of terminating the enzyme reaction) of phospholipase D in the production method of the present invention.

In the modification method of the present invention, it is preferable to further add, in addition to the above-mentioned phospholipase D, an enzyme that contributes to the formation of a cross-linked structure, to food ingredients and allow same to act. In the modification method of the present invention, the definition, examples, amount to be added, and method of addition of the enzyme that contributes to the formation of a cross-linked structure are the same as the definition, examples, amount to be added, and method of addition of the enzyme that contributes to the formation of a cross-linked structure in the production method of the present invention.

The present invention is explained in more detail in the following by illustrating Examples and Experimental Examples; however, the present invention is not limited to these Examples and Experimental Examples.

### [Example]

In the following Experimental Examples 1 to 11, the raw materials and equipment shown in Tables 1 and 2 were used.

**[Table 1]**

| raw materials used | | |
|---|---|---|
| raw material name | trade name | company name |
| egg white (powder) | egg white powder | Taiyo Kagaku Co., Ltd. |
| soybean protein | New Fujipro IJN | FUJI OIL CO., LTD. |
| soybean protein | New Fujipro SEH | FUJI OIL CO., LTD. |
| wheat protein | Fumerit G | Nagata Group Holdings Ltd. |
| wheat protein | A-glu G | Glico Nutrition Co., Ltd. |
| milk protein | Super-Lact No.1 | Taiyo Kagaku Co., Ltd. |
| casein Na | Casein Sodium LW | Nippon Shinyaku Co., Ltd. |
| Cricket Protein | Cricket Flour | TAKEO, Inc. |
| Big Cricket Protein | Big Cricket Powder | TAKEO, Inc. |
| Pea protein | NUTRALYS S85F | Roquette |
| Almond protein | Almond Protein | NutButterConcept |
| Fava protein | Fava Bean Protein | Green Boy |
| emulsifier | Sunlecithin A-1 | Taiyo Kagaku Co., Ltd. |
| frozen fish paste | Alaska pollock land grade 2 | TOKAI DENPUN CO., LTD. |
| frozen fish paste | hairtail | TOKAI DENPUN CO., LTD. |
| potato starch | Nisshoku Ginrei | Nihon Shokuhin Kagaku Kogyo |
| wheat starch | Kitanoyuki | Kitaguni Food |
| Waxy cornstarch | Nisshoku waxy starch | NIHON SHOKUHIN KAKO CO., LTD. |
| Tapioca starch | NOVATION3300 | Ingredion |
| gum arabic | Spastab 2A | Nexira |
| Tamarind seed gum | Glyloid 2A | Sumitomo Pharma Food & Chemical Co., Ltd. |
| yeast extract | Savorboost F | Ajinomoto Co., Inc. |
| coconut oil | Organic Premium Coconut Oil | Cocowell |
| lactic acid | Lactic Acid Powder | Mezzoni Foods |
| processed starch | Actbody A-700 | J-OIL MILLS, INC. |
| medium-strength flour | Suzume | Nisshin Flour Milling Inc. |
| alginic acid ester | kelp acid 501 | KIMICA Corporation |
| brine | Ramen brine | Pioneer Kikaku |
| sodium chloride | NAKURU M | Naikai Trading Co., Ltd. |
| vegetable oil | Canola oil | J-OIL MILLS, INC. |
| sugar | CIG | Itochu Sugar Co., Ltd. |
| "Ajinomoto" | MSG-FC | Ajinomoto Co., Inc. |
| ascorbic acid Na | Sodium L-ascorbate | Nippon Bulk Yakuhin Co., Ltd. |
| 10% sodium nitrite preparation | Highcolor HL | Chiyoda Industry Co., Ltd. |
| carrageenan | SATIGEL RPI730 | Unitech Foods Co., Ltd. |
| reduced starch syrup | Amameal | Mitsubishi Corporation Life Sciences Limited |
| polyphosphosphate preparation | Polygon C | Chiyoda Industry Co., Ltd. |
| cochineal color | SR Red K-6 | San-Ei Gen F.F.I. Inc. |
| gardenia dye | Yellow Color TH-G | T. HASEGAWA CO., LTD. |
| granulated sugar | Granulated sugar CIG | Itochu Sugar Co., Ltd. |
| glucose | hydrated crystalline glucose | NIHON SHOKUHIN KAKO CO., LTD. |
| frozen egg white | frozen egg white | Kewpie Corporation |
| phospholipase D (PLD) | DENAZYME PMD-P1 | Nagase ChemteX Corporation |
| ascorbic acid oxidase (ASO) | ASO-D10FD | Nagase ChemteX Corporation |

**[Table 2]**

| equipment used | | |
|---|---|---|
| equipment name | model | company name |
| vacuum packaging machine | A-300/16 | Tokyo Food Machinery Co., Ltd. |
| hot-water bath | TBN802DA06A | Advantec |
| frozen cutter | FZ | Shonan Sangyo |
| Stephan cutter | UMC-5 | Tokyo Food Machinery Co., Ltd. |
| small steamer | 3-C type | Yanagiya Machinery Co., Ltd. |
| chopper | Great mincer WMG-22 | Watanabe Foodmach Co., Ltd. |
| slicer | Ham slicer HL-2 | mikuniseisakusho |
| impinger | FGHOA9L | Fujimac |
| casing | Krehalon film SEAM DX470R (48mm×300mm) | KUREHA TRADING Co., Ltd. |

### [Experimental Example 1] Confirmation of effect of adding phospholipase D in soy gel system

Soy gel samples 1-1 to 1-7 were prepared according to the sample preparation flow shown in Fig. 1, using the mixing recipes shown in Table 3. The prepared samples exhibit the properties of either a suspension or sol or gel.

The samples 1-1 to 1-7 obtained were subjected to a sensory evaluation of smoothness, hardness, elasticity, and off-taste or off-flavor by four expert panelists according to the following evaluation criteria. The results are shown in Table 4.

**[Table 3]**

| <mixing recipe> unit: wt% | | | | | | | |
|---|---|---|---|---|---|---|---|
| raw materials | sample No. | | | | | | |
| | 1-1 | 1-2 | 1-3 | 1-4 | 1-5 | 1-6 | 1-7 |
| soybean protein (*1) | 20.0 | 20.0 | 20.0 | 20.0 | 20.0 | 20.0 | 20.0 |
| water | 80.0 | 80.0 | 80.0 | 80.0 | 80.0 | 80.0 | 80.0 |
| PLD preparation (*2) | | 0.5 | | 0.5 | | 0.5 | |
| TG preparation (*3) | | | 0.5 | 0.5 | | | |
| ASO preparation (*4) | | | | | 0.5 | 0.5 | |
| emulsifier | | | | | | | 0.5 |
| total | 100.0 | 100.5 | 100.5 | 101.0 | 100.5 | 101.0 | 100.5 |

| | | | | | | | |
|---|---|---|---|---|---|---|---|
| *1 soybean protein: trade name New Fujipro SEH *2 PLD preparation: containing PLD (phospholipase D) 1.5 wt%, dextrin 98.5 wt%; the number of PLD units per 1 g of PLD preparation is 820 U. The amount of PLD in sample 1-2, 1-4, 1-6 is 23.6 U when converted to enzyme activity for 1 g of protein in the sample. *3 TG preparation: containing TG (transglutaminase) 10 wt%, dextrin 90 wt%; the number of TG units per 1 g of TG preparation is 110 U. The amount of TG in samples 1-3, 1-4 is 3.2 U when converted to enzyme activity per 1 g of protein in the same sample. *4 ASO preparation: containing ASO (ascorbic acid oxidase) 1.7 wt%, ascorbic acid Na 27.5 wt%, dextrin 70.8 wt%; the number of units per 1 g of ASO preparation is 19.3 U. The amount of TG in samples 1-5, 1-6 is 70.2 U when converted to enzyme activity per 1 g of ascorbic acid Na (converted to L-ascorbic acid) in the same sample. | | | | | | | |

### [Evaluation criteria (smoothness)]

smoothness: compared to control
   5 points: very smooth texture
   4 points: smooth texture
   3 points: slightly smooth texture 2 points: the same
   1 point: gritty texture

### [Evaluation criteria (hardness)]

hardness : compared to control
5 points: very hard texture
4 points: hard texture
3 points: slightly hard texture
2 points: same
1 point: soft texture

### [Evaluation criteria (elasticity)]

elasticity: compared to control
5 points: very flexible texture
4 points: flexible texture
3 points: slightly flexible texture
2 points: same
1 point: brittle texture

### [Evaluation criteria (off-taste or off-flavor)]

off-taste or off-flavor: compared to control
○: no off-taste or off-flavor
Δ: slightly off-taste or off-flavor
×: off-taste or off-flavor

**[Table 4]**

| | sample No. | | | | | | |
|---|---|---|---|---|---|---|---|
| | 1-1 | 1-2 | 1-3 | 1-4 | 1-5 | 1-6 | 1-7 |
| smoothness | - | 5 | 2 | 5 | 3 | 5 | 4 |
| hardness | - | 2 | 5 | 5 | 4 | 4 | 2 |
| elasticity | - | 2 | 4 | 4 | 5 | 5 | 2 |
| off-taste or off-flavor | ○ | ○ | ○ | ○ | ○ | ○ | × |

From the results of Table 4, sample 1-2, with addition of a PLD preparation, was improved in smoothness compared to sample 1-1 (control).

In addition, sample 1-4, with addition of a PLD preparation and a TG preparation, was improved in smoothness, hardness, and elasticity compared to sample 1-1 (control).

In addition, sample 1-6, with addition of a PLD preparation and an ASO preparation, was improved in smoothness, hardness, and elasticity compared to sample 1-1 (control).

### [Experimental Example 2] Confirmation of effect of adding phospholipase D in various protein solutions and gel systems

Various protein gel samples 2-1 to 2-8, and various protein solution samples 2-9 to 2-12 were prepared according to the sample preparation flow shown in Fig. 5, using the mixing recipes shown in Table 5. The prepared samples exhibit the properties of either a suspension or sol or gel.

The samples 2-1 to 2-12 obtained were subjected to a sensory evaluation of smoothness by four expert panelists according to the following evaluation criteria. The results are shown in Table 6.

**[Table 5]**

| <Mixing recipe> unit: wt% | | | | | | | | | | | | |
|---|---|---|---|---|---|---|---|---|---|---|---|---|
| | sample No. | | | | | | | | | | | |
| raw material | 2-1 (control) | 2-2 | 2-3 (control) | 2-4 | 2-5 (control) | 2-6 | 2-7 (control) | 2-8 | 2-9 (control) | 2-10 | 2-11 (control) | 2-12 |
| soybean protein (*1) | 20.0 | 20.0 | | | | | | | | | | |
| wheat protein | | | 20.0 | 20.0 | | | | | | | | |
| milk protein | | | | | 20.0 | 20.0 | | | | | | |
| casein Na | | | | | | | 20.0 | 20.0 | | | | |
| Cricket Protein | | | | | | | | | 20.0 | 20.0 | | |
| Big Cricket Protein | | | | | | | | | | | 20.0 | 20.0 |
| water | 80.0 | 80.0 | 80.0 | 80.0 | 80.0 | 80.0 | 80.0 | 80.0 | 80.0 | 80.0 | 80.0 | 80.0 |
| PLD preparation (*2) | | 0.5 | | 0.5 | | 0.5 | | 0.5 | | 0.5 | | 0.5 |
| total | 80.0 | 80.5 | 100.0 | 100.5 | 100.0 | 100.5 | 100.0 | 100.5 | 100.0 | 100.5 | 100.0 | 100.5 |

| | | | | | | | | | | | | |
|---|---|---|---|---|---|---|---|---|---|---|---|---|
| *1 soybean protein: trade name New Fujipro SEH *2 PLD preparation: containing PLD (phospholipase D) 1.5 wt%, dextrin 98.5 wt%; the number of PLD units per 1 g of PLD preparation is 820 U. The amount of PLD in samples 2-2, 2-4, 2-6, 2-8, 2-10, 2-12 is 23.6 U, 27.0 U, 29.3 U, 23.0 U, 39.4 U, 37.3 U, respectively, when converted to enzyme activity for 1 g of protein in each sample. | | | | | | | | | | | | |

### [Evaluation criteria (smoothness)]

smoothness: compared to control
5 points: very smooth texture
4 points: smooth texture
3 points: slightly smooth texture
2 points: the same
1 point: gritty texture

**[Table 6]**

| | sample No. | | | | | | | | | | | |
|---|---|---|---|---|---|---|---|---|---|---|---|---|
| | 2-1 | 2-2 | 2-3 | 2-4 | 2-5 | 2-6 | 2-7 | 2-8 | 2-9 | 2-10 | 2-11 | 2-12 |
| smoothness | - | 5 | - | 5 | - | 5 | - | 5 | - | 5 | - | 5 |

From the results of Table 6, sample 2-2, with a PLD preparation added to soybean protein gel, was improved in smoothness compared to sample 2-1 (control).

In addition, sample 2-4, with a PLD preparation added to wheat protein gel, was improved in smoothness compared to sample 2-3 (control).

In addition, sample 2-6, with a PLD preparation added to milk protein gel, was improved in smoothness compared to sample 2-5 (control).

In addition, sample 2-8, with a PLD preparation added to casein Na gel, was improved in smoothness compared to sample 2-7 (control).

In addition, sample 2-10, with a PLD preparation added to Cricket Protein solution, was improved in smoothness compared to sample 2-9 (control).

In addition, sample 2-12, with a PLD preparation added to Big Cricket Protein solution, was improved in smoothness compared to sample 2-11 (control).

### [Experimental Example 3] Confirmation of effect of adding phospholipase D in ham model system

Pickling liquid samples 3-1 to 3-4 were prepared according to the sample preparation flow shown in Fig. 3, using the mixing recipes shown in Table 7. Subsequently, according to the sample preparation flow shown in Fig. 3, the obtained pickling liquid samples 3-1 to 3-4 and raw material meat (pork loin produced in Denmark) were used to prepare ham samples 3-1 to 3-4, respectively. The mixing ratio (weight ratio) of the raw material meat and the pickling liquid was 1:1.

The obtained ham samples 3-1 to 3-4 were subjected to a sensory evaluation of smoothness, hardness, elasticity, off-taste or off-flavor, and overall evaluation by four expert panelists according to the following evaluation criteria. The results are shown in Table 8.

**[Table 7]**

| <pickling liquid mixing recipe> | | | | | |
|---|---|---|---|---|---|
| raw material group | raw material name | pickling liquid sample No. | | | |
| | | 3-1 | 3-2 | 3-3 | 3-4 |
| | | mixing ratio (wt%) | mixing ratio (wt%) | mixing ratio (wt%) | mixing ratio (wt%) |
| B | egg white (powder) <egg white powder> | 2.5 | 1.25 | 1.25 | 1.25 |
| | soybean protein <New Fujipro IJN> | 5 | 6.25 | 6.25 | 6.25 |
| | milk protein <Super-Lact No.1> | 1 | 1 | 1 | 1 |
| | casein Na <Casein Sodium LW> | 0.2 | 0.2 | 0.2 | 0.2 |
| C | sodium chloride <NAKURU M> | 2.3 | 2.3 | 2.3 | 2.3 |
| | ascorbic acid Na <Sodium L-ascorbate> | 0.2 | 0.2 | 0.2 | 0.2 |
| | 10% sodium nitrite preparation <Highcolor HL> | 0.4 | 0.4 | 0.4 | 0.4 |
| D | "Ajinomoto" <MSG-FC> | 0.5 | 0.5 | 0.5 | 0.5 |
| | carrageenan <SATIGEL RPI730> | 0.3 | 0.3 | 0.3 | 0.3 |
| | reduced starch syrup <Amameal> | 10 | 10 | 10 | 10 |
| A | polyphosphosphate preparation <Polygon C> | 0.4 | 0.4 | 0.4 | 0.4 |
| | cochineal color <SR Red K-6> | 0.1 | 0.1 | 0.1 | 0.1 |
| water | | 77.1 | 77.1 | 77.1 | 77.1 |
| total | | 100 | 100 | 100 | 100 |
| TG preparation (*1) | | | | 0.2 | 0.2 |
| PLD preparation (*2) | | | | | 0.1 |

| | | | | | |
|---|---|---|---|---|---|
| *1 TG preparation (trade name Activa (registered trademark) TG-H-NF): containing TG (transglutaminase) 0.4 wt%; the number of TG units per 1 g of TG preparation is 45 U. The amount of TG in pickling liquid samples 3-3, 3-4 is 1.4 U when converted to enzyme activity for 1 g of protein in the same sample. *2 PLD preparation: containing PLD (phospholipase D) 1.5 wt%, dextrin 98.5 wt%; the number of PLD units per 1 g of PLD preparation is 820 U. The amount of PLD in pickling liquid sample 3-4 is 12.9 U when converted to enzyme activity for 1 g of protein in the same sample. | | | | | |

### [Evaluation criteria (smoothness)]

smoothness: as ham
5 points: very smooth texture and favorable
4 points: smooth texture and favorable
3 points: slightly smooth texture and favorable
2 points: average texture
1 point: texture is gritty and unfavorable

### [Evaluation criteria (hardness)]

hardness : as ham
5 points: very hard texture and favorable
4 points: hard texture and favorable
3 points: slightly hard texture and favorable
2 points: average texture
1 point: soft texture and unfavorable

### [Evaluation criteria (elasticity)]

elasticity: as ham
5 points: very flexible texture and favorable
4 points: flexible texture and favorable
3 points: slightly flexible texture and favorable
2 points: average texture
1 point: brittle texture and unfavorable

### [Evaluation criteria (off-taste or off-flavor)]

off-taste or off-flavor: as ham
O: no off-taste or off-flavor
Δ: off-taste or off-flavor is slightly detected
×: off-taste or off-flavor is detected

### [Evaluation criteria (overall evaluation)]

overall evaluation: as ham
⊙: very favorable texture and flavor
O: favorable texture and flavor
Δ: slightly favorable texture and flavor
×: unfavorable texture and flavor

**[Table 8]**

| | sample No. | | | |
|---|---|---|---|---|
| | 3-1 | 3-2 | 3-3 | 3-4 |
| smoothness | 5 | 2 | 2 | 5 |
| hardness | 4 | 3 | 5 | 4 |
| elasticity | 4 | 3 | 5 | 4 |
| off-taste or off-flavor | ○ | ○ | ○ | ○ |
| overall evaluation | ⊙ | Δ | ○ | ⊙ |

From the results of Table 8, sample 3-4, with a PLD preparation and a TG preparation added to a mixture of egg white, soybean protein, milk protein, and casein Na, was improved in smoothness, hardness, and elasticity compared to sample 3-2 (control).

### [Experimental Example 4] Confirmation of effect of adding phospholipase D in water kneading (chikuwa) system

Chikuwa samples 4-1 to 4-2 were prepared according to the sample preparation flow shown in Fig. 4, using the mixing recipes shown in Table 9. The obtained chikuwa samples 4-1 to 4-2 were subjected to a sensory evaluation of smoothness, hardness, elasticity, off-taste or off-flavor, and overall evaluation by four expert panelists according to the following evaluation criteria. The results are shown in Table 10.

**[Table 9]**

| <mixing recipe> unit: wt% | | |
|---|---|---|
| raw materials | sample No. | |
| | 4-1 | 4-2 |
| frozen fish paste (*1) | 42.0 | 42.0 |
| soybean protein | 8.0 | 8.0 |
| potato starch | 4.0 | 4.0 |
| sodium chloride | 1.2 | 1.2 |
| granulated sugar | 1.6 | 1.6 |
| glucose | 1.0 | 1.0 |
| "Ajinomoto" | 0.4 | 0.4 |
| vegetable fats and oils | 1.2 | 1.2 |
| frozen egg white | 1.2 | 1.2 |
| ice+water | 39.4 | 39 |
| PLD preparation (*2) | | 0.4 |
| total | 100.0 | 100.0 |

| | | |
|---|---|---|
| *1 frozen fish paste: trade name Alaska pollock land grade 2 *2 PLD preparation: containing PLD (phospholipase D) 1.5 wt%, dextrin 98.5 wt%; the number of PLD units per 1 g of PLD preparation is 820 U. The amount of PLD in samples 4-2 is 22.4 U, when converted to enzyme activity for 1 g of protein in the same sample. | | |

### [Evaluation criteria (smoothness)]

smoothness: as chikuwa
5 points: very smooth texture and favorable
4 points: smooth texture and favorable
3 points: slightly smooth texture and favorable
2 points: average texture
1 point: texture is gritty and unfavorable

### [Evaluation criteria (hardness)]

hardness: as chikuwa
5 points: very hard texture and favorable
4 points: hard texture and favorable
3 points: slightly hard texture and favorable
2 points: average texture
1 point: soft texture and unfavorable

### [Evaluation criteria (elasticity)]

elasticity: as chikuwa
5 points: very flexible texture and favorable
4 points: flexible texture and favorable
3 points: slightly flexible texture and favorable
2 points: average texture
1 point: brittle texture and unfavorable

### [Evaluation criteria (off-taste or off-flavor)]

off-taste or off-flavor: as chikuwa
○: no off-taste or off-flavor
Δ: off-taste or off-flavor is slightly detected
×: off-taste or off-flavor is detected

### [Evaluation criteria (overall evaluation)]

overall evaluation: as chikuwa
⊙: very favorable texture and flavor
O: favorable texture and flavor
Δ: slightly favorable texture and flavor
×: unfavorable texture and flavor

**[Table 10]**

| | sample No. | |
|---|---|---|
| | 4-1 | 4-2 |
| smoothness | 1 | 5 |
| hardness | 2 | 2 |
| elasticity | 2 | 2 |
| off-taste or off-flavor | ○ | ○ |
| overall evaluation | × | ○ |

From the results of Table 10, chikuwa sample 4-2 produced by adding a PLD preparation to a chikuwa material containing soybean protein was improved in smoothness compared to sample 4-1 (control).

### [Experimental Example 5] Confirmation of effect of adding phospholipase D in water kneading (chikuwa) system

Chikuwa samples 5-1 to 5-4 were prepared according to the sample preparation flow shown in Fig. 5, using the mixing recipes shown in Table 11.

The obtained chikuwa samples 5-1 to 5-4 were subjected to a sensory evaluation of smoothness, hardness, elasticity, off-taste or off-flavor, and overall evaluation by four expert panelists according to the following evaluation criteria. The results are shown in Table 12.

**[Table 11]**

| <mixing recipe> unit: wt% | | | | |
|---|---|---|---|---|
| | sample No. | | | |
| | 5-1 | 5-2 | 5-3 | 5-4 |
| frozen fish paste (trade name Alaska pollock land grade 2) | 50.0 | 25.0 | 25.0 | 25.0 |
| frozen fish paste (trade name hairtail) | | 20.0 | 20.0 | 20.0 |
| soybean protein | 3.0 | 5.0 | 5.0 | 5.0 |
| potato starch | 4.0 | 4.0 | 4.0 | 4.0 |
| sodium chloride | 1.2 | 1.2 | 1.2 | 1.2 |
| granulated sugar | 1.6 | 1.6 | 1.6 | 1.6 |
| glucose | 1.0 | 1.0 | 1.0 | 1.0 |
| "Ajinomoto" | 0.4 | 0.4 | 0.4 | 0.4 |
| vegetable fats and oils | 1.0 | 1.0 | 1.0 | 1.0 |
| frozen egg white | 1.0 | 1.0 | 1.0 | 1.0 |
| ice+water | 36.8 | 39.8 | 39.5 | 39.4 |
| PLD preparation (*1) | | | 0.3 | 0.3 |
| TG preparation (*2) | | | | 0.1 |
| total | 100.0 | 100.0 | 100.0 | 100.0 |

| | | | | |
|---|---|---|---|---|
| *1 PLD preparation: containing PLD (phospholipase D) 1.5 wt%, dextrin 98.5 wt%; the number of PLD units per 1 g of PLD preparation is 820 U. The amount of PLD in samples 5-3, 5-4 is 19.9 U when converted to enzyme activity for 1 g of protein in the same sample. *2 TG preparation (Activa (registered trademark) TG-AK): containing TG (transglutaminase) 6.4 wt%; the number of TG units per 1 g of TG preparation is 69 U. The amount of TG in sample 5-4 is 0.6 U when converted to enzyme activity for 1 g of protein in the same sample. | | | | |

### [Evaluation criteria (smoothness)]

smoothness: as chikuwa
5 points: very smooth texture and favorable
4 points: smooth texture and favorable
3 points: slightly smooth texture and favorable
2 points: average texture
1 point: texture is gritty and unfavorable

### [Evaluation criteria (hardness)]

hardness: as chikuwa
5 points: very hard texture and favorable
4 points: hard texture and favorable
3 points: slightly hard texture and favorable
2 points: average texture
1 point: soft texture and unfavorable

### [Evaluation criteria (elasticity)]

elasticity : as chikuwa
5 points: very flexible texture and favorable
4 points: flexible texture and favorable
3 points: slightly flexible texture and favorable
2 points: average texture
1 point: brittle texture and unfavorable

### [Evaluation criteria (off-taste or off-flavor)]

off-taste or off-flavor: as chikuwa,
○: no off-taste or off-flavor
Δ: off-taste or off-flavor is slightly detected
×: off-taste or off-flavor is detected

### [Evaluation criteria (overall evaluation)]

overall evaluation: as chikuwa,
⊙: very favorable texture and flavor
O: favorable texture and flavor
Δ: slightly favorable texture and flavor
×: unfavorable texture and flavor

**[Table 12]**

| | sample No. | | | |
|---|---|---|---|---|
| | 5-1 | 5-2 | 5-3 | 5-4 |
| smoothness | 4 | 1 | 4 | 4 |
| hardness | 3 | 2 | 2 | 4 |
| elasticity | 4 | 2 | 2 | 4 |
| off-taste or off-flavor | ○ | ○ | ○ | ○ |
| overall evaluation | ○ | × | Δ | ○ |

From the results of Table 12, chikuwa sample 5-3 produced by adding a PLD preparation to a chikuwa material containing soybean protein was improved in smoothness compared to sample 5-2 (control).

In addition, chikuwa sample 5-4 produced by adding a PLD preparation and a TG preparation to a chikuwa material containing soybean protein was improved in smoothness, hardness, and elasticity compared to sample 5-2 (control).

### [Experimental Example 6] Confirmation of effect of adding phospholipase D in water kneading (kamaboko) system

Kamaboko samples 6-1 to 6-2 were prepared according to the sample preparation flow shown in Fig. 6, using the mixing recipes shown in Table 13.

The obtained kamaboko samples 6-1 to 6-2 were subjected to a sensory evaluation of smoothness, hardness, elasticity, off-taste or off-flavor, and overall evaluation by four expert panelists according to the following evaluation criteria. The results are shown in Table 14.

**[Table 13]**

| <mixing recipe> unit: wt% | | |
|---|---|---|
| raw materials | sample No. | |
| | 6-1 | 6-2 |
| frozen fish paste (*1) | 42.0 | 42.0 |
| soybean protein | 8.0 | 8.0 |
| potato starch | 4.0 | 4.0 |
| sodium chloride | 1.2 | 1.2 |
| granulated sugar | 1.6 | 1.6 |
| glucose | 1.0 | 1.0 |
| "Ajinomoto" | 0.4 | 0.4 |
| vegetable fats and oils | 1.2 | 1.2 |
| frozen egg white | 1.2 | 1.2 |
| ice+water | 39.4 | 39 |
| PLD preparation (*2) | | 0.4 |
| total | 100.0 | 100.0 |

| | | |
|---|---|---|
| *1 frozen fish paste: trade name Alaska pollock land grade 2 *2 PLD preparation: containing PLD (phospholipase D) 1.5 wt%, dextrin 98.5 wt%; the number of PLD units per 1 g of PLD preparation is 820 U. The amount of PLD in sample 6-2 is 22.4 U when converted to enzyme activity for 1 g of protein in the same sample. | | |

### [Evaluation criteria (smoothness)]

smoothness: as kamaboko,
5 points: very smooth texture and favorable
4 points: smooth texture and favorable
3 points: slightly smooth texture and favorable
2 points: average texture
1 point: texture is gritty and unfavorable

### [Evaluation criteria (hardness)]

hardness: as kamaboko
5 points: very hard texture and favorable
4 points: hard texture and favorable
3 points: slightly hard texture and favorable
2 points: average texture
1 point: soft texture and unfavorable

### [Evaluation criteria (elasticity)]

elasticity: as kamaboko
5 points: very flexible texture and favorable
4 points: flexible texture and favorable
3 points: slightly flexible texture and favorable
2 points: average texture
1 point: brittle texture and unfavorable

### [Evaluation criteria (off-taste or off-flavor)]

off-taste or off-flavor: as kamaboko
O: no off-taste or off-flavor
Δ: off-taste or off-flavor is slightly detected
×: off-taste or off-flavor is detected

### [Evaluation criteria (overall evaluation)]

overall evaluation: as kamaboko
⊙: very favorable texture and flavor
O: favorable texture and flavor
Δ: slightly favorable texture and flavor
×: unfavorable texture and flavor

**[Table 14]**

| | sample No. | |
|---|---|---|
| | 6-1 | 6-2 |
| smoothness | 1 | 5 |
| hardness | 3 | 3 |
| elasticity | 1 | 2 |
| off-taste or off-flavor | ○ | ○ |
| overall evaluation | × | ○ |

From the results of Table 14, kamaboko sample 6-2 produced by adding a PLD preparation to a kamaboko material containing soybean protein was improved in smoothness and elasticity compared to sample 6-1 (control).

### [Experimental Example 7] Confirmation of effect of adding phospholipase D in soy gel system

Soy gel samples 7-1 to 7-10 were prepared according to the sample preparation flow shown in Fig. 7, using the mixing recipes shown in Table 15. The prepared samples exhibit the properties of either a suspension or sol or gel.

The obtained samples 7-1 to 7-10 were subjected to a sensory evaluation of smoothness by four expert panelists according to the following evaluation criteria. The results are shown in Table 16.

**[Table 15]**

| <mixing recipe> unit: wt% | | | | | | | | | | |
|---|---|---|---|---|---|---|---|---|---|---|
| raw materials | sample No. | | | | | | | | | |
| | 7-1 | 7-2 | 7-3 | 7-4 | 7-5 | 7-6 | 7-7 | 7-8 | 7-9 | 7-10 |
| soybean protein (*1) | 0.1 | 0.1 | 1.0 | 1.0 | 3.0 | 3.0 | 10.0 | 10.0 | 21.0 | 21.0 |
| water | 99.9 | 99.9 | 99.0 | 99.0 | 97.0 | 97.0 | 90.0 | 90.0 | 79.0 | 79.0 |
| PLD preparation (*2) | | 0.5 | | 0.5 | | 0.5 | | 0.5 | | 0.5 |
| total | 100.0 | 100.5 | 100.0 | 100.5 | 100.0 | 100.5 | 100.0 | 100.5 | 100.0 | 100.5 |

| | | | | | | | | | | |
|---|---|---|---|---|---|---|---|---|---|---|
| *1 soybean protein: trade name New Fujipro SEH *2 PLD preparation: containing PLD (phospholipase D) 1.5 wt%, dextrin 98.5 wt%; the number of PLD units per 1 g of PLD preparation is 820 U. The amount of PLD in samples 7-2, 7-4, 7-6, 7-8, 7-10 is 4710 U, 471 U, 157 U, 47 U, 22 U, respectively, when converted to enzyme activity for 1 g of protein in each sample. | | | | | | | | | | |

### [Evaluation criteria (smoothness)]

smoothness: compared to control
5 points: very smooth texture
4 points: smooth texture
3 points: slightly smooth texture
2 points: the same
1 point: gritty texture

**[Table 16]**

| | sample No. | | | | | | | | | |
|---|---|---|---|---|---|---|---|---|---|---|
| | 7-1 | 7-2 | 7-3 | 7-4 | 7-5 | 7-6 | 7-7 | 7-8 | 7-9 | 7-10 |
| smoothness | - | 3 | - | 4 | - | 5 | - | 5 | - | 5 |

From the results of Table 16, samples 7-2, 7-4, 7-6, 7-8, 7-10 obtained by adding PLD preparation to soybean protein gel were improved in smoothness compared to their respective controls, samples 7-1, 7-3, 7-5, 7-7, 7-9.

### [Experimental Example 8] Confirmation of effect of adding phospholipase D in water kneading (chikuwa) system

Chikuwa samples 8-1 to 8-7 were prepared according to the sample preparation flow shown in Fig. 8, using the mixing recipes shown in Table 17.

The obtained chikuwa samples 8-1 to 8-7 were subjected to a sensory evaluation of smoothness, hardness, elasticity, off-taste or off-flavor, and overall evaluation by four expert panelists according to the following evaluation criteria. The results are shown in Table 18.

**[Table 17]**

| <mixing recipe> unit: wt% | | | | | | | |
|---|---|---|---|---|---|---|---|
| raw materials | sample No. | | | | | | |
| | 8-1 | 8-2 | 8-3 | 8-4 | 8-5 | 8-6 | 8-7 |
| frozen fish paste (trade name Alaska pollock land grade 2) | 50.0 | 40.0 | 40.0 | 40.0 | 0.0 | 0.0 | 0.0 |
| frozen fish paste (trade name hairtail) | | 10.0 | 10.0 | 10.0 | 50.0 | 50.0 | 50.0 |
| soybean protein (*1) | 3.0 | 3.0 | 3.0 | 3.0 | 3.0 | 3.0 | 3.0 |
| potato starch | 4.0 | 4.0 | 4.0 | 4.0 | 4.0 | 4.0 | 4.0 |
| sodium chloride | 1.2 | 1.2 | 1.2 | 1.2 | 1.2 | 1.2 | 1.2 |
| granulated sugar | 1.6 | 1.6 | 1.6 | 1.6 | 1.6 | 1.6 | 1.6 |
| glucose | 1.0 | 1.0 | 1.0 | 1.0 | 1.0 | 1.0 | 1.0 |
| "Ajinomoto" | 0.4 | 0.4 | 0.4 | 0.4 | 0.4 | 0.4 | 0.4 |
| vegetable fats and oils | 1.0 | 1.0 | 1.0 | 1.0 | 1.0 | 1.0 | 1.0 |
| frozen egg white | 1.0 | 1.0 | 1.0 | 1.0 | 1.0 | 1.0 | 1.0 |
| ice+water | 36.8 | 36.8 | 36.8 | 36.8 | 36.8 | 36.8 | 36.8 |
| PLD preparation (*2) | | | 0.3 | 0.3 | | 0.3 | 0.3 |
| TG preparation (*3) | | | | 0.1 | | | 0.1 |
| total | 100.0 | 100.0 | 100.3 | 100.4 | 100.0 | 100.3 | 100.4 |

| | | | | | | | |
|---|---|---|---|---|---|---|---|
| *1 soybean protein: trade name New Fujipro SEH *2 PLD preparation: containing PLD (phospholipase D) 1.5 wt%, dextrin 98.5 wt%; the number of PLD units per 1 g of PLD preparation is 820 U. The amount of PLD in samples 8-3, 8-4 is 21.2 U when converted to enzyme activity for 1 g of protein in each sample. The amount of PLD in samples 8-6, 8-7 is 21.9 U when converted to enzyme activity for 1 g of protein in the same sample. *3 TG preparation (trade name Activa (registered trademark) TG-AK): containing TG (transglutaminase) 6.4 wt%; the number of TG units per 1 g of TG preparation is 69 U. The amount of TG in samples 8-4, 8-7 is 0.6 U when converted to enzyme activity for 1 g of protein in the same sample. | | | | | | | |

### [Evaluation criteria (smoothness)]

smoothness: as chikuwa
5 points: very smooth texture and favorable
4 points: smooth texture and favorable
3 points: slightly smooth texture and favorable
2 points: average texture
1 point: texture is gritty and unfavorable

### [Evaluation criteria (hardness)]

hardness: as chikuwa
5 points: very hard texture and favorable
4 points: hard texture and favorable
3 points: slightly hard texture and favorable
2 points: average texture
1 point: soft texture and unfavorable

### [Evaluation criteria (elasticity)]

elasticity: as chikuwa
5 points: very flexible texture and favorable
4 points: flexible texture and favorable
3 points: slightly flexible texture and favorable
2 points: average texture
1 point: brittle texture and unfavorable

### [Evaluation criteria (off-taste or off-flavor)]

off-taste or off-flavor: as chikuwa
O: no off-taste or off-flavor
Δ: slightly off-taste or off-flavor
×: off-taste or off-flavor

### [Evaluation criteria (overall evaluation)]

overall evaluation: as chikuwa
⊙: very favorable texture and flavor
O: favorable texture and flavor
Δ: slightly favorable texture and flavor
×: unfavorable texture and flavor

**[Table 18]**

| | sample No. | | | | | | |
|---|---|---|---|---|---|---|---|
| | 8-1 | 8-2 | 8-3 | 8-4 | 8-5 | 8-6 | 8-7 |
| smoothness | 4 | 2 | 4 | 4 | 1 | 4 | 4 |
| hardness | 3 | 2 | 2 | 3 | 1 | 1 | 3 |
| elasticity | 3 | 2 | 2 | 3 | 1 | 2 | 3 |
| off-taste or off-flavor | ○ | ○ | ○ | ○ | ○ | ○ | ○ |
| overall evaluation | ○ | × | Δ | ○ | × | Δ | ○ |

From the results of Table 18, sample 8-2, in which part of the amount of frozen fish paste (product name: Alaska pollock land grade 2) used as the raw material in sample 8-1 was replaced with a lower grade frozen fish paste (product name: hairtail), was deteriorated in smoothness, hardness, and elasticity. Sample 8-5, in which the entire amount of frozen fish paste (product name: Alaska pollock land grade 2) used as the raw material in sample 8-1 was replaced with a lower grade frozen fish paste (product name: hairtail), was further deteriorated in smoothness, hardness, and elasticity.

Chikuwa sample 8-3 produced by adding a PLD preparation to a chikuwa material containing soybean protein was improved in smoothness compared to sample 8-2 (control).

In addition, chikuwa sample 8-4 produced by adding a PLD preparation and a TG preparation to a chikuwa material containing soybean protein was improved in smoothness, hardness, and elasticity compared to sample 8-2 (control).

Chikuwa sample 8-6 produced by adding a PLD preparation to a chikuwa material containing soybean protein was improved in smoothness and elasticity compared to sample 8-5 (control).

In addition, chikuwa sample 8-7 produced by adding a PLD preparation and a TG preparation to a chikuwa material containing soybean protein was improved in smoothness, hardness, and elasticity compared to sample 8-5 (control).

### [Experimental Example 9] Confirmation of effect of adding phospholipase D in water kneading (chikuwa) system

Chikuwa samples 9-1 to 9-4 were prepared according to the sample preparation flow shown in Fig. 9, using the mixing recipes shown in Table 19.

The obtained chikuwa samples 9-1 to 9-4 were subjected to a sensory evaluation of smoothness, hardness, elasticity, off-taste or off-flavor, and overall evaluation by four expert panelists according to the following evaluation criteria. The results are shown in Table 20.

**[Table 19]**

| <mixing recipe> unit: wt% | | | | |
|---|---|---|---|---|
| raw materials | sample No. | | | |
| | 9-1 | 9-2 | 9-3 | 9-4 |
| frozen fish paste (trade name Alaska pollock land grade 2) | 50.0 | 49.0 | 49.0 | 49.0 |
| soybean protein (*1) | | 1.0 | 1.0 | 1.0 |
| potato starch | 4.0 | 4.0 | 4.0 | 4.0 |
| sodium chloride | 1.2 | 1.2 | 1.2 | 1.2 |
| granulated sugar | 1.6 | 1.6 | 1.6 | 1.6 |
| glucose | 1.0 | 1.0 | 1.0 | 1.0 |
| "Ajinomoto" | 0.4 | 0.4 | 0.4 | 0.4 |
| vegetable fats and oils | 1.0 | 1.0 | 1.0 | 1.0 |
| frozen egg white | 1.0 | 1.0 | 1.0 | 1.0 |
| ice+water | 39.8 | 39.8 | 39.8 | 39.8 |
| PLD preparation (*2) | | | 0.3 | 0.3 |
| TG preparation (*3) | | | | 0.1 |
| total | 100.0 | 100.0 | 100.3 | 100.4 |

| | | | | |
|---|---|---|---|---|
| *1 soybean protein: trade name New Fujipro SEH *2 PLD preparation: containing PLD (phospholipase D) 1.5 wt%, dextrin 98.5 wt%; the number of PLD units per 1 g of PLD preparation is 820 U. The amount of PLD in samples 9-3, 9-4 is 25.1 U when converted to enzyme activity for 1 g of protein in each sample. *3 TG preparation (trade name Activa (registered trademark) TG-AK): containing TG (transglutaminase) 6.4 wt%; the number of TG units per 1 g of TG preparation is 69 U. The amount of TG in sample 9-4 is 0.7 U when converted to enzyme activity for 1 g of protein in the same sample. | | | | |

### [Evaluation criteria (smoothness)]

smoothness: as chikuwa
5 points: very smooth texture and favorable
4 points: smooth texture and favorable
3 points: slightly smooth texture and favorable
2 points: average texture
1 point: texture is gritty and unfavorable

### [Evaluation criteria (hardness)]

hardness: as chikuwa
5 points: very hard texture and favorable
4 points: hard texture and favorable
3 points: slightly hard texture and favorable
2 points: average texture
1 point: soft texture and unfavorable

### [Evaluation criteria (elasticity)]

elasticity: as chikuwa
5 points: very flexible texture and favorable
4 points: flexible texture and favorable
3 points: slightly flexible texture and favorable
2 points: average texture
1 point: brittle texture and unfavorable

### [Evaluation criteria (off-taste or off-flavor)]

off-taste or off-flavor: as chikuwa
∘: no off-taste or off-flavor
Δ: off-taste or off-flavor is slightly detected
×: off-taste or off-flavor is detected

### [Evaluation criteria (overall evaluation)]

overall evaluation: as chikuwa
⊙: very favorable texture and flavor
∘: favorable texture and flavor
Δ: slightly favorable texture and flavor
×: unfavorable texture and flavor

**[Table 20]**

| | sample No. | | | |
|---|---|---|---|---|
| | 9-1 | 9-2 | 9-3 | 9-4 |
| smoothness | 4 | 2 | 4 | 4 |
| hardness | 1 | 2 | 2 | 3 |
| elasticity | 2 | 2 | 2 | 3 |
| off-taste or off-flavor | ○ | ○ | ○ | ○ |
| overall evaluation | Δ | × | Δ | ○ |

From the results of Table 20, sample 9-2, in which part of the amount of frozen fish paste (product name: Alaska pollock land grade 2) used as the raw material in sample 9-1 was replaced with soybean protein was deteriorated in smoothness.

Chikuwa sample 9-3 produced by adding a PLD preparation to a chikuwa material containing soybean protein was improved in smoothness compared to sample 9-2 (control).

In addition, chikuwa sample 9-4 produced by adding a PLD preparation and a TG preparation to a chikuwa material containing soybean protein was improved in smoothness, hardness, and elasticity compared to sample 9-2 (control).

### [Experimental Example 10] Confirmation of effect of adding phospholipase D to cheese analogue

Using the mixing recipe shown in Table 21, the raw materials were emulsified by heating with stirring at 70°C for 5 min (enzyme reaction step) using a heating stirrer (Thermomix (trade name), manufactured by Vorwek). Then, the raw materials were heated with stirring at 90°C for 8 min (enzyme deactivation step). The obtained mixture was filled into a mold and cooled in a refrigerator (5°C) for 48 hr to prepare cheese analog samples 10-1 to 10-6.

The obtained samples 10-1 to 10-6 were subjected to a sensory evaluation of smoothness by three expert panelists according to the following evaluation criteria. The results are shown in Table 22.

**[Table 21]**

| <mixing recipe> unit: wt% | | | | | | |
|---|---|---|---|---|---|---|
| raw materials | sample No. | | | | | |
| | 10-1 | 10-2 | 10-3 | 10-4 | 10-5 | 10-6 |
| Waxy cornstarch | 9.90 | 9.90 | 9.90 | 9.90 | 9.90 | 9.90 |
| tapioca starch | 9.30 | 9.30 | 9.30 | 9.30 | 9.30 | 9.30 |
| gum arabic | 1.83 | 1.83 | 1.83 | 1.83 | 1.83 | 1.83 |
| tamarind seed gum | 0.30 | 0.30 | 0.30 | 0.30 | 0.30 | 0.30 |
| yeast extract | 0.95 | 0.95 | 0.95 | 0.95 | 0.95 | 0.95 |
| coconut oil | 20.00 | 20.00 | 20.00 | 20.00 | 20.00 | 20.00 |
| water | 46.02 | 46.02 | 46.02 | 46.02 | 46.02 | 46.02 |
| Pea protein | 10.00 | 10.00 | | | | |
| Almond protein | | | 10.00 | 10.00 | | |
| Fava protein | | | | | 10.00 | 10.00 |
| sodium chloride | 1.40 | 1.40 | 1.40 | 1.40 | 1.40 | 1.40 |
| lactic acid | 0.30 | 0.30 | 0.30 | 0.30 | 0.30 | 0.30 |
| total | 100.00 | 100.00 | 100.00 | 100.00 | 100.00 | 100.00 |
| PLD preparation *1 | | 0.50 | | 0.50 | | 0.50 |

| | | | | | | |
|---|---|---|---|---|---|---|
| *1 PLD preparation: containing PLD (phospholipase D) 1.5 wt%, dextrin 98.5 wt%; the number of PLD units per 1 g of PLD preparation is 820 U. The amount of PLD in samples 10-2, 10-4, 10-6 is 48.2 U, 68.3 U, 45.6 U, when converted to enzyme activity for 1 g of protein in each sample. | | | | | | |

### [Evaluation criteria]

smoothness: as cheese analogue
5 points: very smooth texture and favorable
4 points: smooth texture and favorable
3 points: slightly smooth texture and favorable
2 points: powdery texture and unfavorable
1 point: very powdery texture and unfavorable

**[Table 22]**

| | sample No. | | | | | |
|---|---|---|---|---|---|---|
| | 10-1 | 10-2 | 10-3 | 10-4 | 10-5 | 10-6 |
| smoothness | 1 | 3 | 3 | 4 | 2 | 4 |
| note | Oil is separated. | Oil separation is suppressed. Powderiness is suppressed and smoothness is afforded. | | Smoothness increases. | Oil is separated. | Oil separation is suppressed. Powderiness is suppressed and smoothness is afforded. |

From the results of Table 22, samples 10-2, 10-4, 10-6 obtained by adding PLD preparation to cheese analogue were improved in smoothness compared to their respective controls, samples 10-1, 10-3, 10-5.

### [Experimental Example 11] Confirmation of effect of adding phospholipase D to Chinese noodles

The powder fraction of the raw materials was placed in a bag in the ratio shown in Table 23 and mixed by shaking by hand. The obtained mixture (charged amount 1 kg) was placed in a vertical kneader (2 kg vacuum kneader, manufactured by Otake Noodle Machine Co., Ltd.), and the water-soluble fraction shown in Table 23 was added over 30 seconds. After dispersing, the mixture was kneaded in a vertical kneader for 5 min (100 rpm 2 min, 50 rpm 3 min). After kneading, the mixture was spread, combined, and rolled in a noodle machine (small continuous rolling noodle machine, manufactured by Sodick Co., Ltd.) to obtain buckwheat dough. The obtained buckwheat dough was cut using a #20 cutting blade, and 70 g of the cut noodles were wrapped in packaging vinyl and frozen at -25°C. The frozen noodles were stored at -18°C.

The frozen noodles were boiled in hot water for 2 min, then drained, cooled and drained to prepare Chinese noodle samples 11-1 to 11-5.

The obtained samples 11-1 to 11-5 were divided into containers and a loosening agent "Soya Up M3000" (Fuji Oil Co., Ltd.) was added in an amount of 3% by weight of the noodles.

After storing in a refrigerator at 10°C or below for 2 days, the noodles were loosened in cold noodle soup in an amount of 33% by weight of the noodles, and a sensory evaluation was performed.

Sensory evaluation of smoothness, hardness, elasticity, and off-taste or off-flavor was performed by five expert panelists according to the following evaluation criteria. The results are shown in Table 24.

**[Table 23]**

| <mixing recipe> unit: wt% | | | | | | |
|---|---|---|---|---|---|---|
| | raw materials | sample No. | | | | |
| | | 11-1 | 11-2 | 11-3 | 11-4 | 11-5 |
| powder fraction | medium-strength flour | 67.0 | 67.0 | 67.5 | 67.5 | 67.5 |
| | processed starch | 26.8 | 26.8 | 27.0 | 27.0 | 27.0 |
| | wheat protein *1 | 4.0 | 4.0 | 4.0 | 4.0 | 4.0 |
| | egg white (powder) | 1.6 | 1.6 | 0.8 | 0.8 | 0.8 |
| | alginic acid ester | 0.5 | 0.5 | 0.5 | 0.5 | 0.5 |
| | gardenia dye | 0.1 | 0.1 | 0.1 | 0.1 | 0.1 |
| | phospholipase D *2 | 0.0037 | 0.0037 | | 0.0027 | 0.0040 |
| | total powder fraction | 100.0 | 100.0 | 100.0 | 100.0 | 100.0 |
| water-soluble fraction | brine | 1.5 | 1.5 | 1.5 | 1.5 | 1.5 |
| | sodium chloride | 2.0 | 2.0 | 2.0 | 2.0 | 2.0 |
| | tap water | 42.0 | 42.0 | 42.0 | 42.0 | 42.0 |

| | | | | | | |
|---|---|---|---|---|---|---|
| *1 wheat protein (trade name A-glu G) *2 PLD preparation: containing PLD (phospholipase D) 1.5 wt%, dextrin 98.5 wt%; the number of PLD units per 1 g of PLD preparation is 820 U. The amount of PLD in samples 11-2, 11-4, 11-5 is 20.0 U, 14.7 U, 21.8 U when converted to enzyme activity for 1 g of protein in each sample. | | | | | | |

### [Evaluation criteria (smoothness)]

smoothness: as Chinese noodles
5 points: very smooth texture and favorable
4 points: smooth texture and favorable
3 points: slightly smooth texture and favorable
2 points: average texture
1 point: gritty texture and unfavorable

### [Evaluation criteria (hardness)]

hardness: as Chinese noodles
5 points: very hard texture and favorable
4 points: hard texture and favorable
3 points: slightly hard texture and favorable
2 points: average texture
1 point: soft texture and unfavorable

### [Evaluation criteria (elasticity)]

elasticity: as Chinese noodles
5 points: very flexible texture and favorable
4 points: flexible texture and favorable
3 points: slightly flexible texture and favorable
2 points: average texture
1 point: brittle texture and unfavorable

### [Evaluation criteria (off-taste or off-flavor)]

off-taste or off-flavor: as Chinese noodles
∘: no off-taste or off-flavor
Δ: off-taste or off-flavor is slightly detected
×: off-taste or off-flavor is detected

**[Table 24]**

| | sample No. | | | | |
|---|---|---|---|---|---|
| | 11-1 | 11-2 | 11-3 | 11-4 | 11-5 |
| smoothness | 2 | 4 | 1 | 3 | 4 |
| hardness | 2 | 4 | 1 | 3 | 4 |
| elasticity | 2 | 4 | 1 | 3 | 4 |
| off-taste or off-flavor | ○ | ○ | ○ | ○ | ○ |

From the results of Table 24, sample 11-2, with a PLD preparation added to Chinese noodles, was improved in smoothness, hardness, and elasticity compared to sample 11-1 (control). Samples 11-4, 11-5, with a PLD preparation added to Chinese noodles, were improved in smoothness, hardness, and elasticity compared to sample 11-3 (control).

In the following Experimental Examples 12 - 32, unless otherwise specified, the raw materials and equipment shown in Tables 25-1, 25-2, 25-3, 26 were used.

**[Table 25-1]**

| raw materials used | | |
|---|---|---|
| raw material name | trade name | company name |
| egg white (powder) | egg white powder | Taiyo Kagaku Co., Ltd. |
| soybean protein | New Fujipro IJN | FUJI OIL CO., LTD. |
| soybean protein | New Fujipro SEH | FUJI OIL CO., LTD. |
| wheat protein | Fumerit G | Nagata Group Holdings Ltd. |
| milk protein | Super-Lact No.1 | Taiyo Kagaku Co., Ltd. |
| casein Na | Casein Sodium LW | Nippon Shinyaku Co., Ltd. |
| Cricket Protein | Cricket Flour | TAKEO, Inc. |
| Big Cricket Protein | Big Cricket Powder | TAKEO, Inc. |
| Cricket | Cricket Protein | TAKEO, Inc. |
| Silkworm Powder | Silkworm Powder | TAKEO, Inc. |
| lecithin | Sunlecithin A-1 | Taiyo Kagaku Co., Ltd. |
| Alaska pollock A (made in US) | Alaska pollock A (made in US) | TOKAI DENPUN CO., LTD. |
| Alaska pollock land grade 2 | Alaska pollock land grade 2 | TOKAI DENPUN CO., LTD. |
| hairtail C | hairtail C | TOKAI DENPUN CO., LTD. |
| potato starch | Nisshoku Ginrei | Nihon Shokuhin Kagaku Kogyo |
| wheat starch | Kitanoyuki | Hokkoku Food Co., Ltd. |
| sodium chloride/ sodium chloride | NAKURU M | Naikai Trading Co., Ltd. |
| vegetable fats and oils | Canola oil | J-OIL MILLS, INC. |
| sugar | CIG | Itochu Sugar Co., Ltd. |
| "Ajinomoto"/sodium glutamate | MSG-FC | Ajinomoto Co., Inc. |
| ascorbic acid Na | Sodium L-ascorbate | Nippon Bulk Yakuhin Co., Ltd. |
| 10% sodium nitrite preparation | Highcolor HL | Chiyoda Industry Co., Ltd. |
| carrageenan | SATIGEL RPI730 | Unitech Foods Co., Ltd. |
| reduced starch syrup | Amameal | Mitsubishi Corporation Life Sciences Limited |
| polyphosphosphate preparation | Polygon C | Chiyoda Industry Co., Ltd. |
| cochineal color | SR Red K-6 | San-Ei Gen F.F.I. Inc. |
| granulated sugar | granulated sugar CIG | Itochu Sugar Co., Ltd. |
| glucose | hydrated crystalline glucose | NIHON SHOKUHIN KAKO CO., LTD. |
| frozen egg white | frozen egg white | Kewpie Corporation |
| soy protein drink | SOY PROTEIN 100 COCOA FLAVOR | SAVAS |
| medium-strength flour | Suzume | Nisshin Flour Milling Inc. |
| processing starch | Actbody A-700 | J-OIL MILLS, INC. |
| wheat protein | A-glu G | Glico Nutrition Co., Ltd. |

**[Table 25-2]**

| raw materials used | | |
|---|---|---|
| raw material name | trade name | company name |
| alginic acid ester | kelp acid 501 | KIMICA Corporation |
| gardenia dye | "Yellow Color TH-G" 2 | T. HASEGAWA CO., LTD. |
| brine | Ramen brine | Pioneer Kikaku |
| phospholipase D (PLD) | DENAZYME PMD-P1 | Nagase & Co., Ltd. |
| ascorbic acid oxidase (ASO) | ASO-D10FD | Nagase & Co., Ltd. |
| sodium carbonate | purified sodium carbonate (anhydrous) | Daito Chemical Co., Ltd. |
| trisodium phosphate | trisodium phosphate | TAIHEI CHEMICAL INDUSTRIAL CO., LTD. |
| tripotassium phosphate | tripotassium phosphate | TAIHEI CHEMICAL INDUSTRIAL CO., LTD. |
| trisodium citrate | sodium citrate | KYUSHUKAKO Co., Ltd. |
| calcium chloride | calcium chloride | Tomita Pharmaceutical Co., Ltd. |
| calcinated shell calcium | calcinated shell calcium | N.C. CORPORATION |
| calcium lactate | calcium lactate | FUSO CHEMICAL CO., LTD. |
| calcium carbonate | MAMACALSO | Nitto Funka Kogyo K.K. |
| magnesium chloride | magnesium chloride | AKO KASEI CO., LTD. |
| magnesium glutamate | MAGNESIUM L-GLUTAMATE | AJINOMOTO FOODS EUROPE |
| glutathione-containing yeast extract | yeast extract powder -SG010 | ANGEL YEAST CO., LTD. |
| cysteine-containing yeast extract | CYE-P | Ajinomoto Co., Inc. |
| iron-containing yeast | HIGH IRON YEAST | GROW COMPANY INC. |
| copper-containing yeast | copper yeast 5% | Medience Corporation |
| manganese-containing yeast | LALMIN MN50 (manganese yeast pulverized product) | MIWA SEIYAKU CO., LTD. |
| glycine | Gly (FC) | Showa Tsusho |
| cystine | L- (Cys) 2 | Ajinomoto Healthy Supply Co., Inc. |
| alanine | DL-Ala | Nippon Kayaku Food Techno Co., Ltd. |
| valine | L-Val | Ajinomoto Co., Inc. |
| leucine | L-Leu | Ajinomoto Co., Inc. |
| isoleucine | L-Ile | Ajinomoto Co., Inc. |
| phenylalanine | L-Phe | Ajinomoto Co., Inc. |
| proline | L-Pro | Ajinomoto Co., Inc. |
| methionine | L-Met | Ajinomoto Co., Inc. |
| threonine | L-Thr | Ajinomoto Co., Inc. |
| serine | L-Ser | Ajinomoto Co., Inc. |
| glutamine | L-Gln | Ajinomoto Co., Inc. |

**[Table 25-3]**

| raw materials used | | |
|---|---|---|
| raw material name | trade name | company name |
| tyrosine | L-Tyr | Ajinomoto Healthy Supply Co., Inc. |
| cysteine | L-cysteine | Ajinomoto Healthy Supply Co., Inc. |
| cysteine hydrochloride | L-Cys HCl | Ajinomoto Healthy Supply Co., Inc. |
| arginine | L-Arq | Ajinomoto Co., Inc. |
| histidine | L-His | Ajinomoto Co., Inc. |
| lysine hydrochloride | L-Lys HCl | Ajinomoto Co., Inc. |
| sodium aspartate | Sodium L-aspartate | SATUMA KAKO CO., LTD. |
| ammonium chloride | food additive ammonium chloride MC | AKO KASEI CO., LTD. |
| sodium alginate | Snow Algin H | Fuji Chemical Industries Co., Ltd. |
| glutamylvalylglycine | γ-glutamylvalylglycine | Ajinomoto Co., Inc. |
| dietary fiber | Fuji FF | Fuji Nihon Seito Corporation |
| glucose oxidase (GO) | Sumizyme PGO | Shin Nihon Chemical Co., Ltd. |
| oat protein | ORPROTEIN (R) OT | ORGANO FOODTECH CORPORATION |
| pea protein | PP-CS | ORGANO FOODTECH CORPORATION |
| broad bean protein | ORPROTEIN (R) FP-AC | ORGANO FOODTECH CORPORATION |
| mung bean protein | ORPROTEIN (R) MP-AC | ORGANO FOODTECH CORPORATION |
| rice protein | Kometan - kissui | Glico Nutrition Co., Ltd. |
| chickpea protein | ORPROTEIN (R) CP-AC | ORGANO FOODTECH CORPORATION |
| rapeseed protein | Puratein G | Merit Functional Foods Corporation |
| corn powder | delicious corn powder | Hokkaido Knorr Foods Co., Ltd. |
| whey powder | Morinaga whey powder | MORINAGA MILK INDUSTRY CO., LTD. |
| whole milk powder | Yotsuba Whole Milk Powder | Yotsuba Milk Products Co., Ltd. |
| skim milk powder | Yotsuba Skim Milk Powder | Yotsuba Milk Products Co., Ltd. |
| Navy bean powder | Navy bean powder | Cargill Japan LLC |
| almond protein | Almond protein powder | Nissei Kyoeki Co., Ltd. |
| peanut powder | Roasted Peanut Protein | Nissei Kyoeki Co., Ltd. |
| spirulina | Spirulina powder | Nature One, Inc. |
| soy milk | Delicious Unsweetened Soy Milk | KIKKOMAN CORPORATION |
| oat milk | alpro | DANONE JAPAN |
| coconut milk | coconut milk | YOUKI FOOD Co., Ltd. |
| phospholipase A1 (PLAL) | phospholipase A1 | Mitsubishi Chemical Corporation |
| phospholipase A2 (PLA2) | PLA2 NAGASE 10P/R | Nagase & Co., Ltd. |
| Blanching enzyme (BE) | Blanching enzyme A | Nagase & Co., Ltd. |

**[Table 26]**

| equipment used | | |
|---|---|---|
| equipment name | model | company name |
| vacuum packaging machine | A-300/16 | Tokyo Food Machinery Co., Ltd. |
| hot-water bath | TBN802DA06A | Advantec |
| frozen cutter | FZ | Shonan Sangyo |
| Stephan cutter | UMC-5 | Tokyo Food Machinery Co., Ltd. |
| small steamer | 3-C type | Yanagiya Machinery Co., Ltd. |
| chopper | Great mincer WMG-22 | Watanabe Foodmach Co., Ltd. |
| slicer | Ham slicer HL-2 | mikuniseisakusho |
| impinger | FGHOA9L | Fujimac |
| casing | Krehalon film SEAM DX470R (48mm×300mm) | KUREHA TRADING Co., Ltd. |
| food processor | RM-3200VD | FMI Corporation |

### [Experimental Example 12] Confirmation of effect of adding phospholipase D in soy gel system

Soy gel samples 12-2 to 12-15 were prepared according to the sample preparation flow shown in Fig. 10, using the mixing recipes shown in Tables 27-1, 27-2. In addition, egg white gel sample 12-1 was prepared according to the sample preparation flow shown in Fig. 10, using the mixing recipes shown in Table 27-1. The prepared samples exhibit the properties of either a suspension or sol or gel.

The obtained each sample was subjected to a sensory evaluation of smoothness by three expert panelists according to the following evaluation criteria, using sample 12-2 as a control. The results are shown in Tables 28-1, 28-2.

### [Evaluation criteria (smoothness)]

smoothness: compared to control
5 points: very smooth texture
4 points: smooth texture
3 points: slightly smooth texture
2 points: the same
1 point: gritty texture

**[Table 27-1]**

| <Mixing recipe> | | | | | | | |
|---|---|---|---|---|---|---|---|
| | | sample No. | | | | | |
| | | 12-1 | 12-2 (control) | 12-3 | 12-4 | 12-5 | 12-6 |
| raw material (weight %) | soybean protein (*1) | | 10.0 | 10.0 | 10.0 | 10.0 | 10.0 |
| | egg white (powder) (*2) | 10.0 | | | | | |
| | water | 90.0 | 90.0 | 90.0 | 90.0 | 90.0 | 90.0 |
| | DENAZYME PMD-P1 | | | 0.0000000001 | 0.00000001 | 0.0000001 | 0.000001 |
| | total | 100.0 | 100.0 | 100.0 | 100.0 | 100.0 | 100.0 |
| PLD activity (U) for 1 g of protein in sample | | | | 0.00000065 | 0.000065 | 0.00065 | 0.0065 |

| | | | | | | | |
|---|---|---|---|---|---|---|---|
| *1 soybean protein: trade name New Fujipro SEH, protein content 87 wt% *2 egg white (powder): protein content 83 wt% | | | | | | | |

**[Table 27-2]**

| <Mixing recipe> | | | | | | | | | | |
|---|---|---|---|---|---|---|---|---|---|---|
| | | sample No. | | | | | | | | |
| | | 12-7 | 12-8 | 12-9 | 12-10 | 12-11 | 12-12 | 12-13 | 12-14 | 12-15 |
| raw material (weight %) | soybean protein (*1) | 10.0 | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 | 90.0 |
| | DENAZYME PMD-P1 | 0.00001 | 0.0001 | 0.001 | 0.003 | 0.01 | 0.10 | 0.30 | 0.50 | 1.0 |
| | total | 100.0 | 100.0 | 100.0 | 100.0 | 100.0 | 100.1 | 100.3 | 100.5 | 101.0 |
| PLD activity (U) for 1 g of protein in sample | | 0.065 | 0.65 | 6.5 | 19.5 | 64.9 | 649.4 | 1948.3 | 3247.1 | 6494.3 |

| | | | | | | | | | | |
|---|---|---|---|---|---|---|---|---|---|---|
| *1 soybean protein: trade name New Fujipro SEH, protein content 87 wt% | | | | | | | | | | |

### [Evaluation criteria (smoothness)]

smoothness: compared to control
5 points: very smooth texture
4 points: smooth texture
3 points: slightly smooth texture
2 points: the same
1 point: gritty texture

**[Table 28-1]**

| | sample No. | | | | | |
|---|---|---|---|---|---|---|
| | 12-1 | 12-2 (control) | 12-3 | 12-4 | 12-5 | 12-6 |
| smoothness | 3 | - | 2.5 | 2.5 | 2.5 | 2.5 |

**[Table 28-2]**

| | sample No. | | | | | | | | |
|---|---|---|---|---|---|---|---|---|---|
| | 12-7 | 12-8 | 12-9 | 12-10 | 12-11 | 12-12 | 12-13 | 12-14 | 12-15 |
| smoothness | 3 | 3 | 3 | 3 | 3 | 3 | 3 | 3 | 2.5 |

From the results of Table 28-1, 28-2, samples 12-3 to sample 12-15 in which a PLD preparation was added such that the enzyme activity for 1 g of protein in the sample was in the range of 0.00000065 to 6494.3 U were improved in smoothness compared to sample 12-2 (control).

### [Experimental Example 13] Confirmation of effect of combined use of phospholipase D and auxiliary materials in soy gel system

Classification of the fauxiliary materials used is shown in Table 29.

Each soy gel (egg white gel in sample 13-1) sample was prepared according to the sample preparation flow shown in Fig. 10, using the mixing recipes shown in Tables 29-1 to 29-41. The prepared samples exhibit the properties of either a suspension or sol or gel.

The obtained each sample was subjected to a sensory evaluation of smoothness by three expert panelists according to the same evaluation criteria as in Experimental Example 12, using sample 13-2 as a control. The results are shown in Tables 30-1 to 30-41.

**[Table 29]**

| auxiliary materials | | |
|---|---|---|
| classification | auxiliary material No. | auxiliary material name |
| A: alkali salt | A1 | sodium carbonate |
| | A2 | trisodium phosphate |
| | A3 | tripotassium phosphate |
| | A4 | trisodium citrate |
| B: calcium salt or calcium oxide | B1 | calcium chloride |
| | B2 | calcinated shell calcium |
| | B3 | calcium lactate |
| | B4 | calcium carbonate |
| C: magnesium salt or oxidation magnesium | C1 | magnesium chloride |
| | C2 | magnesium glutamate |
| D: reducing agent | D1 | glutathione-containing yeast extract |
| | D2 | cysteine-containing yeast extract |
| E: metal ion | E1 | iron-containing yeast |
| | E2 | copper-containing yeast |
| | E3 | manganese-containing yeast |
| F: non-polar amino acid or non-polar amino acid salt | F1 | glycine |
| | F2 | cystine |
| | F3 | alanine |
| | F4 | valine |
| | F5 | leucine |
| | F6 | isoleucine |
| | F7 | phenylalanine |
| | F8 | proline |
| | F9 | methionine |
| G: uncharged amino acid or uncharged amino acid salt | G1 | threonine |
| | G2 | serine |
| | G3 | glutamine |
| | G4 | tyrosine |
| | G5 | cysteine |
| | G6 | cysteine hydrochloride |
| H: basic amino acid or basic amino acid salt | H1 | arginine |
| | H2 | histidine |
| | H3 | lysine hydrochloride |
| I: acidic amino acid or acidic amino acid salt | I1 | sodium aspartate |
| | I2 | sodium glutamate |

**[Table 29-1]**

| <Mixing recipe> unit: wt% | | | |
|---|---|---|---|
| raw material | sample No. | | |
| | 13-1 | 13-2 (control) | 13-3 |
| soybean protein (*1) | | 10.0 | 10.0 |
| egg white (powder) (*2) | 10.0 | | |
| water | 90.0 | 90.0 | 90.0 |
| DENAZYME PMD-P1 (*3) | | | 0.003 |
| total | 100.0 | 100.0 | 100.0 |

| | | | |
|---|---|---|---|
| *1 soybean protein: trade name New Fujipro SEH, protein content 87 wt% *2 egg white (powder): protein content 83 wt% *3 The amount of PLD in sample 13-3 is 19.5 U when converted to enzyme activity for 1 g of protein in the sample. | | | |

**[Table 29-2]**

| <Mixing recipe> unit: wt% | | | | | | | |
|---|---|---|---|---|---|---|---|
| raw material | sample No. | | | | | | |
| | 13-A1-1 | 13-A1-2 | 13-A1-3 | 13-A1-4 | 13-A1-5 | 13-A1-6 | 13-A1-7 |
| soybean protein (*1) | 10.0 | 10.0 | 10.0 | 10.0 | 10.0 | 10.0 | 10.0 |
| water | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 |
| sodium carbonate | 0.1 | 0.000001 | 0.00001 | 0.0001 | 0.001 | 0.01 | 0.1 |
| total | 100.1 | 100.0 | 100.0 | 100.0 | 100.0 | 100.0 | 100.1 |

| | | | | | | | |
|---|---|---|---|---|---|---|---|
| *1 is the same as *1 in the footnote of Table 29-1. *2 The amount of PLD in samples 13-A1-2 to 13-A1-7 is 19.5 U when converted to enzyme activity for 1 g of protein in the sample. | | | | | | | |

**[Table 29-3]**

| <Mixing recipe> unit: wt% | | | | | | | | | |
|---|---|---|---|---|---|---|---|---|---|
| raw material | sample No. | | | | | | | | |
| | 13-A2-1 | 13-A2-2 | 13-A2-3 | 13-A2-4 | 13-A2-5 | 13-A2-6 | 13-A2-7 | 13-A2-8 | 13-A2-9 |
| soybean protein (*1) | 10.0 | 10.0 | 10.0 | 10.0 | 10.0 | 10.0 | 10.0 | 10.0 | 10.0 |
| water | 90.0 | 90.0 | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 | 0.003 | 0.003 |
| trisodium phosphate | 0.1 | 0.00000001 | 0.000001 | 0.00001 | 0.0001 | 0.001 | 0.01 | 0.1 | 0.5 |
| total | 100.1 | 100.0 | 100.0 | 100.0 | 100.0 | 100.0 | 100.0 | 100.1 | 100.5 |

| | | | | | | | | | |
|---|---|---|---|---|---|---|---|---|---|
| *1 is the same as *1 in the footnote of Table 29-1. *2 The amount of PLD in samples 13-A2-2 to 13-A2-9 is 19.5 U when converted to enzyme activity for 1 g of protein in the sample. | | | | | | | | | |

**[Table 29-4]**

| <Mixing recipe> unit: wt% | | | |
|---|---|---|---|
| raw material | sample No. | | |
| | 13-A3-1 | 13-A3-2 | 13-A3-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| tripotassium phosphate | 0.1 | 0.000001 | 0.01 |
| total | 100.1 | 100.0 | 100.0 |

| | | | |
|---|---|---|---|
| *1 is the same as *1 in the footnote of Table 29-1. *2 The amount of PLD in samples 13-A3-2 to 13-A3-3 is 19.5 U when converted to enzyme activity for 1 g of protein in the sample. | | | |

**[Table 29-5]**

| <Mixing recipe> unit: wt% | | |
|---|---|---|
| raw material | sample No. | |
| | 13-A4-1 | 13-A4-2 |
| soybean protein (*1) | 10.0 | 10.0 |
| water | 90.0 | 90.0 |
| DENAZYME PMD-P1 (*2) | | 0.003 |
| trisodium citrate | 0.1 | 0.01 |
| total | 100.1 | 100.0 |

| | | |
|---|---|---|
| *1 is the same as *1 in the footnote of Table 29-1. *2 The amount of PLD in sample 13-A4-2 is 19.5 U when converted to enzyme activity for 1 g of protein in the sample. | | |

**[Table 29-6]**

| <Mixing recipe> unit: wt% | | | | | | | | | |
|---|---|---|---|---|---|---|---|---|---|
| raw material | sample No. | | | | | | | | |
| | 13-B1-1 | 13-B1-2 | 13-B1-3 | 13-B1-4 | 13-B1-5 | 13-B1-6 | 13-B1-7 | 13-B1-8 | 13-B1-9 |
| soybean protein (*1) | 10.0 | 10.0 | 10.0 | 10.0 | 10.0 | 10.0 | 10.0 | 10.0 | 10.0 |
| water | 90.0 | 90.0 | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 | 0.003 | 0.003 |
| calcium chloride | 0.1 | 0.00000001 | 0.000001 | 0.00001 | 0.0001 | 0.001 | 0.01 | 0.1 | 0.5 |
| total | 100.1 | 100.0 | 100.0 | 100.0 | 100.0 | 100.0 | 100.0 | 100.1 | 100.5 |

| | | | | | | | | | |
|---|---|---|---|---|---|---|---|---|---|
| *1 is the same as *1 in the footnote of Table 29-1. *2 The amount of PLD in samples 13-B1-2 to 13-B1-9 is 19.5 U when converted to enzyme activity for 1 g of protein in the sample. | | | | | | | | | |

**[Table 29-7]**

| <Mixing recipe> unit: wt% | | | | | | | |
|---|---|---|---|---|---|---|---|
| raw material | sample No. | | | | | | |
| | 13-B2-1 | 13-B2-2 | 13-B2-3 | 13-B2-4 | 13-B2-5 | 13-B2-6 | 13-B2-7 |
| soybean protein (*1) | 10.0 | 10.0 | 10.0 | 10.0 | 10.0 | 10.0 | 10.0 |
| water | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 |
| calcinated shell calcium | 0.1 | 0.000001 | 0.00001 | 0.0001 | 0.001 | 0.01 | 0.1 |
| total | 100.1 | 100.0 | 100.0 | 100.0 | 100.0 | 100.0 | 100.1 |

| | | | | | | | |
|---|---|---|---|---|---|---|---|
| *1 is the same as *1 in the footnote of Table 29-1. *2 The amount of PLD in samples 13-B2-2 to 13-B2-7 is 19.5 U when converted to enzyme activity for 1 g of protein in the sample. | | | | | | | |

**[Table 29-8]**

| <Mixing recipe> unit: wt% | | | | |
|---|---|---|---|---|
| raw material | sample No. | | | |
| | 13-B3-1 | 13-B3-2 | 13-B3-3 | 13-B3-4 |
| soybean protein (*1) | 10.0 | 10.0 | 10.0 | 10.0 |
| water | 90.0 | 90.0 | 90.0 | 90.0 |
| DENAZYME PMD-P1 (*2) | | | 0.003 | 0.003 |
| calcium lactate | 0.001 | 0.1 | 0.00001 | 0.0001 |
| total | 100.0 | 100.1 | 100.0 | 100.0 |

| | | | | |
|---|---|---|---|---|
| *1 is the same as *1 in the footnote of Table 29-1. *2 The amount of PLD in samples 13-B3-3 to 13-B3-4 is 19.5 U when converted to enzyme activity for 1 g of protein in the sample. | | | | |

**[Table 29-9]**

| <Mixing recipe> unit: wt% | | | |
|---|---|---|---|
| raw material | sample No. | | |
| | 13-B4-1 | 13-B4-2 | 13-B4-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| calcium carbonate | 0.1 | 0.000001 | 0.01 |
| total | 100.1 | 100.0 | 100.0 |

| | | | |
|---|---|---|---|
| *1 is the same as *1 in the footnote of Table 29-1. *2 The amount of PLD in samples 13-B4-2 to 13-B4-3 is 19.5 U when converted to enzyme activity for 1 g of protein in the sample. | | | |

**[Table 29-10]**

| <Mixing recipe> unit: wt% | | | | | | | | |
|---|---|---|---|---|---|---|---|---|
| raw material | sample No. | | | | | | | |
| | 13-C1-1 | 13-C1-2 | 13-C1-3 | 13-C1-4 | 13-C1-5 | 13-C1-6 | 13-C1-7 | 13-C1-8 |
| soybean protein (*1) | 10.0 | 10.0 | 10.0 | 10.0 | 10.0 | 10.0 | 10.0 | 10.0 |
| water | 90.0 | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 | 0.003 |
| magnesium chloride | 0.1 | 0.00000001 | 0.000001 | 0.00001 | 0.0001 | 0.001 | 0.01 | 0.1 |
| total | 100.1 | 100.0 | 100.0 | 100.0 | 100.0 | 100.0 | 100.0 | 100.1 |

| | | | | | | | | |
|---|---|---|---|---|---|---|---|---|
| *1 is the same as *1 in the footnote of Table 29-1. *2 The amount of PLD in samples 13-C1-2 to 13-C1-8 is 19.5 U when converted to enzyme activity for 1 g of protein in the sample. | | | | | | | | |

**[Table 29-11]**

| <Mixing recipe> unit: wt% | | | |
|---|---|---|---|
| raw material | sample No. | | |
| | 13-C2-1 | 13-C2-2 | 13-C2-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| magnesium glutamate | 0.1 | 0.00000001 | 0.01 |
| total | 100.1 | 100.0 | 100.0 |

| | | | |
|---|---|---|---|
| *1 is the same as *1 in the footnote of Table 29-1. *2 The amount of PLD in samples 13-C2-2 to 13-C2-3 is 19.5 U when converted to enzyme activity for 1 g of protein in the sample. | | | |

**[Table 29-12]**

| <Mixing recipe> unit: wt% | | | | | | | | | |
|---|---|---|---|---|---|---|---|---|---|
| raw material | sample No. | | | | | | | | |
| | 13-D1-1 | 13-D1-2 | 13-D1-3 | 13-D1-4 | 13-D1-5 | 13-D1-6 | 13-D1-7 | 13-D1-8 | 13-D1-9 |
| soybean protein (*1) | 10.0 | 10.0 | 10.0 | 10.0 | 10.0 | 10.0 | 10.0 | 10.0 | 10.0 |
| water | 90.0 | 90.0 | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 | 0.003 | 0.003 |
| glutathione-containing yeast extract | 0.1 | 0.00000001 | 0.000001 | 0.00001 | 0.0001 | 0.001 | 0.01 | 0.1 | 0.5 |
| total | 100.1 | 100.0 | 100.0 | 100.0 | 100.0 | 100.0 | 100.0 | 100.1 | 100.5 |

| | | | | | | | | | |
|---|---|---|---|---|---|---|---|---|---|
| *1 is the same as *1 in the footnote of Table 29-1. *2 The amount of PLD in samples 13-D1-2 to 13-D1-9 is 19.5 U when converted to enzyme activity for 1 g of protein in the sample. | | | | | | | | | |

**[Table 29-13]**

| <Mixing recipe> unit: wt% | | | | | | | | | | |
|---|---|---|---|---|---|---|---|---|---|---|
| raw material | sample No. | | | | | | | | | |
| | 13-D2-1 | 13-D2-2 | 13-D2-3 | 13-D2-4 | 13-D2-5 | 13-D2-6 | 13-D2-7 | 13-D2-8 | 13-D2-9 | 13-D2-10 |
| soybean protein (*1) | 10.0 | 10.0 | 10.0 | 10.0 | 10.0 | 10.0 | 10.0 | 10.0 | 10.0 | 10.0 |
| water | 90.0 | 90.0 | 90.0 | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 | 0.003 | 0.003 | 0.003 |
| cysteine-containing yeast extract | 0.1 | 0.0000000001 | 0.00000001 | 0.000001 | 0.00001 | 0.0001 | 0.001 | 0.01 | 0.1 | 0.5 |
| total | 100.1 | 100.0 | 100.0 | 100.0 | 100.0 | 100.0 | 100.0 | 100.0 | 100.1 | 100.5 |

| | | | | | | | | | | |
|---|---|---|---|---|---|---|---|---|---|---|
| *1 is the same as *1 in the footnote of Table 29-1. *2 The amount of PLD in samples 13-D2-2 to 13-D2-10 is 19.5 U when converted to enzyme activity for 1 g of protein in the sample. | | | | | | | | | | |

**[Table 29-14]**

| <Mixing recipe> unit: wt% | | | | | | | | | |
|---|---|---|---|---|---|---|---|---|---|
| raw material | sample No. | | | | | | | | |
| | 13-E1-1 | 13-E1-2 | 13-E1-3 | 13-E1-4 | 13-E1-5 | 13-E1-6 | 13-E1-7 | 13-E1-8 | 13-E1-9 |
| soybean protein (*1) | 10.0 | 10.0 | 10.0 | 10.0 | 10.0 | 10.0 | 10.0 | 10.0 | 10.0 |
| water | 90.0 | 90.0 | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 | 0.003 | 0.003 |
| iron-containing yeast | 0.1 | 0.00000001 | 0.000001 | 0.00001 | 0.0001 | 0.001 | 0.01 | 0.1 | 0.5 |
| total | 100.1 | 100.0 | 100.0 | 100.0 | 100.0 | 100.0 | 100.0 | 100.1 | 100.5 |

| | | | | | | | | | |
|---|---|---|---|---|---|---|---|---|---|
| *1 is the same as *1 in the footnote of Table 29-1. *2 The amount of PLD in samples 13-E1-2 to 13-E1-9 is 19.5 U when converted to enzyme activity for 1 g of protein in the sample. | | | | | | | | | |

**[Table 29-15]**

| <Mixing recipe> unit: wt% | | | | | | |
|---|---|---|---|---|---|---|
| raw material | sample No. | | | | | |
| | 13-E2-1 | 13-E2-2 | 13-E2-3 | 13-E2-4 | 13-E2-5 | 13-E2-6 |
| soybean protein (*1) | 10.0 | 10.0 | 10.0 | 10.0 | 10.0 | 10.0 |
| water | 90.0 | 90.0 | 90.0 | 90.0 | 90.0 | 90.0 |
| DENAZYME PMD-P1 (*2) | | | 0.003 | 0.003 | 0.003 | 0.003 |
| copper-containing yeast | 0.001 | 0.1 | 0.000001 | 0.00001 | 0.0001 | 0.001 |
| total | 100.0 | 100.1 | 100.0 | 100.0 | 100.0 | 100.0 |

| | | | | | | |
|---|---|---|---|---|---|---|
| *1 is the same as *1 in the footnote of Table 29-1. *2 The amount of PLD in samples 13-E2-3 to 13-E2-6 is 19.5 U when converted to enzyme activity for 1 g of protein in the sample. | | | | | | |

**[Table 29-16]**

| <Mixing recipe> unit: wt% | | | | | | | |
|---|---|---|---|---|---|---|---|
| raw material | sample No. | | | | | | |
| | 13-E3-1 | 13-E3-2 | 13-E3-3 | 13-E3-4 | 13-E3-5 | 13-E3-6 | 13-E3-7 |
| soybean protein (*1) | 10.0 | 10.0 | 10.0 | 10.0 | 10.0 | 10.0 | 10.0 |
| water | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 |
| manganese-containing yeast | 0.1 | 0.000001 | 0.00001 | 0.0001 | 0.001 | 0.01 | 0.1 |
| total | 100.1 | 100.0 | 100.0 | 100.0 | 100.0 | 100.0 | 100.1 |

| | | | | | | | |
|---|---|---|---|---|---|---|---|
| *1 is the same as *1 in the footnote of Table 29-1. *2 The amount of PLD in samples 13-E3-2 to 13-E3-7 is 19.5 U when converted to enzyme activity for 1 g of protein in the sample. | | | | | | | |

**[Table 29-17]**

| <Mixing recipe> unit: wt% | | | | | | | |
|---|---|---|---|---|---|---|---|
| raw material | sample No. | | | | | | |
| | 13-F1-1 | 13-F1-2 | 13-F1-3 | 13-F1-4 | 13-F1-5 | 13-F1-6 | 13-F1-7 |
| soybean protein (*1) | 10.0 | 10.0 | 10.0 | 10.0 | 10.0 | 10.0 | 10.0 |
| water | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 |
| glycine | 0.1 | 0.00000001 | 0.000001 | 0.00001 | 0.0001 | 0.001 | 0.01 |
| total | 100.1 | 100.0 | 100.0 | 100.0 | 100.0 | 100.0 | 100.0 |

| | | | | | | | |
|---|---|---|---|---|---|---|---|
| *1 is the same as *1 in the footnote of Table 29-1. *2 The amount of PLD in samples 13-F1-2 to 13-F1-7 is 19.5 U when converted to enzyme activity for 1 g of protein in the sample. | | | | | | | |

**[Table 29-18]**

| <Mixing recipe> unit: wt% | | | | | | | | | |
|---|---|---|---|---|---|---|---|---|---|
| raw material | sample No. | | | | | | | | |
| | 13-F2-1 | 13-F2-2 | 13-F2-3 | 13-F2-4 | 13-F2-5 | 13-F2-6 | 13-F2-7 | 13-F2-8 | 13-F2-9 |
| soybean protein (*1) | 10.0 | 10.0 | 10.0 | 10.0 | 10.0 | 10.0 | 10.0 | 10.0 | 10.0 |
| water | 90.0 | 90.0 | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 | 0.003 | 0.003 |
| cystine | 0.1 | 0.00000001 | 0.000001 | 0.00001 | 0.0001 | 0.001 | 0.01 | 0.1 | 0.5 |
| total | 100.1 | 100.0 | 100.0 | 100.0 | 100.0 | 100.0 | 100.0 | 100.1 | 100.5 |

| | | | | | | | | | |
|---|---|---|---|---|---|---|---|---|---|
| *1 is the same as *1 in the footnote of Table 29-1. *2 The amount of PLD in samples 13-F2-2 to 13-F2-9 is 19.5 U when converted to enzyme activity for 1 g of protein in the sample. | | | | | | | | | |

**[Table 29-19]**

| <Mixing recipe> unit: wt% | | | |
|---|---|---|---|
| raw material | sample No. | | |
| | 13-F3-1 | 13-F3-2 | 13-F3-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| alanine | 0.1 | 0.00000001 | 0.01 |
| total | 100.1 | 100.0 | 100.0 |

| | | | |
|---|---|---|---|
| *1 is the same as *1 in the footnote of Table 29-1. *2 The amount of PLD in samples 13-F3-2 to 13-F3-3 is 19.5 U when converted to enzyme activity for 1 g of protein in the sample. | | | |

**[Table 29-20]**

| <Mixing recipe> unit: wt% | | | |
|---|---|---|---|
| raw material | sample No. | | |
| | 13-F4-1 | 13-F4-2 | 13-F4-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| valine | 0.1 | 0.00000001 | 0.01 |
| total | 100.1 | 100.0 | 100.0 |

| | | | |
|---|---|---|---|
| *1 is the same as *1 in the footnote of Table 29-1. *2 The amount of PLD in samples 13-F4-2 to 13-F4-3 is 19.5 U when converted to enzyme activity for 1 g of protein in the sample. | | | |

**[Table 29-21]**

| <Mixing recipe> unit: wt% | | | |
|---|---|---|---|
| raw material | sample No. | | |
| | 13-F5-1 | 13-F5-2 | 13-F5-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| leucine | 0.1 | 0.00000001 | 0.01 |
| total | 100.1 | 100.0 | 100.0 |

| | | | |
|---|---|---|---|
| *1 is the same as *1 in the footnote of Table 29-1. *2 The amount of PLD in samples 13-F5-2 to 13-F5-3 is 19.5 U when converted to enzyme activity for 1 g of protein in the sample. | | | |

**[Table 29-22]**

| <Mixing recipe> unit: wt% | | | |
|---|---|---|---|
| raw material | sample No. | | |
| | 13-F6-1 | 13-F6-2 | 13-F6-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| isoleucine | 0.1 | 0.0001 | 0.01 |
| total | 100.1 | 100.0 | 100.0 |

| | | | |
|---|---|---|---|
| *1 is the same as *1 in the footnote of Table 29-1. *2 The amount of PLD in samples 13-F6-2 to 13-F6-3 is 19.5 U when converted to enzyme activity for 1 g of protein in the sample. | | | |

**[Table 29-23]**

| <Mixing recipe> unit: wt% | | | | |
|---|---|---|---|---|
| raw material | sample No. | | | |
| | 13-F7-1 | 13-F7-2 | 13-F7-3 | 13-F7-4 |
| soybean protein (*1) | 10.0 | 10.0 | 10.0 | 10.0 |
| water | 90.0 | 90.0 | 90.0 | 90.0 |
| DENAZYME PMD-P1 (*2) | | | 0.003 | 0.003 |
| phenylalanine | 0.001 | 0.1 | 0.000001 | 0.0001 |
| total | 100.0 | 100.1 | 100.0 | 100.0 |

| | | | | |
|---|---|---|---|---|
| *1 is the same as *1 in the footnote of Table 29-1. *2 The amount of PLD in samples 13-F7-3 to 13-F7-4 is 19.5 U when converted to enzyme activity for 1 g of protein in the sample. | | | | |

**[Table 29-24]**

| <Mixing recipe> unit: wt% | | | | |
|---|---|---|---|---|
| raw material | sample No. | | | |
| | 13-F8-1 | 13-F8-2 | 13-F8-3 | 13-F8-4 |
| soybean protein (*1) | 10.0 | 10.0 | 10.0 | 10.0 |
| water | 90.0 | 90.0 | 90.0 | 90.0 |
| DENAZYME PMD-P1 (*2) | | | 0.003 | 0.003 |
| proline | 0.001 | 0.1 | 0.000001 | 0.0001 |
| total | 100.0 | 100.1 | 100.0 | 100.0 |

| | | | | |
|---|---|---|---|---|
| *1 is the same as *1 in the footnote of Table 29-1. *2 The amount of PLD in samples 13-F8-3 to 13-F8-4 is 19.5 U when converted to enzyme activity for 1 g of protein in the sample. | | | | |

**[Table 29-25]**

| <Mixing recipe> unit: wt% | | | |
|---|---|---|---|
| raw material | sample No. | | |
| | 13-F9-1 | 13-F9-2 | 13-F9-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| methionine | 0.1 | 0.000001 | 0.01 |
| total | 100.1 | 100.0 | 100.0 |

| | | | |
|---|---|---|---|
| *1 is the same as *1 in the footnote of Table 29-1. *2 The amount of PLD in samples 13-F9-2 to 13-F9-3 is 19.5 U when converted to enzyme activity for 1 g of protein in the sample. | | | |

**[Table 29-26]**

| <Mixing recipe> unit: wt% | | | | | | | | | |
|---|---|---|---|---|---|---|---|---|---|
| raw material | sample No. | | | | | | | | |
| | 13-G1-1 | 13-G1-2 | 13-G1-3 | 13-G1-4 | 13-G1-5 | 13-G1-6 | 13-G1-7 | 13-G1-8 | 13-G1-9 |
| soybean protein (*1) | 10.0 | 10.0 | 10.0 | 10.0 | 10.0 | 10.0 | 10.0 | 10.0 | 10.0 |
| water | 90.0 | 90.0 | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 | 0.003 | 0.003 |
| threonine | 0.1 | 0.0000000001 | 0.00000001 | 0.000001 | 0.00001 | 0.0001 | 0.001 | 0.01 | 0.1 |
| total | 100.1 | 100.0 | 100.0 | 100.0 | 100.0 | 100.0 | 100.0 | 100.0 | 100.1 |

| | | | | | | | | | |
|---|---|---|---|---|---|---|---|---|---|
| *1 is the same as *1 in the footnote of Table 29-1. *2 The amount of PLD in samples 13-G1-2 to 13-G1-9 is 19.5 U when converted to enzyme activity for 1 g of protein in the sample. | | | | | | | | | |

**[Table 29-27]**

| <Mixing recipe> unit: wt% | | | |
|---|---|---|---|
| raw material | sample No. | | |
| | 13-G2-1 | 13-G2-2 | 13-G2-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| serine | 0.1 | 0.000001 | 0.01 |
| total | 100.1 | 100.0 | 100.0 |

| | | | |
|---|---|---|---|
| *1 is the same as *1 in the footnote of Table 29-1. *2 The amount of PLD in samples 13-G2-2 to 13-G2-3 is 19.5 U when converted to enzyme activity for 1 g of protein in the sample. | | | |

**[Table 29-28]**

| <Mixing recipe> unit: wt% | | | |
|---|---|---|---|
| raw material | sample No. | | |
| | 13-G3-1 | 13-G3-2 | 13-G3-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| glutamine | 0.1 | 0.00000001 | 0.01 |
| total | 100.1 | 100.0 | 100.0 |

| | | | |
|---|---|---|---|
| *1 is the same as *1 in the footnote of Table 29-1. *2 The amount of PLD in samples 13-G3-2 to 13-G3-3 is 19.5 U when converted to enzyme activity for 1 g of protein in the sample. | | | |

**[Table 29-29]**

| <Mixing recipe> unit: wt% | | | | |
|---|---|---|---|---|
| raw material | sample No. | | | |
| | 13-G4-1 | 13-G4-2 | 13-G4-3 | 13-G4-4 |
| soybean protein (*1) | 10.0 | 10.0 | 10.0 | 10.0 |
| water | 90.0 | 90.0 | 90.0 | 90.0 |
| DENAZYME PMD-P1 (*2) | | | 0.003 | 0.003 |
| tyrosine | 0.001 | 0.1 | 0.000001 | 0.0001 |
| total | 100.0 | 100.1 | 100.0 | 100.0 |

| | | | | |
|---|---|---|---|---|
| *1 is the same as *1 in the footnote of Table 29-1. *2 The amount of PLD in samples 13-G4-3 to 13-G4-4 is 19.5 U when converted to enzyme activity for 1 g of protein in the sample. | | | | |

**[Table 29-30]**

| <Mixing recipe> unit: wt% | | | | | |
|---|---|---|---|---|---|
| raw material | sample No. | | | | |
| | 13-G5-1 | 13-G5-2 | 13-G5-3 | 13-G5-4 | 13-G5-5 |
| soybean protein (*1) | 10.0 | 10.0 | 10.0 | 10.0 | 10.0 |
| water | 90.0 | 90.0 | 90.0 | 90.0 | 90.0 |
| DENAZYME PMD-P1 (*2) | | | 0.003 | 0.003 | 0.003 |
| cysteine | 0.001 | 0.1 | 0.00000001 | 0.01 | 0.1 |
| total | 100.0 | 100.1 | 100.0 | 100.0 | 100.1 |

| | | | | | |
|---|---|---|---|---|---|
| *1 is the same as *1 in the footnote of Table 29-1. *2 The amount of PLD in samples 13-G5-3 to 13-G5-5 is 19.5 U when converted to enzyme activity for 1 g of protein in the sample. | | | | | |

**[Table 29-31]**

| <Mixing recipe> unit: wt% | | | | | | | |
|---|---|---|---|---|---|---|---|
| raw material | sample No. | | | | | | |
| | 13-G6-1 | 13-G6-2 | 13-G6-3 | 13-G6-4 | 13-G6-5 | 13-G6-6 | 13-G6-7 |
| soybean protein (*1) | 10.0 | 10.0 | 10.0 | 10.0 | 10.0 | 10.0 | 10.0 |
| water | 90.0 | 90.0 | 90.0 | 90.0 | 90.0 | 90.0 | 90.0 |
| DENAZYME PMD-P1 (*2) | | | 0.003 | 0.003 | 0.003 | 0.003 | 0.003 |
| cysteine hydrochloride | 0.001 | 0.1 | 0.00000001 | 0.000001 | 0.0001 | 0.01 | 0.1 |
| total | 100.0 | 100.1 | 100.0 | 100.0 | 100.0 | 100.0 | 100.1 |

| | | | | | | | |
|---|---|---|---|---|---|---|---|
| *1 is the same as *1 in the footnote of Table 29-1. *2 The amount of PLD in samples 13-G6-3 to 13-G6-7 is 19.5 U when converted to enzyme activity for 1 g of protein in the sample. | | | | | | | |

**[Table 29-32]**

| <Mixing recipe> unit: wt% | | | |
|---|---|---|---|
| raw material | sample No. | | |
| | 13-H1-1 | 13-H1-2 | 13-H1-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| arginine | 0.1 | 0.000001 | 0.0001 |
| total | 100.1 | 100.0 | 100.0 |

| | | | |
|---|---|---|---|
| *1 is the same as *1 in the footnote of Table 29-1. *2 The amount of PLD in samples 13-H1-2 to 13-H1-3 is 19.5 U when converted to enzyme activity for 1 g of protein in the sample. | | | |

**[Table 29-33]**

| <Mixing recipe> unit: wt% | | | |
|---|---|---|---|
| raw material | sample No. | | |
| | 13-H2-1 | 13-H2-2 | 13-H2-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| histidine | 0.1 | 0.000001 | 0.0001 |
| total | 100.1 | 100.0 | 100.0 |

| | | | |
|---|---|---|---|
| *1 is the same as *1 in the footnote of Table 29-1. *2 The amount of PLD in samples 13-H2-2 to 13-H2-3 is 19.5 U when converted to enzyme activity for 1 g of protein in the sample. | | | |

**[Table 29-34]**

| <Mixing recipe> unit: wt% | | | |
|---|---|---|---|
| raw material | sample No. | | |
| | 13-H3-1 | 13-H3-2 | 13-H3-3 |
| soybean protein (*1) | 10.0 | 10.0 | 10.0 |
| water | 90.0 | 90.0 | 90.0 |
| DENAZYME PMD-P1 (*2) | | 0.003 | 0.003 |
| lysine hydrochloride | 0.1 | 0.00000001 | 0.01 |
| total | 100.1 | 100.0 | 100.0 |

| | | | |
|---|---|---|---|
| *1 is the same as *1 in the footnote of Table 29-1. *2 The amount of PLD in samples 13-H3-2 to 13-H3-3 is 19.5 U when converted to enzyme activity for 1 g of protein in the sample. | | | |

**[Table 29-35]**

| <Mixing recipe> unit: wt% | | | | | | | |
|---|---|---|---|---|---|---|---|
| raw material | sample No. | | | | | | |
| | 13-11-1 | 13-11-2 | 13-I1-3 | 13-11-4 | 13-I1-5 | 13-I1-6 | 13-I1-7 |
| soybean protein (*1) | 10.0 | 10.0 | 10.0 | 10.0 | 10.0 | 10.0 | 10.0 |
| water | 90.0 | 90.0 | 90.0 | 90.0 | 90.0 | 90.0 | 90.0 |
| DENAZYME PMD-P1 (*2) | | 0.003 | 0.003 | 0.003 | 0.003 | 0.003 | 0.003 |
| sodium aspartate | 0.1 | 0.00000001 | 0.000001 | 0.00001 | 0.0001 | 0.001 | 0.01 |
| total | 100.1 | 100.0 | 100.0 | 100.0 | 100.0 | 100.0 | 100.0 |

| | | | | | | | |
|---|---|---|---|---|---|---|---|
| *1 is the same as *1 in the footnote of Table 29-1. *2 The amount of PLD in samples 13-I1-2 to 13-I1-7 is 19.5 U when converted to enzyme activity for 1 g of protein in the sample. | | | | | | | |

**[Table 29-36]**

| <Mixing recipe> unit: wt% | | | | |
|---|---|---|---|---|
| raw material | sample No. | | | |
| | 13-I2-1 | 13-I2-2 | 13-I2-3 | 13-I2-4 |
| soybean protein (*1) | 10.0 | 10.0 | 10.0 | 10.0 |
| water | 90.0 | 90.0 | 90.0 | 90.0 |
| DENAZYME PMD-P1 (*2) | | | 0.003 | 0.003 |
| sodium glutamate | 0.001 | 0.1 | 0.000001 | 0.0001 |
| total | 100.0 | 100.1 | 100.0 | 100.0 |

| | | | | |
|---|---|---|---|---|
| *1 is the same as *1 in the footnote of Table 29-1. *2 The amount of PLD in samples 13-I2-3 to 13-I2-4 is 19.5 U when converted to enzyme activity for 1 g of protein in the sample. | | | | |

**[Table 30-1]**

| | sample No. | | |
|---|---|---|---|
| | 13-1 | 13-2 (control) | 13-3 |
| smoothness | 3 | - | 3 |

**[Table 30-2]**

| | sample No. | | | | | | |
|---|---|---|---|---|---|---|---|
| | 13-A1-1 | 13-A1-2 | 13-A1-3 | 13-A1-4 | 13-A1-5 | 13-A1-6 | 13-A1-7 |
| smoothness | 2 | 4 | 5 | 5 | 5 | 5 | 4 |

**[Table 30-3]**

| | sample No. | | | | | | | | |
|---|---|---|---|---|---|---|---|---|---|
| | 13-A2-1 | 13-A2-2 | 13-A2-3 | 13-A2-4 | 13-A2-5 | 13-A2-6 | 13-A2-7 | 13-A2-8 | 13-A2-9 |
| smoothness | 2.5 | 3.5 | 4 | 4 | 5 | 5 | 5 | 5 | 5 |

**[Table 30-4]**

| | sample No. | | |
|---|---|---|---|
| | 13-A3-1 | 13-A3-2 | 13-A3-3 |
| smoothness | 2 | 4 | 4 |

**[Table 30-5]**

| | sample No. | |
|---|---|---|
| | 13-A4-1 | 13-A4-2 |
| smoothness | 2 | 4 |

**[Table 30-6]**

| | sample No. | | | | | | | | |
|---|---|---|---|---|---|---|---|---|---|
| | 13-B1-1 | 13-B1-2 | 13-B1-3 | 13-B1-4 | 13-B1-5 | 13-B1-6 | 13-B1-7 | 13-B1-8 | 13-B1-9 |
| smoothness | 2 | 4 | 5 | 5 | 5 | 5 | 5 | 5 | 5 |

**[Table 30-7]**

| | sample No. | | | | | | |
|---|---|---|---|---|---|---|---|
| | 13-B2-1 | 13-B2-2 | 13-B2-3 | 13-B2-4 | 13-B2-5 | 13-B2-6 | 13-B2-7 |
| smoothness | 2 | 4 | 5 | 5 | 5 | 5 | 4 |

**[Table 30-8]**

| | sample No. | | | |
|---|---|---|---|---|
| | 13-B3-1 | 13-B3-2 | 13-B3-3 | 13-B3-4 |
| smoothness | 2 | 2 | 4 | 4 |

**[Table 30-9]**

| | sample No. | | |
|---|---|---|---|
| | 13-B4-1 | 13-B4-2 | 13-B4-3 |
| smoothness | 2.5 | 4 | 3.5 |

**[Table 30-10]**

| | sample No. | | | | | | | |
|---|---|---|---|---|---|---|---|---|
| | 13-C1-1 | 13-C1-2 | 13-C1-3 | 13-C1-4 | 13-C1-5 | 13-C1-6 | 13-C1-7 | 13-C1-8 |
| smoothness | 2 | 3.5 | 4 | 5 | 5 | 5 | 4 | 3.5 |

**[Table 30-11]**

| | sample No. | | |
|---|---|---|---|
| | 13-C2-1 | 13-C2-2 | 13-C2-3 |
| smoothness | 2 | 3.5 | 4 |

**[Table 30-12]**

| | sample No. | | | | | | | | |
|---|---|---|---|---|---|---|---|---|---|
| | 13-D1-1 | 13-D1-2 | 13-D1 | 13-D1-4 | 13-D1-5 | 13-D1-6 | 13-D1-7 | 13-D1-8 | 13-D1-9 |
| smoothness | 3 | 3.5 | 3.5 | 5 | 5 | 5 | 5 | 5 | 5 |

**[Table 30-13]**

| | sample No. | | | | | | | | | |
|---|---|---|---|---|---|---|---|---|---|---|
| | 13-D2-1 | 13-D2-2 | 13-D2-3 | 13-D2-4 | 13-D2-5 | 13-D2-6 | 13-D2-7 | 13-D2-8 | 13-D2-9 | 13-D2-10 |
| smoothness | 3 | 3.5 | 4 | 5 | 5 | 5 | 5 | 4 | 4 | 5 |

**[Table 30-14]**

| | sample No. | | | | | | | | |
|---|---|---|---|---|---|---|---|---|---|
| | 13-E1-1 | 13-E1-2 | 13-E1-3 | 13-E1-4 | 13-E1-5 | 13-E1-6 | 13-E1-7 | 13-E1-8 | 13-E1-9 |
| smoothness | 3 | 3.5 | 4.5 | 5 | 5 | 5 | 5 | 5 | 5 |

**[Table 30-15]**

| | sample No. | | | | | |
|---|---|---|---|---|---|---|
| | 13-E2-1 | 13-E2-2 | 13-E2-3 | 13-E2-4 | 13-E2-5 | 13-E2-6 |
| smoothness | 2 | 1 | 4 | 4 | 5 | 4 |

**[Table 30-16]**

| | sample No. | | | | | | |
|---|---|---|---|---|---|---|---|
| | 13-E3-1 | 13-E3-2 | 13-E3-3 | 13-E3-4 | 13-E3-5 | 13-E3-6 | 13-E3-7 |
| smoothness | 2 | 4 | 4 | 4 | 4 | 4 | 4 |

**[Table 30-17]**

| | sample No. | | | | | | |
|---|---|---|---|---|---|---|---|
| | 13-F1-1 | 13-F1-2 | 13-F1-3 | 13-F1-4 | 13-F1-5 | 13-F1-6 | 13-F1-7 |
| smoothness | 2 | 3.5 | 4 | 4 | 5 | 5 | 4 |

**[Table 30-18]**

| | sample No. | | | | | | | | |
|---|---|---|---|---|---|---|---|---|---|
| | 13-F2-1 | 13-F2-2 | 13-F2-3 | 13-F2-4 | 13-F2-5 | 13-F2-6 | 13-F2-7 | 13-F2-8 | 13-F2-9 |
| smoothness | 3 | 4 | 5 | 5 | 5 | 5 | 5 | 5 | 5 |

**[Table 30-19]**

| | sample No. | | |
|---|---|---|---|
| | 13-F3-1 | 13-F3-2 | 13-F3-3 |
| smoothness | 2 | 3.5 | 4 |

**[Table 30-20]**

| | sample No. | | |
|---|---|---|---|
| | 13-F4-1 | 13-F4-2 | 13-F4-3 |
| smoothness | 2 | 3.5 | 4 |

**[Table 30-21]**

| | sample No. | | |
|---|---|---|---|
| | 13-F5-1 | 13-F5-2 | 13-F5-3 |
| smoothness | 2 | 4 | 4 |

**[Table 30-22]**

| | sample No. | | |
|---|---|---|---|
| | 13-F6-1 | 13-F6-2 | 13-F6-3 |
| smoothness | 2 | 4 | 3.5 |

**[Table 30-23]**

| | sample No. | | | |
|---|---|---|---|---|
| | 13-F7-1 | 13-F7-2 | 13-F7-3 | 13-F7-4 |
| smoothness | 2 | 2 | 3.5 | 4 |

**[Table 30-24]**

| | sample No. | | | |
|---|---|---|---|---|
| | 13-F8-1 | 13-F8-2 | 13-F8-3 | 13-F8-4 |
| smoothness | 2 | 2 | 3.5 | 4 |

**[Table 30-25]**

| | sample No. | | |
|---|---|---|---|
| | 13-F9-1 | 13-F9-2 | 13-F9-3 |
| smoothness | 2 | 3.5 | 3.5 |

**[Table 30-26]**

| | sample No. | | | | | | | | |
|---|---|---|---|---|---|---|---|---|---|
| | 13-G1-1 | 13-G1-2 | 13-G1-3 | 13-G1-4 | 13-G1-5 | 13-G1-6 | 13-G1-7 | 13-G1-8 | 13-G1-9 |
| smoothness | 2 | 4 | 4 | 5 | 5 | 5 | 5 | 5 | 5 |

**[Table 30-27]**

| | sample No. | | |
|---|---|---|---|
| | 13-G2-1 | 13-G2-2 | 13-G2-3 |
| smoothness | 2 | 4 | 3.5 |

**[Table 30-28]**

| | sample No. | | |
|---|---|---|---|
| | 13-G3-1 | 13-G3-2 | 13-G3-3 |
| smoothness | 2 | 4 | 3.5 |

**[Table 30-29]**

| | sample No. | | |
|---|---|---|---|
| | 13-G4-1 | 13-G4-2 | 13-G4-3 13-G4-4 |
| smoothness | 2 | 2 | 3.5 3.5 |

**[Table 30-30]**

| | sample No. | | | | |
|---|---|---|---|---|---|
| | 13-G5-1 | 13-G5-2 | 13-G5-3 | 13-G5-4 | 13-G5-5 |
| smoothness | 3 | liquid and not evaluable | 4.5 | 5 | 5 |

**[Table 30-31]**

| | sample No. | | | | | | |
|---|---|---|---|---|---|---|---|
| | 13-G6-1 | 13-G6-2 | 13-G6-3 | 13-G6-4 | 13-G6-5 | 13-G6-6 | 13-G6-7 |
| smoothness | 3 | liquid and not evaluable | 4 | 5 | 5 | 5 | 5 |

**[Table 30-32]**

| | sample No. | | |
|---|---|---|---|
| | 13-H1-1 | 13-H1-2 | 13-H1-3 |
| smoothness | 2 | 4 | 4 |

**[Table 30-33]**

| | sample No. | | |
|---|---|---|---|
| | 13-H2-1 | 13-H2-2 | 13-H2-3 |
| smoothness | 2 | 4 | 4 |

**[Table 30-34]**

| | sample No. | | |
|---|---|---|---|
| | 13-H3-1 | 13-H3-2 | 13-H3-3 |
| smoothness | 2 | 3.5 | 4 |

**[Table 30-35]**

| | sample No. | | | | | | |
|---|---|---|---|---|---|---|---|
| | 13-I1-1 | 13-I1-2 | 13-I1-3 | 13-I1-4 | 13-I1-5 | 13-I1-6 | 13-I1-7 |
| smoothness | 2 | 3.5 | 4 | 5 | 5 | 5 | 5 |

**[Table 30-36]**

| | sample No. | | | |
|---|---|---|---|---|
| | 13-I2-1 | 13-I2-2 | 13-I2-3 | 13-I2-4 |
| smoothness | 2 | 2 | 3.5 | 3.5 |

From the results of Table 30-2, samples 13-A1-2 to 13-A1-7, with the addition of PLD and sodium carbonate, were improved in smoothness compared to sample 13-2 (control). In addition, samples 13-A1-2 to 13-A1-7 were improved in smoothness compared to sample 13-3 (see Table 30-1) with the addition of PLD but without addition of sodium carbonate.

From the results of Table 30-3, samples 13-A2-2 to 13-A2-9, with the addition of PLD and trisodium phosphate, were improved in smoothness compared to sample 13-2 (control). In addition, samples 13-A2-2 to 13-A2-9 were improved in smoothness compared to sample 13-3 (see Table 30-1) with the addition of PLD but without addition of trisodium phosphate.

From the results of Table 30-4, samples 13-A3-2 to 13-A3-3, with the addition of PLD and tripotassium phosphate, were improved in smoothness compared to sample 13-2 (control). In addition, samples 13-A3-2 to 13-A3-3 were improved in smoothness compared to sample 13-3 (see Table 30-1) with the addition of PLD but without addition of tripotassium phosphate.

From the results of Table 30-5, sample 13-A4-2, with the addition of PLD and trisodium citrate, was improved in smoothness compared to sample 13-2 (control). In addition, sample 13-A4-2 was improved in smoothness compared to sample 13-3 (see Table 30-1) with the addition of PLD but without addition of trisodium citrate.

From the results of Table 30-6, samples 13-B1-2 to 13-B1-9, with the addition of PLD and calcium chloride, were improved in smoothness compared to sample 13-2 (control). In addition, samples 13-B1-2 to 13-B1-9 were improved in smoothness compared to sample 13-3 (see Table 30-1) with the addition of PLD but without addition of calcium chloride.

From the results of Table 30-7, samples 13-B2-2 to 13-B2-7, with the addition of PLD and calcinated shell calcium, were improved in smoothness compared to sample 13-2 (control). In addition, samples 13-B2-2 to 13-B2-7 were improved in smoothness compared to sample 13-3 (see Table 30-1) with the addition of PLD but without addition of calcinated shell calcium.

From the results of Table 30-8, samples 13-B3-3 to 13-B3-4, with the addition of PLD and calcium lactate, were improved in smoothness compared to sample 13-2 (control). In addition, samples 13-B3-3 to 13-B3-4 were improved in smoothness compared to sample 13-3 (see Table 30-1) with the addition of PLD but without addition of calcium lactate.

From the results of Table 30-9, samples 13-B4-2 to 13-B4-3, with the addition of PLD and calcium carbonate, were improved in smoothness compared to sample 13-2 (control). In addition, samples 13-B4-2 to 13-B4-3 were improved in smoothness compared to sample 13-3 (see Table 30-1) with the addition of PLD but without addition of calcium carbonate.

From the results of Table 30-10, samples 13-C1-2 to 13-C1-8, with the addition of PLD and magnesium chloride, were improved in smoothness compared to sample 13-2 (control). In addition, samples 13-C1-2 to 13-C1-8 were improved in smoothness compared to sample 13-3 (see Table 30-1) with the addition of PLD but without addition of magnesium chloride.

From the results of Table 30-11, samples 13-C2-2 to 13-C2-3, with the addition of PLD and magnesium glutamate, were improved in smoothness compared to sample 13-2 (control). In addition, samples 13-C2-2 to 13-C2-3 were improved in smoothness compared to sample 13-3 (see Table 30-1) with the addition of PLD but without addition of magnesium glutamate.

From the results of Table 30-12, samples 13-D1-2 to 13-D1-9, with the addition of PLD and glutathione-containing yeast extract, were improved in smoothness compared to sample 13-2 (control). In addition, samples 13-D1-2 to 13-D1-9 were improved in smoothness compared to sample 13-3 (see Table 30-1) with the addition of PLD but without addition of glutathione-containing yeast extract.

From the results of Table 30-13, samples 13-D2-2 to 13-D2-10, with the addition of PLD and cysteine-containing yeast extract, were improved in smoothness compared to sample 13-2 (control). In addition, samples 13-D2-2 to 13-D2-10 were improved in smoothness compared to sample 13-3 (see Table 30-1) with the addition of PLD but without addition of cysteine-containing yeast extract.

From the results of Table 30-14, samples 13-E1-2 to 13-E1-9, with the addition of PLD and iron-containing yeast, were improved in smoothness compared to sample 13-2 (control). In addition, samples 13-E1-2 to 13-E1-9 were improved in smoothness compared to sample 13-3 (see Table 30-1) with the addition of PLD but without addition of iron-containing yeast.

From the results of Table 30-15, samples 13-E2-3 to 13-E2-6, with the addition of PLD and copper-containing yeast, were improved in smoothness compared to sample 13-2 (control). In addition, samples 13-E2-3 to 13-E2-6 were improved in smoothness compared to sample 13-3 (see Table 30-1) with the addition of PLD but without addition of copper-containing yeast.

From the results of Table 30-16, samples 13-E3-2 to 13-E3-7, with the addition of PLD and manganese-containing yeast, were improved in smoothness compared to sample 13-2 (control). In addition, samples 13-E3-2 to 13-E3-7 were improved in smoothness compared to sample 13-3 (see Table 30-1) with the addition of PLD but without addition of manganese-containing yeast.

From the results of Table 30-17, samples 13-F1-2 to 13-F1-7, with the addition of PLD and glycine, were improved in smoothness compared to sample 13-2 (control). In addition, samples 13-F1-2 to 13-F1-7 were improved in smoothness compared to sample 13-3 (see Table 30-1) with the addition of PLD but without addition of glycine.

From the results of Table 30-18, samples 13-F2-2 to 13-F2-9, with the addition of PLD and cystine, were improved in smoothness compared to sample 13-2 (control). In addition, samples 13-F2-2 to 13-F2-9 were improved in smoothness compared to sample 13-3 (see Table 30-1) with the addition of PLD but without addition of cystine.

From the results of Table 30-19, samples 13-F3-2 to 13-F3-3, with the addition of PLD and alanine, were improved in smoothness compared to sample 13-2 (control). In addition, samples 13-F3-2 to 13-F3-3 were improved in smoothness compared to sample 13-3 (see Table 30-1) with the addition of PLD but without addition of alanine.

From the results of Table 30-20, samples 13-F4-2 to 13-F4-3, with the addition of PLD and valine, were improved in smoothness compared to sample 13-2 (control). In addition, samples 13-F4-2 to 13-F4-3 were improved in smoothness compared to sample 13-3 (see Table 30-1) with the addition of PLD but without addition of valine.

From the results of Table 30-21, samples 13-F5-2 to 13-F5-3, with the addition of PLD and leucine, were improved in smoothness compared to sample 13-2 (control). In addition, samples 13-F5-2 to 13-F5-3 were improved in smoothness compared to sample 13-3 (see Table 30-1) with the addition of PLD but without addition of leucine.

From the results of Table 30-22, samples 13-F6-2 to 13-F6-3, with the addition of PLD and isoleucine, were improved in smoothness compared to sample 13-2 (control). In addition, samples 13-F6-2 to 13-F6-3 were improved in smoothness compared to sample 13-3 (see Table 30-1) with the addition of PLD but without addition of isoleucine.

From the results of Table 30-23, samples 13-F7-3 to 13-F7-4, with the addition of PLD and phenylalanine, were improved in smoothness compared to sample 13-2 (control). In addition, samples 13-F7-3 to 13-F7-4 were improved in smoothness compared to sample 13-3 (see Table 30-1) with the addition of PLD but without addition of phenylalanine.

From the results of Table 30-24, samples 13-F8-3 to 13-F8-4, with the addition of PLD and proline, were improved in smoothness compared to sample 13-2 (control). In addition, samples 13-F8-3 to 13-F8-4 were improved in smoothness compared to sample 13-3 (see Table 30-1) with the addition of PLD but without addition of proline.

From the results of Table 30-25, samples 13-F9-2 to 13-F9-3, with the addition of PLD and methionine, were improved in smoothness compared to sample 13-2 (control). In addition, samples 13-F9-2 to 13-F9-3 were improved in smoothness compared to sample 13-3 (see Table 30-1) with the addition of PLD but without addition of methionine.

From the results of Table 30-26, samples 13-G1-2 to 13-G1-9, with the addition of PLD and threonine, were improved in smoothness compared to sample 13-2 (control). In addition, samples 13-G1-2 to 13-G1-9 were improved in smoothness compared to sample 13-3 (see Table 30-1) with the addition of PLD but without addition of threonine.

From the results of Table 30-27, samples 13-G2-2 to 13-G2-3, with the addition of PLD and serine, were improved in smoothness compared to sample 13-2 (control). In addition, samples 13-G2-2 to 13-G2-3 were improved in smoothness compared to sample 13-3 (see Table 30-1) with the addition of PLD but without addition of serine.

From the results of Table 30-28, samples 13-G3-2 to 13-G3-3, with the addition of PLD and glutamine, were improved in smoothness compared to sample 13-2 (control). In addition, samples 13-G3-2 to 13-G3-3 were improved in smoothness compared to sample 13-3 (see Table 30-1) with the addition of PLD but without addition of glutamine.

From the results of Table 30-29, samples 13-G4-3 to 13-G4-4, with the addition of PLD and tyrosine, were improved in smoothness compared to sample 13-2 (control). In addition, samples 13-G4-3 to 13-G4-4 were improved in smoothness compared to sample 13-3 (see Table 30-1) with the addition of PLD but without addition of tyrosine.

From the results of Table 30-30, samples 13-G5-3 to 13-G5-5, with the addition of PLD and cysteine, were improved in smoothness compared to sample 13-2 (control). In addition, samples 13-G5-3 to 13-G5-5 were improved in smoothness compared to sample 13-3 (see Table 30-1) with the addition of PLD but without addition of cysteine.

From the results of Table 30-31, samples 13-G6-3 to 13-G6-7, with the addition of PLD and cysteine hydrochloride, were improved in smoothness compared to sample 13-2 (control). In addition, samples 13-G6-3 to 13-G6-7 were improved in smoothness compared to sample 13-3 (see Table 30-1) with the addition of PLD but without addition of cysteine hydrochloride.

From the results of Table 30-32, samples 13-H1-2 to 13-H1-3, with the addition of PLD and arginine, were improved in smoothness compared to sample 13-2 (control). In addition, samples 13-H1-2 to 13-H1-3 were improved in smoothness compared to sample 13-3 (see Table 30-1) with the addition of PLD but without addition of arginine.

From the results of Table 30-33, samples 13-H2-2 to 13-H2-3, with the addition of PLD and histidine, were improved in smoothness compared to sample 13-2 (control). In addition, samples 13-H2-2 to 13-H2-3 were improved in smoothness compared to sample 13-3 (see Table 30-1) with the addition of PLD but without addition of histidine.

From the results of Table 30-34, samples 13-H3-2 to 13-H3-3, with the addition of PLD and lysine hydrochloride, were improved in smoothness compared to sample 13-2 (control). In addition, samples 13-H3-2 to 13-H3-3 were improved in smoothness compared to sample 13-3 (see Table 30-1) with the addition of PLD but without addition of lysine hydrochloride.

From the results of Table 30-35, samples 13-I1-2 to 13-I1-7, with the addition of PLD and sodium aspartate, were improved in smoothness compared to sample 13-2 (control). In addition, samples 13-I1-2 to 13-I1-7 were improved in smoothness compared to sample 13-3 (see Table 30-1) with the addition of PLD but without addition of sodium aspartate.

From the results of Table 30-36, samples 13-I2-3 to 13-I2-4, with the addition of PLD and sodium glutamate, were improved in smoothness compared to sample 13-2 (control). In addition, samples 13-I2-3 to 13-I2-4 were improved in smoothness compared to sample 13-3 (see Table 30-1) with the addition of PLD but without addition of sodium glutamate.

### [Experimental Example 14] Confirmation of (1) effect of combined use of phospholipase D and ascorbic acid oxidase, and (2) effect of combined use of phospholipase D and glucose oxidase, in soy gel system

Each soy gel (egg white gel in sample 14-1) sample was prepared according to the sample preparation flow shown in Fig. 10, using the mixing recipes shown in Tables 31-1 to 31-9. The prepared samples exhibit the properties of either a suspension or sol or gel.

The obtained each sample was subjected to a sensory evaluation of smoothness by three expert panelists according to the same evaluation criteria as in Experimental Example 12, using sample 14-2 as a control. The results are shown in Tables 32-1 to 32-5.

**[Table 31-1]**

| <Mixing recipe> | | | | |
|---|---|---|---|---|
| | | sample No. | | |
| | | 14-1 | 14-2 (control) | 14-3 |
| raw material (weight %) | soybean protein (*1) | | 10.0 | 10.0 |
| | egg white (powder) (*2) | 10.0 | | |
| | water | 90.0 | 90.0 | 90.0 |
| | DENAZYME PMD-P1(*3) | | | 0.003 |
| | total | 100.0 | 100.0 | 100.0 |

| | | | | |
|---|---|---|---|---|
| *1 soybean protein: trade name New Fujipro SEH, protein content 87 wt% *2 egg white (powder): protein content 83 wt% *3 The amount of PLD in sample 14-3 is 19.5 U when converted to enzyme activity for 1 g of protein in the sample. | | | | |

**[Table 31-2]**

| (A) PLD×sodium ascorbate×ascorbic acid oxidase (study of the amount of sodium ascorbate) | | | | | |
|---|---|---|---|---|---|
| <Mixing recipe> | | | | | |
| | | sample No. | | | |
| | | 14A-4 | 14A-5 | 14A-6 | 14A-7 |
| raw material (weight %) | soybean protein (*1) | 10.0 | 10.0 | 10.0 | 10.0 |
| | water | 90.0 | 90.0 | 90.0 | 90.0 |
| | DENAZYME PMD-P1 (*2) | | | 0.003 | 0.003 |
| | sodium L-ascorbate | 0.10 | 0.10 | 0.0000000001 | 0.00000001 |
| | ascorbic acid oxidase | | 0.10 | 0.10 | 0.10 |
| | total | 100.1 | 100.2 | 100.1 | 100.1 |
| ascorbic acid oxidase activity (U) for 1 g of sodium L-ascorbate content (converted to L-ascorbic acid) in sample | | | 1200.0 | 1200000000000.0 | 12000000000.0 |

| | | | | | |
|---|---|---|---|---|---|
| *1 soybean protein: trade name New Fujipro SEH, protein content 87 wt% *2 The amount of PLD in samples 14A-6 to 14A-7 is 19.5 U when converted to enzyme activity for 1 g of protein in the sample. | | | | | |

**[Table 31-3]**

| (A) PLD×sodium ascorbate×ascorbic acid oxidase (study of the amount of sodium ascorbate) | | | | | | | | |
|---|---|---|---|---|---|---|---|---|
| <Mixing recipe> | | | | | | | | |
| | | sample No. | | | | | | |
| | | 14A-8 | 14A-9 | 14A-10 | 14A-11 | 14A-12 | 14A-13 | 14A-14 |
| raw material (weight %) | soybean protein (*1) | 10.0 | 10.0 | 10.0 | 10.0 | 10.0 | 10.0 | 10.0 |
| | water | 90.0 | 90.0 | 90.0 | 90.0 | 90.0 | 90.0 | 90.0 |
| | DENAZYME PMD-P1 (*2) | 0.003 | 0.003 | 0.003 | 0.003 | 0.003 | 0.003 | 0.003 |
| | sodium L-ascorbate | 0.000001 | 0.00001 | 0.0001 | 0.001 | 0.01 | 0.10 | 0.50 |
| | ascorbic acid oxidase | 0.10 | 0.10 | 0.10 | 0.10 | 0.10 | 0.10 | 0.10 |
| | total | 100.1 | 100.1 | 100.1 | 100.1 | 100.1 | 100.2 | 100.6 |
| ascorbic acid oxidase activity (U) for 1 g of sodium L-ascorbate content (converted to L-ascorbic acid) in sample | | 120000000.0 | 12000000.0 | 1200000.0 | 120000.0 | 12000.0 | 1200.0 | 240.0 |

| | | | | | | | | |
|---|---|---|---|---|---|---|---|---|
| *1 soybean protein: trade name New Fujipro SEH, protein content 87 wt% *2 The amount of PLD in samples 14A-8 to 14A-14 is 19.5 U when converted to enzyme activity for 1 g of protein in the sample. | | | | | | | | |

**[Table 31-4]**

| (B) PLD×sodium ascorbate×ascorbic acid oxidase (study of the amount of ascorbic acid oxidase) | | | | | |
|---|---|---|---|---|---|
| <Mixing recipe> | | | | | |
| | | sample No. | | | |
| | | 14B-4 | 14B-5 | 14B-6 | 14B-7 |
| raw material (weight %) | soybean protein (*1) | 10.0 | 10.0 | 10.0 | 10.0 |
| | water | 90.0 | 90.0 | 90.0 | 90.0 |
| | DENAZYME PMD-P1 (*2) | | | 0.003 | 0.003 |
| | sodium L-ascorbate | | 0.10 | 0.10 | 0.10 |
| | ascorbic acid oxidase | 0.10 | 0.10 | 0.0000000001 | 0.00000001 |
| | total | 100.1 | 100.2 | 100.1 | 100.1 |
| ascorbic acid oxidase activity (U) for 1 g of sodium L-ascorbate content (converted to L-ascorbic acid) in sample | | | 1200.0 | 0.0000012 | 0.00012 |

| | | | | | |
|---|---|---|---|---|---|
| *1 soybean protein: trade name New Fujipro SEH, protein content 87 wt% *2 The amount of PLD in samples 14B-6 to 14B-7 is 19.5 U when converted to enzyme activity for 1 g of protein in the sample. | | | | | |

**[Table 31-5]**

| (B) PLD×sodium ascorbate×ascorbic acid oxidase (study of the amount of ascorbic acid oxidase) | | | | | | | | |
|---|---|---|---|---|---|---|---|---|
| <Mixing recipe> | | | | | | | | |
| | | sample No. | | | | | | |
| | | 14B-8 | 14B-9 | 14B-10 | 14B-11 | 14B-12 | 14B-13 | 14B-14 |
| raw material (weight %) | soybean protein (*1) | 10.0 | 10.0 | 10.0 | 10.0 | 10.0 | 10.0 | 10.0 |
| | water | 90.0 | 90.0 | 90.0 | 90.0 | 90.0 | 90.0 | 90.0 |
| | DENAZYME PMD-P1 (*2) | 0.003 | 0.003 | 0.003 | 0.003 | 0.003 | 0.003 | 0.003 |
| | sodium L-ascorbate | 0.10 | 0.10 | 0.10 | 0.10 | 0.10 | 0.10 | 0.10 |
| | ascorbic acid oxidase | 0.000001 | 0.00001 | 0.0001 | 0.001 | 0.01 | 0.10 | 0.50 |
| | total | 100.1 | 100.1 | 100.1 | 100.1 | 100.1 | 100.2 | 100.6 |
| ^{.}ascorbic acid oxidase activity (U) for 1 g of sodium L-ascorbate content (converted to L-ascorbic acid) in sample | | 0.012 | 0.12 | 1.2 | 12.0 | 120.0 | 1200.0 | 6000.0 |

| | | | | | | | | |
|---|---|---|---|---|---|---|---|---|
| *1 soybean protein: trade name New Fujipro SEH, protein content 87 wt% *2 The amount of PLD in samples 14B-8 to 14B-14 is 19.5 U when converted to enzyme activity for 1 g of protein in the sample. | | | | | | | | |

**[Table 31-6]**

| (C) PLD×glucose×glucose oxidase (study of the amount of glucose) | | | | | |
|---|---|---|---|---|---|
| <Mixing recipe> | | | | | |
| | | sample No. | | | |
| | | 14C-4 | 14C-5 | 14C-6 | 14C-7 |
| raw material (weight %) | soybean protein (*1) | 10.0 | 10.0 | 10.0 | 10.0 |
| | water | 90.0 | 90.0 | 90.0 | 90.0 |
| | DENAZYME PMD-P1 (*2) | | | 0.003 | 0.003 |
| | glucose | 0.10 | 0.10 | 0.00000001 | 0.000001 |
| | glucose oxidase | | 0.10 | 0.10 | 0.10 |
| | total | 100.1 | 100.2 | 100.1 | 100.1 |
| glucose oxidase activity (U) for 1 g of glucose in sample | | | 2150.0 | 21500000000.0 | 215000000.0 |

| | | | | | |
|---|---|---|---|---|---|
| *1 soybean protein: trade name New Fujipro SEH, protein content 87 wt% The amount of PLD in samples 14C-6 to 14C-7 is 19.5 U when converted to enzyme activity for 1 g of protein in the sample. | | | | | |

**[Table 31-7]**

| (C) PLD×glucose×glucose oxidase (study of the amount of glucose) | | | | | |
|---|---|---|---|---|---|
| <Mixing recipe> | | | | | |
| | | sample No. | | | |
| | | 14C-8 | 14C-9 | 14C-10 | 14C-11 |
| raw material (weight %) | soybean protein (*1) | 10.0 | 10.0 | 10.0 | 10.0 |
| | water | 90.0 | 90.0 | 90.0 | 90.0 |
| | DENAZYME PMD-P1 (*2) | 0.003 | 0.003 | 0.003 | 0.003 |
| | glucose | 0.00001 | 0.0001 | 0.001 | 0.01 |
| | glucose oxidase | 0.10 | 0.10 | 0.10 | 0.10 |
| | total | 100.1 | 100.1 | 100.1 | 100.1 |
| glucose oxidase activity (U) for 1 g of glucose in sample | | 21500000.0 | 2150000.0 | 215000.0 | 21500.0 |

| | | | | | |
|---|---|---|---|---|---|
| *1 soybean protein: trade name New Fujipro SEH, protein content 87 wt% *2 The amount of PLD in samples 14C-8 to 14C-11 is 19.5 U when converted to enzyme activity for 1 g of protein in the sample. | | | | | |

**[Table 31-8]**

| (D) PLD×glucose×glucose oxidase (study of the amount of glucose oxidase) | | | | | |
|---|---|---|---|---|---|
| <Mixing recipe> | | | | | |
| | | sample No. | | | |
| | | 14D-4 | 14D-5 | 14D-6 | 14D-7 |
| raw material (weight %) | soybean protein (*1) | 10.0 | 10.0 | 10.0 | 10.0 |
| | water | 90.0 | 90.0 | 90.0 | 90.0 |
| | DENAZYME PMD-P1 (*2) | | | 0.003 | 0.003 |
| | glucose | | 0.10 | 0.10 | 0.10 |
| | glucose oxidase | 0.10 | 0.10 | 0.0000000001 | 0.00000001 |
| | total | 100.1 | 100.2 | 100.1 | 100.1 |
| glucose oxidase activity (U) for 1 g of glucose in sample | | | 2150.0 | 0.0000022 | 0.00022 |

| | | | | | |
|---|---|---|---|---|---|
| *1 soybean protein: trade name New Fujipro SEM, protein content 87 wt% *2 The amount of PLD in samples 14D-6 to 14D-7 is 19.5 U when converted to enzyme activity for 1 g of protein in the sample. | | | | | |

**[Table 31-9]**

| (D) PLD×glucose×glucose oxidase (study of the amount of glucose oxidase) | | | | | |
|---|---|---|---|---|---|
| <Mixing recipe> | | | | | |
| | | sample No. | | | |
| | | 14D-8 | 14D-9 | 14D-10 | 14D-11 |
| raw material (weight %) | soybean protein (*1) | 10.0 | 10.0 | 10.0 | 10.0 |
| | water | 90.0 | 90.0 | 90.0 | 90.0 |
| | DENAZYME PMD-P1 (*2) | 0.003 | 0.003 | 0.003 | 0.003 |
| | glucose | 0.10 | 0.10 | 0.10 | 0.10 |
| | glucose oxidase | 0.000001 | 0.00001 | 0.0001 | 0.001 |
| | total | 100.1 | 100.1 | 100.1 | 100.1 |
| glucose oxidase activity (U) for 1 g of glucose in sample | | 0.022 | 0.22 | 2.2 | 21.5 |

| | | | | | |
|---|---|---|---|---|---|
| *1 soybean protein: trade name New Fujipro SEH, protein content 87 wt% *2 The amount of PLD in samples 14D-8 to 14D-14 is 19.5 U when converted to enzyme activity for 1 g of protein in the sample. | | | | | |

**[Table 32-1]**

| | sample No. | | |
|---|---|---|---|
| | 14-1 | 14-2 | 14-3 |
| smoothness | 3 | - | 3 |

**[Table 32-2]**

| | sample No. | | | | | | | | | | |
|---|---|---|---|---|---|---|---|---|---|---|---|
| | 14A-4 | 14A-5 | 14A-6 | 14A-7 | 14A-8 | 14A-9 | 14A-10 | 14A-11 | 14A-12 | 14A-13 | 14A-14 |
| smoothness | 3 | 3 | 3.5 | 5 | 5 | 5 | 5 | 5 | 5 | 5 | 4 |

**[Table 32-3]**

| | sample No. | | | | | | | | | | |
|---|---|---|---|---|---|---|---|---|---|---|---|
| | 14B-4 | 14B-5 | 14B-6 | 14B-7 | 14B-8 | 14B-9 | 14B-10 | 14B-11 | 14B-12 | 14B-13 | 14B-14 |
| smoothness | 2 | 3 | 3.5 | 5 | 5 | 5 | 5 | 5 | 5 | 5 | 5 |

**[Table 32-4]**

| | sample No. | | | | | | | |
|---|---|---|---|---|---|---|---|---|
| | 14C-4 | 14C-5 | 14C-6 | 14C-7 | 14C-8 | 14C-9 | 14C10 | 14C-11 |
| smoothness | 2 | 3 | 3.5 | 4 | 4 | 4 | 4 | 4 |

**[Table 32-5]**

| | sample No. | | | | | | | |
|---|---|---|---|---|---|---|---|---|
| | 14D-4 | 14D-5 | 14D-6 | 14D-7 | 14D-8 | 14D-9 | 14D-10 | 14D-11 |
| smoothness | 2 | 3 | 4 | 5 | 5 | 5 | 5 | 4 |

From the results of Tables 32-2, 32-3, samples 14A-6 to 14A-14 and samples 14B-6 to 14B-14, with the addition of PLD and ASO, were improved in smoothness compared to sample 14-2 (control). In addition, samples 14A-6 to 14A-14 and samples 14B-6 to 14B-14 were improved in smoothness compared to sample 14-3 (see Table 32-1) with the addition of PLD but without addition of ASO.

From the results of Tables 32-4, 32-5, samples 14C-6 to 14C-11 and samples 14D-6 to 14D-11, with the addition of PLD and GO, were improved in smoothness compared to sample 14-2 (control). In addition, samples 14C-6 to 14C-11 and samples 14D-6 to 14D-11 were improved in smoothness compared to sample 14-3 (see Table 32-1) with the addition of PLD but without addition of GO.

### [Experimental Example 15] Confirmation of effect of adding phospholipase D alone and effect of combined use of phospholipase D and auxiliary materials, in various protein systems

The trade name, company name, and protein content of various proteins used are shown in Table 33.

Each of various protein gel samples was prepared according to the sample preparation flow shown in Fig. 10, using the mixing recipes shown in Tables 33-1 to 33-29. The prepared samples exhibit the properties of either a suspension or sol or gel.

The obtained each sample was subjected to a sensory evaluation of smoothness by three expert panelists according to the following evaluation criteria. The results are shown in Tables 34-1 to 34-29.

### [Evaluation criteria (smoothness)]

smoothness: compared to control
5 points: very smooth texture
4 points: smooth texture
3 points: slightly smooth texture
2 points: the same
1 point: gritty texture

**[Table 33]**

| raw material protein name | trade name | protein content | company name |
|---|---|---|---|
| oat protein | ORPROTEIN (R) OT | 57% | ORGANO FOODTECH CORPORATION |
| pea protein | PP-CS | 79% | ORGANO FOODTECH CORPORATION |
| broad bean protein | ORPROTEIN (R) FP-AC | 84 wt% | ORGANO FOODTECH CORPORATION |
| mung bean protein | ORPROTEIN (R) MP-AC | 75 wt% | ORGANO FOODTECH CORPORATION |
| rice protein | Kometan - kissui | 75 wt% | Glico Nutrition Co., Ltd. |
| chickpea protein | ORPROTEIN (R) CP-AC | 63 wt% | ORGANO FOODTECH CORPORATION |
| rapeseed protein | Puratein G | 90 wt% | Merit Functional Foods Corporation |
| egg white (powder) | egg white powder | 83 wt% | Taiyo Kagaku Co., Ltd. |
| corn powder | Delicious corn powder | 12 wt% | Hokkaido Knorr Foods Co., Ltd. |
| whey powder | Morinaga whey powder | 13 wt% | MORINAGA MILK INDUSTRY CO., LTD. |
| whole milk powder | Yotsuba Whole Milk Powder | 27 wt% | Yotsuba Milk Products Co., Ltd. |
| skim milk powder | Yotsuba Skim Milk Powder | 36 wt% | Yotsuba Milk Products Co., Ltd. |
| Navy bean powder | Navy bean powder | 22 wt% | Cargill Japan LLC |
| almond protein | Almond protein powder | 44 wt% | Nissei Kyoeki Co., Ltd. |
| peanut powder | Roasted Peanut Protein | 46 wt% | Nissei Kyoeki Co., Ltd. |
| Cricket | Cricket Protein | 52 wt% | TAKEO, Inc. |
| Big Cricket Protein | Big Cricket Protein | 55 wt% | TAKEO, Inc. |
| Silkworm Powder | Silkworm Powder | 55 wt% | TAKEO, Inc. |
| spirulina | Spirulina powder | 62 wt% | Nature One, Inc. |

**[Table 33-1]**

| <Mixing recipe> | | | | | | | | | |
|---|---|---|---|---|---|---|---|---|---|
| | | sample No. | | | | | | | |
| | | 15A-1 (control) | 15A-2 | 15B-1 (control) | 15B-2 | 15C-1 (control) | 15C-2 | 15D-1 (control) | 15D-2 |
| raw material (weight %) | oat protein | 1.0 | 1.0 | | | | | | |
| | pea protein | | | 1.0 | 1.0 | | | | |
| | broad bean protein | | | | | 1.0 | 1.0 | | |
| | mung bean protein | | | | | | | 1.0 | 1.0 |
| | water | 99.0 | 99.0 | 99.0 | 99.0 | 99.0 | 99.0 | 99.0 | 99.0 |
| | DENAZYME PMD-P1 | | 0.003 | | 0.003 | | 0.003 | | 0.003 |
| | total | 100.0 | 100.0 | 100.0 | 100.0 | 100.0 | 100.0 | 100.0 | 100.0 |
| PLD activity (U) for 1 g of protein in sample | | | 297.4 | | 215.4 | | 201.8 | | 226.0 |

**[Table 33-2]**

| <Mixing recipe> | | | | | | | |
|---|---|---|---|---|---|---|---|
| raw material | | sample No. | | | | | |
| | | 15E-1 (control) | 15E-2 | 15F-1 (control) | 15F-2 | 15H-1 (control) | 15H-2 |
| raw material (weight %) | rice protein | 1.0 | 1.0 | | | | |
| | chickpea protein | | | 1.0 | 1.0 | | |
| | rapeseed protein | | | | | | |
| | egg white (powder) | | | | | 1.0 | 1.0 |
| | water | 99.0 | 99.0 | 99.0 | 99.0 | 99.0 | 99.0 |
| | DENAZYME PMD-P1 | | 0.003 | | 0.003 | | 0.003 |
| | total | 100.0 | 100.0 | 100.0 | 100.0 | 100.0 | 100.0 |
| PLD activity (U) for 1 g of protein in sample | | | 226.0 | | 269.0 | | 204.2 |

**[Table 33-3]**

| <Mixing recipe> | | | | | | | | | |
|---|---|---|---|---|---|---|---|---|---|
| raw material | | sample No. | | | | | | | |
| | | 15I-1 (control) | 15I-2 | 15J-1 (control) | 15J-2 | 15K-1 (control) | 15K-2 | 15L-1 (control) | 15L-2 |
| raw material (weight %) | corn powder | 1.0 | 1.0 | | | | | | |
| | whey powder | | | 1.0 | 1.0 | | | | |
| | whole milk powder | | | | | 1.0 | 1.0 | | |
| | skim milk powder | | | | | | | 1.0 | 1.0 |
| | water | 99.0 | 99.0 | 99.0 | 99.0 | 99.0 | 99.0 | 99.0 | 99.0 |
| | DENAZYME PMD-P1 | | 0.003 | | 0.003 | | 0.003 | | 0.003 |
| | total | 100.0 | 100.0 | 100.0 | 100.0 | 100.0 | 100.0 | 100.0 | 100.0 |
| PLD activity (U) for 1 g of protein in sample | | | 1461.2 | | 1356.0 | | 625.5 | | 476.1 |

**[Table 33-4]**

| <Mixing recipe> | | | | | | | | | |
|---|---|---|---|---|---|---|---|---|---|
| raw material | | sample No. | | | | | | | |
| | | 15M-1 (control) | 15M-2 | 15N-1 (control) | 15N-2 | 150-1 (control) | 150-2 | 15P-1 (control) | 15P-2 |
| raw material (weight %) | Navy bean powder | 1.0 | 1.0 | | | | | | |
| | almond protein | | | 1.0 | 1.0 | | | | |
| | peanut powder | | | | | 1.0 | 1.0 | | |
| | Cricket | | | | | | | 1.0 | 1.0 |
| | water | 99.0 | 99.0 | 99.0 | 99.0 | 99.0 | 99.0 | 99.0 | 99.0 |
| | DENAZYME PMD-P1 | | 0.003 | | 0.003 | | 0.003 | | 0.003 |
| | total | 100.0 | 100.0 | 100.0 | 100.0 | 100.0 | 100.0 | 100.0 | 100.0 |
| PLD activity (U) for 1 g of protein in sample | | | 767.0 | | 385.2 | | 368.5 | | 327.9 |

**[Table 33-5]**

| <Mixing recipe> | | | | | | | |
|---|---|---|---|---|---|---|---|
| raw material | | sample No. | | | | | |
| | | 15Q-1 (control) | 15Q-2 | 15R-1 (control) | 15R-2 | 15S-1 (control) | 15S-2 |
| raw material (weight %) | Big Cricket Protein | 1.0 | 1.0 | | | | |
| | Silkworm Powder | | | 1.0 | 1.0 | | |
| | spirulina | | | | | 1.0 | 1.0 |
| | water | 99.0 | 99.0 | 99.0 | 99.0 | 99.0 | 99.0 |
| | DENAZYME PMD-P1 | | 0.003 | | 0.003 | | 0.003 |
| | total | 100.0 | 100.0 | 100.0 | 100.0 | 100.0 | 100.0 |
| PLD activity (U) for 1 g of protein in sample | | | 306.5 | | 309.9 | | 274.3 |

**[Table 33-6]**

| <Mixing recipe> | | | | | | | | | |
|---|---|---|---|---|---|---|---|---|---|
| raw material | | sample No. | | | | | | | |
| | | 15A-3 (control) | 15A-4 | 15A-5 | 15A-6 | 15A-7 | 15A-8 | 15A-9 | 15A-10 |
| raw material (weight %) | oat protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 29.7 | 29.7 | 29.7 | 29.7 | 29.7 | 29.7 | 29.7 |

**[Table 33-7]**

| <Mixing recipe> | | | | | | | | | |
|---|---|---|---|---|---|---|---|---|---|
| raw material | | sample No. | | | | | | | |
| | | 15B-3 (control) | 15B-4 | 15B-5 | 15B-6 | 15B-7 | 15B-8 | 15B-9 | 15B-10 |
| raw material (weight %) | pea protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 21.5 | 21.5 | 21.5 | 21.5 | 21.5 | 21.5 | 21.5 |

**[Table 33-8]**

| <Mixing recipe> | | | | | | | | | |
|---|---|---|---|---|---|---|---|---|---|
| raw material | | sample No. | | | | | | | |
| | | 15C-3 (control) | 15C-4 | 15C-5 | 15C-6 | 15C-7 | 15C-8 | 15C-9 | 15C-10 |
| raw material (weight %) | broad bean protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 20.2 | 20.2 | 20.2 | 20.2 | 20.2 | 20.2 | 20.2 |

**[Table 33-9]**

| <Mixing recipe> | | | | | | | | | |
|---|---|---|---|---|---|---|---|---|---|
| raw material | | sample No. | | | | | | | |
| | | 15D-3 (control) | 15D-4 | 15D-5 | 15D-6 | 15D-7 | 15D-8 | 15D-9 | 15D-10 |
| raw material (weight %) | mung bean protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 22.6 | 22.6 | 22.6 | 22.6 | 22.6 | 22.6 | 22.6 |

**[Table 33-10]**

| <Mixing recipe> | | | | | | | | | |
|---|---|---|---|---|---|---|---|---|---|
| raw material | | sample No. | | | | | | | |
| | | 15E-3 (control) | 15E-4 | 15E-5 | 15E-6 | 15E-7 | 15E-8 | 15E-9 | 15E-10 |
| raw material (weight %) | rice protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 22.6 | 22.6 | 22.6 | 22.6 | 22.6 | 22.6 | 22.6 |

**[Table 33-11]**

| <Mixing recipe> | | | | | | | | | |
|---|---|---|---|---|---|---|---|---|---|
| raw material | | sample No. | | | | | | | |
| | | 15F-3 (control) | 15F-4 | 15F-5 | 15F-6 | 15F-7 | 15F-8 | 15F-9 | 15F-10 |
| raw material (weight %) | chickpea protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 26.9 | 26.9 | 26.9 | 26.9 | 26.9 | 26.9 | 26.9 |

**[Table 33-12]**

| <Mixing recipe> | | | | | | | | | |
|---|---|---|---|---|---|---|---|---|---|
| raw material | | sample No. | | | | | | | |
| | | 15G-3 (control) | 15G-4 | 15G-5 | 15G-6 | 15G-7 | 15G-8 | 15G-9 | 15G-10 |
| raw material (weight %) | rapeseed protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 18.8 | 18.8 | 18.8 | 18.8 | 18.8 | 18.8 | 18.8 |

**[Table 33-13]**

| <Mixing recipe> | | | | | | | | | |
|---|---|---|---|---|---|---|---|---|---|
| raw material | | sample No. | | | | | | | |
| | | 15H-3 (control) | 15H-4 | 15H-5 | 15H-6 | 15H-7 | 15H-8 | 15H-9 | 15H-10 |
| raw material (weight %) | egg white (powder) | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 20.4 | 20.4 | 20.4 | 20.4 | 20.4 | 20.4 | 20.4 |

**[Table 33-14]**

| <Mixing recipe> | | | | | | | | | |
|---|---|---|---|---|---|---|---|---|---|
| raw material | | sample No. | | | | | | | |
| | | 15I-3 (control) | 151-4 | 15I-5 | 15I-6 | 15I-7 | 15I-8 | 151-9 | 15I-10 |
| raw material (weight %) | corn powder | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 146.1 | 146.1 | 146.1 | 146.1 | 146.1 | 146.1 | 146.1 |

**[Table 33-15]**

| <Mixing recipe> | | | | | | | | | |
|---|---|---|---|---|---|---|---|---|---|
| raw material | | sample No. | | | | | | | |
| | | 15J-3 (control) | 15J-4 | 15J-5 | 15J-6 | 15J-7 | 15J-8 | 51J-9 | 15J-10 |
| raw material (weight %) | whey powder | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 135.6 | 135.6 | 135.6 | 135.6 | 135.6 | 135.6 | 135.6 |

**[Table 33-16]**

| <Mixing recipe> | | | | | | | | | |
|---|---|---|---|---|---|---|---|---|---|
| raw material | | sample No. | | | | | | | |
| | | 15K-3 (control) | 15K-4 | 15K-5 | 15K-6 | 15K-7 | 15K-8 | 15K-9 | 15K-10 |
| raw material (weight %) | whole milk powder | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 62.5 | 62.5 | 62.5 | 62.5 | 62.5 | 62.5 | 62.5 |

**[Table 33-17]**

| <Mixing recipe> | | | | | | | | | |
|---|---|---|---|---|---|---|---|---|---|
| raw material | | sample No. | | | | | | | |
| | | 15L-3 (control) | 15L-4 | 15L-5 | 15L-6 | 15L-7 | 15L-8 | 15L-9 | 15L-10 |
| raw material (weight %) | skim milk powder | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 47.6 | 47.6 | 47.6 | 47.6 | 47.6 | 47.6 | 47.6 |

**[Table 33-18]**

| <Mixing recipe> | | | | | | | | | |
|---|---|---|---|---|---|---|---|---|---|
| raw material | | sample No. | | | | | | | |
| | | 15M-3 (control) | 15M-4 | 15M-5 | 15M-6 | 15M-7 | 15M-8 | 15M-9 | 15M-10 |
| raw material (weight %) | Navy bean powder | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 76.7 | 76.7 | 76.7 | 76.7 | 76.7 | 76.7 | 76.7 |

**[Table 33-19]**

| <Mixing recipe> | | | | | | | | | |
|---|---|---|---|---|---|---|---|---|---|
| raw material | | sample No. | | | | | | | |
| | | 15N-3 (control) | 15N-4 | 15N-5 | 15N-6 | 15N-7 | 15N-8 | 15N-9 | 15N-10 |
| raw material (weight %) | almond protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 38.5 | 38.5 | 38.5 | 38.5 | 38.5 | 38.5 | 38.5 |

**[Table 33-20]**

| <Mixing recipe> | | | | | | | | | |
|---|---|---|---|---|---|---|---|---|---|
| raw material | | sample No. | | | | | | | |
| | | 150-3 (control) | 150-4 | 150-5 | 150-6 | 150-7 | 150-8 | 150-9 | 150-10 |
| raw material (weight %) | peanut powder | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 36.8 | 36.8 | 36.8 | 36.8 | 36.8 | 36.8 | 36.8 |

**[Table 33-21]**

| <Mixing recipe> | | | | | | | | | |
|---|---|---|---|---|---|---|---|---|---|
| raw material | | sample No. | | | | | | | |
| | | 15P-3 (control) | 15P-4 | 15P-5 | 15P-6 | 15P-7 | 15P-8 | 15P-9 | 15P-10 |
| raw material (weight %) | Cricket | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 32.8 | 32.8 | 32.8 | 32.8 | 32.8 | 32.8 | 32.8 |

**[Table 33-22]**

| <Mixing recipe> | | | | | | | | | |
|---|---|---|---|---|---|---|---|---|---|
| raw material | | sample No. | | | | | | | |
| | | 51Q-3 (control) | 15Q-4 | 15Q-5 | 15Q-6 | 15Q-7 | 15Q-8 | 15Q-9 | 15Q-10 |
| raw material (weight %) | Big Cricket Protein | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 30.7 | 30.7 | 30.7 | 30.7 | 30.7 | 30.7 | 30.7 |

**[Table 33-23]**

| <Mixing recipe> | | | | | | | | | |
|---|---|---|---|---|---|---|---|---|---|
| raw material | | sample No. | | | | | | | |
| | | 15R-3 (control) | 15R-4 | 15R-5 | 15R-6 | 15R-7 | 15R-8 | 15R-9 | 15R-10 |
| raw material (weight %) | Silkworm Powder | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 31.0 | 31.0 | 31.0 | 31.0 | 31.0 | 31.0 | 31.0 |

**[Table 33-24]**

| <Mixing recipe> | | | | | | | | | |
|---|---|---|---|---|---|---|---|---|---|
| raw material | | sample No. | | | | | | | |
| | | 15S-3 (control) | 15S-4 | 15S-5 | 15S-6 | 15S-7 | 15S-8 | 15S-9 | 15S-10 |
| raw material (weight %) | spirulina | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | DENAZYME PMD-P1 | | 0.003 | 0.003 | 0.003 | 0.003 | 0.003 | 0.003 | 0.003 |
| | threonine | | | 0.001 | | | | | |
| | manganese-containing yeast | | | | 0.001 | | | | |
| | glutathione-containing yeast extract | | | | | 0.001 | | | |
| | alanine | | | | | | 0.001 | | |
| | cysteine hydrochloride | | | | | | | 0.001 | |
| | cysteine | | | | | | | | 0.001 |
| | total | 100.0 | 100.0 | 100.0 | 100.0 | 100.0 | 100.0 | 100.0 | 100.0 |
| PLD activity (U) for 1 g of protein in sample | | | 27.4 | 27.4 | 27.4 | 27.4 | 27.4 | 27.4 | 27.4 |

**[Table 33-25]**

| <Mixing recipe> | | | | | | | | | |
|---|---|---|---|---|---|---|---|---|---|
| raw material | | sample No. | | | | | | | |
| | | 15A-11 (control) | 15A-12 | 15B-11 (control) | 15B-12 | 15C-11 (control) | 15C-12 | 15D-11 (control) | 15D-12 |
| raw material (weight %) | oat protein | 20.0 | 20.0 | | | | | | |
| | pea protein | | | 20.0 | 20.0 | | | | |
| | broad bean protein | | | | | 20.0 | 20.0 | | |
| | mung bean protein | | | | | | | 20.0 | 20.0 |
| | water | 80.0 | 80.0 | 80.0 | 80.0 | 80.0 | 80.0 | 80.0 | 80.0 |
| | DENAZYME PMD-P1 | | 0.003 | | 0.003 | | 0.003 | | 0.003 |
| | total | 100.0 | 100.0 | 100.0 | 100.0 | 100.0 | 100.0 | 100.0 | 100.0 |
| PLD activity (U) for 1 g of protein in sample | | | 14.9 | | 10.8 | | 10.1 | | 11.3 |

**[Table 33-26]**

| <Mixing recipe> | | | | | | | | | |
|---|---|---|---|---|---|---|---|---|---|
| raw material | | sample No. | | | | | | | |
| | | 15E-11 (control) | 15E-12 | 15F-11 (control) | 15F-12 | 15G-11 (control) | 15G-12 | 15H-11 (control) | 15H-12 |
| raw material (weight %) | rice protein | 20.0 | 20.0 | | | | | | |
| | chickpea protein | | | 20.0 | 20.0 | | | | |
| | rapeseed protein | | | | | 20.0 | 20.0 | | |
| | egg white (powder) | | | | | | | 20.0 | 20.0 |
| | water | 80.0 | 80.0 | 80.0 | 80.0 | 80.0 | 80.0 | 80.0 | 80.0 |
| | DENAZYME PMD-P1 | | 0.003 | | 0.003 | | 0.003 | | 0.003 |
| | total | 100.0 | 100.0 | 100.0 | 100.0 | 100.0 | 100.0 | 100.0 | 100.0 |
| PLD activity (U) for 1 g of protein in sample | | | 11.3 | | 13.5 | | 9.4 | | 10.2 |

**[Table 33-27]**

| <Mixing recipe> | | | | | | | | | |
|---|---|---|---|---|---|---|---|---|---|
| raw material | | sample No. | | | | | | | |
| | | 15I-11 (control) | 15I-12 | 15J-11 (control) | 15J-12 | 15K-11 (control) | 15K-12 | 15L-11 (control) | 15L-12 |
| raw material (weight %) | corn powder | 20.0 | 20.0 | | | | | | |
| | whey powder | | | 20.0 | 20.0 | | | | |
| | whole milk powder | | | | | 20.0 | 20.0 | | |
| | skim milk powder | | | | | | | 20.0 | 20.0 |
| | water | 80.0 | 80.0 | 80.0 | 80.0 | 80.0 | 80.0 | 80.0 | 80.0 |
| | DENAZYME PMD-P1 | | 0.003 | | 0.003 | | 0.003 | | 0.003 |
| | total | 100.0 | 100.0 | 100.0 | 100.0 | 100.0 | 100.0 | 100.0 | 100.0 |
| PLD activity (U) for 1 g of protein in sample | | | 73.1 | | 67.8 | | 31.3 | | 23.8 |

**[Table 33-28]**

| <Mixing recipe> | | | | | | | | | |
|---|---|---|---|---|---|---|---|---|---|
| raw material | | sample No. | | | | | | | |
| | | 15M-11 (control) | 15M-12 | 15N-11 (control) | 15N-12 | 15O-11 (control) | 15O-12 | 15P-11 (control) | 15P-12 |
| raw material (weight %) | Navy bean powder | 20.0 | 20.0 | | | | | | |
| | almond protein | | | 20.0 | 20.0 | | | | |
| | peanut powder | | | | | 20.0 | 20.0 | | |
| | Cricket | | | | | | | 20.0 | 20.0 |
| | water | 80.0 | 80.0 | 80.0 | 80.0 | 80.0 | 80.0 | 80.0 | 80.0 |
| | DENAZYME PMD-P1 | | 0.003 | | 0.003 | | 0.003 | | 0.003 |
| | total | 100.0 | 100.0 | 100.0 | 100.0 | 100.0 | 100.0 | 100.0 | 100.0 |
| PLD activity (U) for 1 g of protein in sample | | | 38.3 | | 19.3 | | 18.4 | | 16.4 |

**[Table 33-29]**

| <Mixing recipe> | | | | | | | |
|---|---|---|---|---|---|---|---|
| raw material | | sample No. | | | | | |
| | | 15Q-11 (control) | 15Q-12 | 15R-11 (control) | 15R-12 | 15S-11 (control) | 15S-12 |
| raw material (weight %) | Big Cricket Protein | 20.0 | 20.0 | | | | |
| | Silkworm Powder | | | 20.0 | 20.0 | | |
| | spirulina | | | | | 20.0 | 20.0 |
| | water | 80.0 | 80.0 | 80.0 | 80.0 | 80.0 | 80.0 |
| | DENAZYME PMD-P1 | | 0.003 | | 0.003 | | 0.003 |
| | total | 100.0 | 100.0 | 100.0 | 100.0 | 100.0 | 100.0 |
| PLD activity (U) for 1 g of protein in sample | | | 15.3 | | 15.5 | | 13.7 |

**[Table 34-1]**

| | sample No. | | | | | | | |
|---|---|---|---|---|---|---|---|---|
| | 15A-1 | 15A-2 | 15B-1 | 15B-2 | 15C-1 | 15C-2 | 15D-1 | 15D-2 |
| smoothness | - | 3 | - | 3 | - | 3 | - | 3 |

**[Table 34-2]**

| | sample No. | | | | | |
|---|---|---|---|---|---|---|
| | 15E-1 | 15E-2 | 15F-1 | 15F-2 | 15H-1 | 15H-2 |
| smoothness | - | 3 | - | 3 | - | 2.5 |

**[Table 34-3]**

| | sample No. | | | | | | | |
|---|---|---|---|---|---|---|---|---|
| | 15I-1 | 15I-2 | 15J-1 | 15J-2 | 15K-1 | 15K-2 | 15L-1 | 15L-2 |
| smoothness | - | 3 | - | 3 | - | 3 | - | 3 |

**[Table 34-4]**

| | sample No. | | | | | | | |
|---|---|---|---|---|---|---|---|---|
| | 15M-1 | 15M-2 | 15N-1 | 15N-2 | 15O-1 | 15O-2 | 15P-1 | 15P-2 |
| smoothness | - | 3 | - | 3 | - | 3 | - | 3 |

**[Table 34-5]**

| | sample No. | | | | | |
|---|---|---|---|---|---|---|
| | 15Q-1 | 15Q-2 | 15R-1 | 15R-2 | 15S-1 | 15S-2 |
| smoothness | - | 3 | - | 3 | - | 3 |

**[Table 34-6]**

| | sample No. | | | | | | | |
|---|---|---|---|---|---|---|---|---|
| | 15A-3 | 15A-4 | 15A-5 | 15A-6 | 15A-7 | 15A-8 | 15A-9 | 15A-10 |
| smoothness | - | 3 | 4 | 4 | 4 | 4 | 4 | 4 |

**[Table 34-7]**

| | sample No. | | | | | | | |
|---|---|---|---|---|---|---|---|---|
| | 15B-3 | 15B-4 | 15B-5 | 15B-6 | 15B-7 | 15B-8 | 15B-9 | 15B-10 |
| smoothness | - | 3 | 4 | 3.5 | 4 | 3.5 | 4 | 4 |

**[Table 34-8]**

| | sample No. | | | | | | | |
|---|---|---|---|---|---|---|---|---|
| | 15C-3 | 15C-4 | 15C-5 | 15C-6 | 15C-7 | 15C-8 | 15C-9 | 15C-10 |
| smoothness | - | 3.5 | 4.5 | 4.5 | 4.5 | 4.5 | 4.5 | 4.5 |

**[Table 34-9]**

| | sample No. | | | | | | | |
|---|---|---|---|---|---|---|---|---|
| | 15D-3 | 15D-4 | 15D-5 | 15D-6 | 15D-7 | 15D-8 | 15D-9 | 15D-10 |
| smoothness | - | 3 | 5 | 4.5 | 5 | 4.5 | 5 | 5 |

**[Table 34-10]**

| | sample No. | | | | | | | |
|---|---|---|---|---|---|---|---|---|
| | 15E-3 | 15E-4 | 15E-5 | 15E-6 | 15E-7 | 15E-8 | 15E-9 | 15E-10 |
| smoothness | - | 3 | 4 | 3.5 | 4 | 3.5 | 4 | 4 |

**[Table 34-11]**

| | sample No. | | | | | | | |
|---|---|---|---|---|---|---|---|---|
| | 15F-3 | 15F-4 | 15F-5 | 15F-6 | 15F-7 | 15F-8 | 15F-9 | 15F-10 |
| smoothness | - | 3 | 4.5 | 4 | 4.5 | 4 | 4.5 | 4.5 |

**[Table 34-12]**

| | sample No. | | | | | | | |
|---|---|---|---|---|---|---|---|---|
| | 15G-3 | 15G-4 | 15G-5 | 15G-6 | 15G-7 | 15G-8 | 15G-9 | 15G-10 |
| smoothness | - | 3 | 4 | 4 | 4 | 4 | 4 | 4 |

**[Table 34-13]**

| | sample No. | | | | | | | |
|---|---|---|---|---|---|---|---|---|
| | 15H-3 | 15H-4 | 15H-5 | 15H-6 | 15H-7 | 15H-8 | 15H-9 | 15H-10 |
| smoothness | - | 2.5 | 3 | 3 | 3 | 3 | 3 | 3 |

**[Table 34-14]**

| | sample No. | | | | | | | |
|---|---|---|---|---|---|---|---|---|
| | 151-3 | 15I-4 | 15I-5 | 15I-6 | 15I-7 | 15I-8 | 15I-9 | 15I-10 |
| smoothness | - | 3 | 4 | 4 | 4 | 4 | 4 | 4 |

**[Table 34-15]**

| | sample No. | | | | | | | |
|---|---|---|---|---|---|---|---|---|
| | 15J-3 | 15J-4 | 15J-5 | 15J-6 | 15J-7 | 15J-8 | 15J-9 | 15J-10 |
| smoothness | - | 3 | 4 | 3.5 | 4 | 3.5 | 4 | 4 |

**[Table 34-16]**

| | sample No. | | | | | | | |
|---|---|---|---|---|---|---|---|---|
| | 15K-3 | 15K-4 | 15K-5 | 15K-6 | 15K-7 | 15K-8 | 15K-9 | 15K-10 |
| smoothness | - | 3 | 4.5 | 4 | 4.5 | 4 | 4.5 | 4.5 |

**[Table 34-17]**

| | sample No. | | | | | | | |
|---|---|---|---|---|---|---|---|---|
| | 15L-3 | 15L-4 | 15L-5 | 15L-6 | 15L-7 | 15L-8 | 15L-9 | 15L-10 |
| smoothness | - | 3 | 4 | 3.5 | 4 | 3.5 | 4 | 4 |

**[Table 34-18]**

| | sample No. | | | | | | | |
|---|---|---|---|---|---|---|---|---|
| | 15M-3 | 15M-4 | 15M-5 | 15M-6 | 15M-7 | 15M-8 | 15M-9 | 15M-10 |
| smoothness | - | 3 | 3.5 | 3.5 | 4 | 3.5 | 4 | 4 |

**[Table 34-19]**

| | sample No. | | | | | | | |
|---|---|---|---|---|---|---|---|---|
| | 15N-3 | 15N-4 | 15N-5 | 15N-6 | 15N-7 | 15N-8 | 15N-9 | 15N-10 |
| smoothness | - | 3 | 4 | 3.5 | 3.5 | 3.5 | 3.5 | 3.5 |

**[Table 34-20]**

| | sample No. | | | | | | | |
|---|---|---|---|---|---|---|---|---|
| | 15O-3 | 15O-4 | 15O-5 | 15O-6 | 15O-7 | 15O-8 | 15O-9 | 15O-10 |
| smoothness | - | 3 | 4 | 3.5 | 4 | 3.5 | 4 | 4 |

**[Table 34-21]**

| | sample No. | | | | | | | |
|---|---|---|---|---|---|---|---|---|
| | 15P-3 | 15P-4 | 15P-5 | 15P-6 | 15P-7 | 15P-8 | 15P-9 | 15P-10 |
| smoothness | - | 3 | 4 | 3.5 | 4 | 3.5 | 4 | 4 |

**[Table 34-22]**

| | sample No. | | | | | | | |
|---|---|---|---|---|---|---|---|---|
| | 15Q-3 | 15Q-4 | 15Q-5 | 15Q-6 | 15Q-7 | 15Q-8 | 15Q-9 | 150-10 |
| smoothness | - | 3 | 4 | 3.5 | 4 | 3.5 | 3.5 | 3.5 |

**[Table 34-23]**

| | sample No. | | | | | | | |
|---|---|---|---|---|---|---|---|---|
| | 15R-3 | 15R-4 | 15R-5 | 15R-6 | 15R-7 | 15R-8 | 15R-9 | 15R-10 |
| smoothness | - | 3 | 4 | 3.5 | 4 | 3.5 | 4 | 4 |

**[Table 34-24]**

| | sample No. | | | | | | | |
|---|---|---|---|---|---|---|---|---|
| | 15S-3 | 15S-4 | 15S-5 | 15S-6 | 158-7 | 15S-8 | 15S-9 | 15S-10 |
| smoothness | - | 3 | 4.5 | 3.5 | 4 | 3.5 | 4 | 4 |

**[Table 34-25]**

| | sample No. | | | | | | | |
|---|---|---|---|---|---|---|---|---|
| | 15A-11 | 15A-12 | 15B-11 | 15B-12 | 15C-11 | 15C-12 | 15D-11 | 15D-12 |
| smoothness | - | 3 | - | 3.5 | - | 3.5 | - | 3 |

**[Table 34-26]**

| | sample No. | | | | | | | |
|---|---|---|---|---|---|---|---|---|
| | 15E-11 | 15E-12 | 15F-11 | 15F-12 | 15G-11 | 15G-12 | 15H-11 | 15H-12 |
| smoothness | - | 3 | - | 3 | - | 3 | - | 3 |

**[Table 34-27]**

| | sample No. | | | | | | | |
|---|---|---|---|---|---|---|---|---|
| | 15I-11 | 15I-12 | 15J-11 | 15J-12 | 15K-11 | 15K-12 | 15L-11 | 15L-12 |
| smoothness | - | 3 | - | 3 | - | 3 | - | 3 |

**[Table 34-28]**

| | sample No. | | | | | | | |
|---|---|---|---|---|---|---|---|---|
| | 15M-11 | 15M-12 | 15N-11 | 15N-12 | 15O-11 | 15O-12 | 15P-11 | 15P-12 |
| smoothness | - | 3.5 | - | 3.5 | - | 3.5 | - | 3 |

**[Table 34-29]**

| | sample No. | | | | | |
|---|---|---|---|---|---|---|
| | 15Q-11 | 15Q-12 | 15R-11 | 15R-12 | 15S-11 | 15S-12 |
| smoothness | - | 3 | - | 3 | - | 3 |

From the results of Tables 34-1 to 34-29, the following was shown.

Sample 15A-2 (PLD activity value 297.4 U/protein 1 g) obtained by adding PLD to oat protein was improved in smoothness compared to sample 15A-1 (control).

Sample 15A-4 (PLD activity value 29.7 U/protein 1 g) obtained by adding PLD to oat protein was improved in smoothness compared to sample 15A-3 (control).

Sample 15A-12 (PLD activity value 14.9 U/protein 1 g) obtained by adding PLD to oat protein was improved in smoothness compared to sample 15A-11 (control).

Sample 15B-2 (PLD activity value 215.4 U/protein 1 g) obtained by adding PLD to pea protein was improved in smoothness compared to sample 15B-1 (control).

Sample 15B-4 (PLD activity value 21.5 U/protein 1 g) obtained by adding PLD to pea protein was improved in smoothness compared to sample 15B-3 (control).

Sample 15B-12 (PLD activity value 10.8 U/protein 1 g) obtained by adding PLD to pea protein was improved in smoothness compared to sample 15B-11 (control).

Sample 15C-2 (PLD activity value 201.8 U/protein 1 g) obtained by adding PLD to broad bean protein was improved in smoothness compared to sample 15C-1 (control).

Sample 15C-4 (PLD activity value 20.2 U/protein 1 g) obtained by adding PLD to broad bean protein was improved in smoothness compared to sample 15C-3 (control).

Sample 15C-12 (PLD activity value 10.1 U/protein 1 g) obtained by adding PLD to broad bean protein was improved in smoothness compared to sample 15C-11 (control).

Sample 15D-2 (PLD activity value 226.0 U/protein 1 g) obtained by adding PLD to mung bean protein was improved in smoothness compared to sample 15D-1 (control).

Sample 15D-4 (PLD activity value 22.6 U/protein 1 g) obtained by adding PLD to mung bean protein was improved in smoothness compared to sample 15D-3 (control).

Sample 15D-12 (PLD activity value 11.3 U/protein 1 g) obtained by adding PLD to mung bean protein was improved in smoothness compared to sample 15D-11 (control).

Sample 15E-2 (PLD activity value 226.0 U/protein 1 g) obtained by adding PLD to rice protein was improved in smoothness compared to sample 15E-1 (control).

Sample 15E-4 (PLD activity value 22.6 U/protein 1 g) obtained by adding PLD to rice protein was improved in smoothness compared to sample 15E-3 (control).

Sample 15E-12 (PLD activity value 11.3 U/protein 1 g) obtained by adding PLD to rice protein was improved in smoothness compared to sample 15E-11 (control).

Sample 15F-2 (PLD activity value 269.0 U/protein 1 g) obtained by adding PLD to chickpea protein was improved in smoothness compared to sample 15F-1 (control).

Sample 15F-4 (PLD activity value 26.9 U/protein 1 g) obtained by adding PLD to chickpea protein was improved in smoothness compared to sample 15F-3 (control).

Sample 15F-12 (PLD activity value 13.5 U/protein 1 g) obtained by adding PLD to chickpea protein was improved in smoothness compared to sample 15F-11 (control).

Sample 15G-4 (PLD activity value 18.8 U/protein 1 g) obtained by adding PLD to rapeseed protein was improved in smoothness compared to sample 15G-3 (control).

Sample 15G-12 (PLD activity value 9.4 U/protein 1 g) obtained by adding PLD to rapeseed protein was improved in smoothness compared to sample 15G-11 (control).

Sample 15H-2 (PLD activity value 204.2 U/protein 1 g) obtained by adding PLD to egg white was improved in smoothness compared to sample 15H-1 (control).

Sample 15H-4 (PLD activity value 20.4 U/protein 1 g) obtained by adding PLD to egg white was improved in smoothness compared to sample 15H-3 (control).

Sample 15H-12 (PLD activity value 10.2 U/protein 1 g) obtained by adding PLD to egg white was improved in smoothness compared to sample 15H-11 (control).

Sample 151-2 (PLD activity value 1461.2 U/protein 1 g) obtained by adding PLD to corn protein was improved in smoothness compared to sample 15I-1 (control).

Sample 15I-4 (PLD activity value 146.1 U/protein 1 g) obtained by adding PLD to corn protein was improved in smoothness compared to sample 15I-3 (control).

Sample 15I-12 (PLD activity value 73.1 U/protein 1 g) obtained by adding PLD to corn protein was improved in smoothness compared to sample 15I-11 (control).

Sample 15J-2 (PLD activity value 1356.0 U/protein 1 g) obtained by adding PLD to whey protein was improved in smoothness compared to sample 15J-1 (control).

Sample 15J-4 (PLD activity value 135.6 U/protein 1 g) obtained by adding PLD to whey protein was improved in smoothness compared to sample 15J-3 (control).

Sample 15J-12 (PLD activity value 67.8 U/protein 1 g) obtained by adding PLD to whey protein was improved in smoothness compared to sample 15J-11 (control).

Sample 15K-2 (PLD activity value 625.5 U/protein 1 g) obtained by adding PLD to whole milk protein powder was improved in smoothness compared to sample 15K-1 (control).

Sample 15K-4 (PLD activity value 62.5 U/protein 1 g) obtained by adding PLD to whole milk protein powder was improved in smoothness compared to sample 15K-3 (control).

Sample 15K-12 (PLD activity value 31.3 U/protein 1 g) obtained by adding PLD to whole milk protein powder was improved in smoothness compared to sample 15K-11 (control).

Sample 15L-2 (PLD activity value 476.1 U/protein 1 g) obtained by adding PLD to skim milk protein was improved in smoothness compared to sample 15L-1 (control).

Sample 15L-4 (PLD activity value 47.6 U/protein 1 g) obtained by adding PLD to skim milk protein was improved in smoothness compared to sample 15L-3 (control).

Sample 15L-12 (PLD activity value 23.8 U/protein 1 g) obtained by adding PLD to skim milk protein was improved in smoothness compared to sample 15L-11 (control).

Sample 15M-2 (PLD activity value 767.0 U/protein 1 g) obtained by adding PLD to Navy bean protein was improved in smoothness compared to sample 15M-1 (control).

Sample 15M-4 (PLD activity value 76.7 U/protein 1 g) obtained by adding PLD to Navy bean protein was improved in smoothness compared to sample 15M-3 (control).

Sample 15M-12 (PLD activity value 38.3 U/protein 1 g) obtained by adding PLD to Navy bean protein was improved in smoothness compared to sample 15M-11 (control).

Sample 15N-2 (PLD activity value 385.2 U/protein 1 g) obtained by adding PLD to almond protein was improved in smoothness compared to sample 15N-1 (control).

Sample 15N-4 (PLD activity value 38.5 U/protein 1 g) obtained by adding PLD to almond protein was improved in smoothness compared to sample 15N-3 (control).

Sample 15N-12 (PLD activity value 19.3 U/protein 1 g) obtained by adding PLD to almond protein was improved in smoothness compared to sample 15N-11 (control).

Sample 150-2 (PLD activity value 368.5 U/protein 1 g) obtained by adding PLD to peanut protein was improved in smoothness compared to sample 150-1 (control).

Sample 150-4 (PLD activity value 36.8 U/protein 1 g) obtained by adding PLD to peanut protein was improved in smoothness compared to sample 150-3 (control).

Sample 150-12 (PLD activity value 18.4 U/protein 1 g) obtained by adding PLD to peanut protein was improved in smoothness compared to sample 150-11 (control).

Sample 15P-2 (PLD activity value 327.9 U/protein 1 g) obtained by adding PLD to Cricket protein was improved in smoothness compared to sample 15P-1 (control).

Sample 15P-4 (PLD activity value 32.8 U/protein 1 g) obtained by adding PLD to Cricket protein was improved in smoothness compared to sample 15P-3 (control).

Sample 15P-12 (PLD activity value 16.4 U/protein 1 g) obtained by adding PLD to Cricket protein was improved in smoothness compared to sample 15P-11 (control).

Sample 15Q-2 (PLD activity value 306.5 U/protein 1 g) obtained by adding PLD to Big Cricket protein was improved in smoothness compared to sample 15Q-1 (control).

Sample 15Q-4 (PLD activity value 30.7 U/protein 1 g) obtained by adding PLD to Big Cricket protein was improved in smoothness compared to sample 15Q-3 (control).

Sample 15Q-12 (PLD activity value 15.3 U/protein 1 g) obtained by adding PLD to Big Cricket protein was improved in smoothness compared to sample 15Q-11 (control).

Sample 15R-2 (PLD activity value 309.9 U/protein 1 g) obtained by adding PLD to Silkworm protein was improved in smoothness compared to sample 15R-1 (control).

Sample 15R-4 (PLD activity value 31.0 U/protein 1 g) obtained by adding PLD to Silkworm protein was improved in smoothness compared to sample 15R-3 (control).

Sample 15R-12 (PLD activity value 15.5 U/protein 1 g) obtained by adding PLD to Silkworm protein was improved in smoothness compared to sample 15R-11 (control).

Sample 15S-2 (PLD activity value 274.3 U/protein 1 g) obtained by adding PLD to spirulina protein was improved in smoothness compared to sample 15S-1 (control).

Sample 15S-4 (PLD activity value 27.4 U/protein 1 g) obtained by adding PLD to spirulina protein was improved in smoothness compared to sample 15S-3 (control).

Sample 15S-12 (PLD activity value 13.7 U/protein 1 g) obtained by adding PLD to spirulina protein was improved in smoothness compared to sample 15S-11 (control).

From the results of Table 34-6, samples 15A-5 to 15A-10, obtained by adding PLD and an auxiliary material (threonine, manganese-containing yeast, glutathione-containing yeast extract, alanine, cysteine hydrochloride, or cysteine) to oat protein, were improved in smoothness compared to sample 15A-4 with the addition of PLD but without addition of the above-mentioned auxiliary materials.

From the results of Table 34-7, samples 15B-5 to 15B-10, obtained by adding PLD and an auxiliary material (threonine, manganese-containing yeast, glutathione-containing yeast extract, alanine, cysteine hydrochloride, or cysteine) to pea protein, were improved in smoothness compared to sample 15B-4 with the addition of PLD but without addition of the above-mentioned auxiliary materials.

From the results of Table 34-8, samples 15C-5 to 15C-10, obtained by adding PLD and an auxiliary material (threonine, manganese-containing yeast, glutathione-containing yeast extract, alanine, cysteine hydrochloride, or cysteine) to broad bean protein, were improved in smoothness compared to sample 15C-4 with the addition of PLD but without addition of the above-mentioned auxiliary materials.

From the results of Table 34-9, samples 15D-5 to 15D-10, obtained by adding PLD and an auxiliary material (threonine, manganese-containing yeast, glutathione-containing yeast extract, alanine, cysteine hydrochloride, or cysteine) to mung bean protein, were improved in smoothness compared to sample 15D-4 with the addition of PLD but without addition of the above-mentioned auxiliary materials.

From the results of Table 34-10, samples 15E-5 to 15E-10, obtained by adding PLD and an auxiliary material (threonine, manganese-containing yeast, glutathione-containing yeast extract, alanine, cysteine hydrochloride, or cysteine) to rice protein, were improved in smoothness compared to sample 15E-4 with the addition of PLD but without addition of the above-mentioned auxiliary materials.

From the results of Table 34-11, samples 15F-5 to 15F-10, obtained by adding PLD and an auxiliary material (threonine, manganese-containing yeast, glutathione-containing yeast extract, alanine, cysteine hydrochloride, or cysteine) to chickpea protein, were improved in smoothness compared to sample 15F-4 with the addition of PLD but without addition of the above-mentioned auxiliary materials.

From the results of Table 34-12, samples 15G-5 to 15G-10, obtained by adding PLD and an auxiliary material (threonine, manganese-containing yeast, glutathione-containing yeast extract, alanine, cysteine hydrochloride, or cysteine) to rapeseed protein, were improved in smoothness compared to sample 15G-4 with the addition of PLD but without addition of the above-mentioned auxiliary materials.

From the results of Table 34-13, samples 15H-5 to 15H-10, obtained by adding PLD and an auxiliary material (threonine, manganese-containing yeast, glutathione-containing yeast extract, alanine, cysteine hydrochloride, or cysteine) to egg white, were improved in smoothness compared to sample 15H-4 with the addition of PLD but without addition of the above-mentioned auxiliary materials.

From the results of Table 34-14, samples 15I-5 to 15I-10, obtained by adding PLD and an auxiliary material (threonine, manganese-containing yeast, glutathione-containing yeast extract, alanine, cysteine hydrochloride, or cysteine) to corn protein, were improved in smoothness compared to sample 15I-4 with the addition of PLD but without addition of the above-mentioned auxiliary materials.

From the results of Table 34-15, samples 15J-5 to 15J-10, obtained by adding PLD and an auxiliary material (threonine, manganese-containing yeast, glutathione-containing yeast extract, alanine, cysteine hydrochloride, or cysteine) to whey protein, were improved in smoothness compared to sample 15J-4 with the addition of PLD but without addition of the above-mentioned auxiliary materials.

From the results of Table 34-16, samples 15K-5 to 15K-10, obtained by adding PLD and an auxiliary material (threonine, manganese-containing yeast, glutathione-containing yeast extract, alanine, cysteine hydrochloride, or cysteine) to whole milk protein powder, were improved in smoothness compared to sample 15K-4 with the addition of PLD but without addition of the above-mentioned auxiliary materials.

From the results of Table 34-17, samples 15L-5 to 15L-10, obtained by adding PLD and an auxiliary material (threonine, manganese-containing yeast, glutathione-containing yeast extract, alanine, cysteine hydrochloride, or cysteine) to skim milk protein powder, were improved in smoothness compared to sample 15L-4 with the addition of PLD but without addition of the above-mentioned auxiliary materials.

From the results of Table 34-18, samples 15M-5 to 15M-10, obtained by adding PLD and an auxiliary material (threonine, manganese-containing yeast, glutathione-containing yeast extract, alanine, cysteine hydrochloride, or cysteine) to Navy bean protein, were improved in smoothness compared to sample 15M-4 with the addition of PLD but without addition of the above-mentioned auxiliary materials.

From the results of Table 34-19, samples 15N-5 to 15N-10, obtained by adding PLD and an auxiliary material (threonine, manganese-containing yeast, glutathione-containing yeast extract, alanine, cysteine hydrochloride, or cysteine) to almond protein, were improved in smoothness compared to sample 15N-4 with the addition of PLD but without addition of the above-mentioned auxiliary materials.

From the results of Table 34-20, samples 150-5 to 150-10, obtained by adding PLD and an auxiliary material (threonine, manganese-containing yeast, glutathione-containing yeast extract, alanine, cysteine hydrochloride, or cysteine) to peanut protein, were improved in smoothness compared to sample 150-4 with the addition of PLD but without addition of the above-mentioned auxiliary materials.

From the results of Table 34-21, samples 15P-5 to 15P-10, obtained by adding PLD and an auxiliary material (threonine, manganese-containing yeast, glutathione-containing yeast extract, alanine, cysteine hydrochloride, or cysteine) to Cricket protein, were improved in smoothness compared to sample 15P-4 with the addition of PLD but without addition of the above-mentioned auxiliary materials.

From the results of Table 34-22, samples 15Q-5 to 15Q-10, obtained by adding PLD and an auxiliary material (threonine, manganese-containing yeast, glutathione-containing yeast extract, alanine, cysteine hydrochloride, or cysteine) to Big Cricket protein, were improved in smoothness compared to sample 15Q-4 with the addition of PLD but without addition of the above-mentioned auxiliary materials.

From the results of Table 34-23, samples 15R-5 to 15R-10, obtained by adding PLD and an auxiliary material (threonine, manganese-containing yeast, glutathione-containing yeast extract, alanine, cysteine hydrochloride, or cysteine) to Silkworm protein, were improved in smoothness compared to sample 15R-4 with the addition of PLD but without addition of the above-mentioned auxiliary materials.

From the results of Table 34-24, samples 15S-5 to 15S-10, obtained by adding PLD and an auxiliary material (threonine, manganese-containing yeast, glutathione-containing yeast extract, alanine, cysteine hydrochloride, or cysteine) to spirulina protein, were improved in smoothness compared to sample 15S-4 with the addition of PLD but without addition of the above-mentioned auxiliary materials.

### [Experimental Example 16] Confirmation of effect of adding phospholipase D alone and effect of combined use of phospholipase D and auxiliary materials, in various protein systems

The trade name, company name, and protein content of various proteins used are shown in Table 35.

Each protein gel sample was prepared according to the sample preparation flow shown in Fig. 10, using the mixing recipes shown in Tables 35-1 to 35-3. The prepared samples exhibit the properties of either a suspension or sol or gel.

The obtained each sample was subjected to a sensory evaluation of smoothness by three expert panelists according to the following evaluation criteria. The results are shown in Tables 36-1 to 36-3.

### [Evaluation criteria (smoothness)]

smoothness: compared to control
5 points: very smooth texture
4 points: smooth texture
3 points: slightly smooth texture
2 points: the same
1 point: gritty texture

**[Table 35]**

| name of milk | trade name | protein content | company name |
|---|---|---|---|
| soy milk | Delicious Unsweetened Soy Milk | 8 wt% | Kikkoman Corporation |
| oat milk | alpro | 0.2 wt% | DANONE JAPAN |
| coconut milk | coconut milk | 2 wt% | YOUKI FOOD Co., Ltd. |

**[Table 35-1]**

| <Mixing recipe> unit: wt% | | | | | | | | |
|---|---|---|---|---|---|---|---|---|
| raw material | sample No. | | | | | | | |
| | 16A-1 (control) | 16A-2 | 16A-3 | 16A-4 | 16A-5 | 16A-6 | 16A-7 | 16A-8 |
| soy milk | 100.0 | 100.0 | 100.0 | 100.0 | 100.0 | 100.0 | 100.0 | 100.0 |
| DENAZYME PMD-P1 (*1) | | 0.003 | 0.003 | 0.003 | 0.003 | 0.003 | 0.003 | 0.003 |
| threonine | | | 0.001 | | | | | |
| manganese-containing yeast | | | | 0.001 | | | | |
| glutathione-containing yeast extract | | | | | 0.001 | | | |
| alanine | | | | | | 0.001 | | |
| cysteine hydrochloride | | | | | | | 0.001 | |
| cysteine | | | | | | | | 0.001 |
| total | 100.0 | 100.0 | 100.0 | 100.0 | 100.0 | 100.0 | 100.0 | 100.0 |

| | | | | | | | | |
|---|---|---|---|---|---|---|---|---|
| *1 The amount of PLD in samples 16A-2 to 16A-8 is 20.4 U when converted to enzyme activity for 1 g of protein in the sample. | | | | | | | | |

**[Table 35-2]**

| <Mixing recipe> unit: wt% | | | | | | | | |
|---|---|---|---|---|---|---|---|---|
| raw material | sample No. | | | | | | | |
| | 16B-1 (control) | 16B-2 | 16B-3 | 16B-4 | 16B-5 | 16B-6 | 16B-7 | 16B-8 |
| oat milk | 100.0 | 100.0 | 100.0 | 100.0 | 100.0 | 100.0 | 100.0 | 100.0 |
| DENAZYME PMD-P1 (*1) | | 0.003 | 0.003 | 0.003 | 0.003 | 0.003 | 0.003 | 0.003 |
| threonine | | | 0.001 | | | | | |
| manganese-containing yeast | | | | 0.001 | | | | |
| glutathione-containing yeast extract | | | | | 0.001 | | | |
| alanine | | | | | | 0.001 | | |
| cysteine hydrochloride | | | | | | | 0.001 | |
| cysteine | | | | | | | | 0.001 |
| total | 100.0 | 100.0 | 100.0 | 100.0 | 100.0 | 100.0 | 100.0 | 100.0 |

| | | | | | | | | |
|---|---|---|---|---|---|---|---|---|
| *1 The amount of PLD in samples 16B-2 to 16B-8 is 847.5 U when converted to enzyme activity for 1 g of protein in the sample. | | | | | | | | |

**[Table 35-3]**

| <Mixing recipe> unit: wt% | | | | | | | | |
|---|---|---|---|---|---|---|---|---|
| raw material | sample No. | | | | | | | |
| | 16C-1 (control) | 16C-2 | 16C-3 | 16C-4 | 16C-5 | 16C-6 | 16C-7 | 16C-8 |
| coconut milk | 100.0 | 100.0 | 100.0 | 100.0 | 100.0 | 100.0 | 100.0 | 100.0 |
| DENAZYME PMD-P1 (*1) | | 0.003 | 0.003 | 0.003 | 0.003 | 0.003 | 0.003 | 0.003 |
| threonine | | | 0.001 | | | | | |
| manganese-containing yeast | | | | 0.001 | | | | |
| glutathione-containing yeast extract | | | | | 0.001 | | | |
| alanine | | | | | | 0.001 | | |
| cysteine hydrochloride | | | | | | | 0.001 | |
| cysteine | | | | | | | | 0.001 |
| total | 100.0 | 100.0 | 100.0 | 100.0 | 100.0 | 100.0 | 100.0 | 100.0 |

| | | | | | | | | |
|---|---|---|---|---|---|---|---|---|
| *1 The amount of PLD in samples 16C-2 to 16C-8 is 89.2 U when converted to enzyme activity for 1 g of protein in the sample. | | | | | | | | |

**[Table 36-1]**

| | sample No. | | | | | | | |
|---|---|---|---|---|---|---|---|---|
| | 16A-1 | 16A-2 | 16A-3 | 16A-4 | 16A-5 | 16A-6 | 16A-7 | 16A-8 |
| smoothness | - | 3 | 4 | 3.5 | 4 | 3.5 | 4 | 4 |

**[Table 36-2]**

| | sample No. | | | | | | | |
|---|---|---|---|---|---|---|---|---|
| | 16B-1 | 16B-2 | 16B-3 | 16B-4 | 16B-5 | 16B-6 | 16B-7 | 16B-8 |
| smoothness | - | 2.5 | 3.5 | 3 | 3 | 3 | 3.5 | 3.5 |

**[Table 36-3]**

| | sample No. | | | | | | | |
|---|---|---|---|---|---|---|---|---|
| | 16C-1 | 16C-2 | 16C-3 | 16C-4 | 16C-5 | 16C-6 | 16C-7 | 16C-8 |
| smoothness | - | 3 | 4.5 | 3.5 | 3.5 | 3.5 | 4.5 | 4.5 |

From the results of Table 36-1, samples 16A-2 to 16A-8, obtained by adding PLD to soy milk, were improved in smoothness compared to sample 16A-1 (control). In addition, 16A-3 to 16A-8, obtained by adding PLD and an auxiliary material (threonine, manganese-containing yeast, glutathione-containing yeast extract, alanine, cysteine hydrochloride, or cysteine) to soy milk, were improved in smoothness compared to sample 16A-2 with the addition of PLD but without addition of the above-mentioned auxiliary materials.

From the results of Table 36-2, samples 16B-2 to 16B-8, obtained by adding PLD to oat milk, were improved in smoothness compared to sample 16B-1 (control). In addition, 16B-3 to 16B-8, obtained by adding PLD and an auxiliary material (threonine, manganese-containing yeast, glutathione-containing yeast extract, alanine, cysteine hydrochloride, or cysteine) to oat milk, were improved in smoothness compared to sample 16B-2 with the addition of PLD but without addition of the above-mentioned auxiliary materials.

From the results of Table 36-3, samples 16C-2 to 16C-8, obtained by adding PLD to coconut milk, were improved in smoothness compared to sample 16C-1 (control). In addition, 16C-3 to 16C-8, obtained by adding PLD and an auxiliary material (threonine, manganese-containing yeast, glutathione-containing yeast extract, alanine, cysteine hydrochloride, or cysteine) to coconut milk, were improved in smoothness compared to sample 16C-2 with the addition of PLD but without addition of the above-mentioned auxiliary materials.

### [Experimental Example 17] Confirmation of effect of adding phospholipase D alone and effect of combined use of phospholipase D and auxiliary materials, in various protein systems,

The trade name, company name, and protein content of various proteins used are shown in Table 37.

Each protein gel sample was prepared according to the sample preparation flow shown in Fig. 11, using the mixing recipes shown in Tables 37-1 to 37-3.

The obtained each sample was subjected to a sensory evaluation of smoothness by three expert panelists according to the following evaluation criteria. The results are shown in Tables 38-1 to 38-3.

### [Evaluation criteria (smoothness)]

smoothness: compared to control
5 points: very smooth texture
4 points: smooth texture
3 points: slightly smooth texture
2 points: the same
1 point: gritty texture

**[Table 37]**

| meat name | product name | protein content | company name |
|---|---|---|---|
| beef belly (lean only) | beef belly (lean only) | 21 wt% | Tokyo Packer Co., Ltd. |
| pork arm (lean only) | pork arm (lean only) | 22 wt% | Tokyo Packer Co., Ltd. |
| chicken breast | chicken breast | 23 wt% | Tokyo Packer Co., Ltd. |

**[Table 37-1]**

| <Mixing recipe> unit: wt% | | | | | | | | |
|---|---|---|---|---|---|---|---|---|
| raw material | sample No. | | | | | | | |
| | 17A-1 (control) | 17A-2 | 17A-3 | 17A-4 | 17A-5 | 617A- | 17A-7 | 17A-8 |
| beef belly (lean only) | 82.6 | 82.6 | 82.6 | 82.6 | 82.6 | 82.6 | 82.6 | 82.6 |
| sodium chloride | 0.9 | 0.9 | 0.9 | 0.9 | 0.9 | 0.9 | 0.9 | 0.9 |
| water | 16.5 | 16.5 | 16.5 | 16.5 | 16.5 | 16.5 | 16.5 | 16.5 |
| DENAZYME PMD-P1 (*1) | | 0.003 | 0.003 | 0.003 | 0.003 | 0.003 | 0.003 | 0.003 |
| threonine | | | 0.001 | | | | | |
| manganese-containing yeast | | | | 0.001 | | | | |
| glutathione-containing yeast extract | | | | | 0.001 | | | |
| alanine | | | | | | 0.001 | | |
| cysteine hydrochloride | | | | | | | 0.001 | |
| cysteine | | | | | | | | 0.001 |
| total | 100.0 | 100.0 | 100.0 | 100.0 | 100.0 | 100.0 | 100.0 | 100.0 |

| | | | | | | | | |
|---|---|---|---|---|---|---|---|---|
| *1 The amount of PLD in samples 17A-2 to 17A-8 is 9.6 U when converted to enzyme activity for 1 g of protein in the sample. | | | | | | | | |

**[Table 37-2]**

| <Mixing recipe> unit: wt% | | | | | | | | |
|---|---|---|---|---|---|---|---|---|
| raw material | sample No. | | | | | | | |
| | 17B-1 (control) | 17B-2 | 17B-3 | 17B-4 | 17B-5 | 17B-6 | 17B-7 | 17B-8 |
| pork arm (lean only) | 82.6 | 82.6 | 82.6 | 82.6 | 82.6 | 82.6 | 82.6 | 82.6 |
| sodium chloride | 0.9 | 0.9 | 0.9 | 0.9 | 0.9 | 0.9 | 0.9 | 0.9 |
| water | 16.5 | 16.5 | 16.5 | 16.5 | 16.5 | 16.5 | 16.5 | 16.5 |
| DENAZYME PMD-P1 (*1) | | 0.003 | 0.003 | 0.003 | 0.003 | 0.003 | 0.003 | 0.003 |
| threonine | | | 0.001 | | | | | |
| manganese-containing yeast | | | | 0.001 | | | | |
| glutathione-containing yeast extract | | | | | 0.001 | | | |
| alanine | | | | | | 0.001 | | |
| cysteine hydrochloride | | | | | | | 0.001 | |
| cysteine | | | | | | | | 0.001 |
| total | 100.0 | 100.0 | 100.0 | 100.0 | 100.0 | 100.0 | 100.0 | 100.0 |

| | | | | | | | | |
|---|---|---|---|---|---|---|---|---|
| *1 The amount of PLD in samples 17B-2 to 17B-8 is 9.3 U when converted to enzyme activity for 1 g of protein in the sample. | | | | | | | | |

**[Table 37-3]**

| <Mixing recipe> unit: wt% | | | | | | | | |
|---|---|---|---|---|---|---|---|---|
| raw material | sample No. | | | | | | | |
| | 17C-1 (control) | 17C-2 | 17C-3 | 17C-4 | 17C-5 | 17C-6 | 17C-7 | 17C-8 |
| chicken breast | 82.6 | 82.6 | 82.6 | 82.6 | 82.6 | 82.6 | 82.6 | 82.6 |
| sodium chloride | 0.9 | 0.9 | 0.9 | 0.9 | 0.9 | 0.9 | 0.9 | 0.9 |
| water | 16.5 | 16.5 | 16.5 | 16.5 | 16.5 | 16.5 | 16.5 | 16.5 |
| DENAZYME PMD-P1 (*1) | | 0.003 | 0.003 | 0.003 | 0.003 | 0.003 | 0.003 | 0.003 |
| threonine | | | 0.001 | | | | | |
| manganese-containing yeast | | | | 0.001 | | | | |
| glutathione-containing yeast extract | | | | | 0.001 | | | |
| alanine | | | | | | 0.001 | | |
| cysteine hydrochloride | | | | | | | 0.001 | |
| cysteine | | | | | | | | 0.001 |
| total | 100.0 | 100.0 | 100.0 | 100.0 | 100.0 | 100.0 | 100.0 | 100.0 |

| | | | | | | | | |
|---|---|---|---|---|---|---|---|---|
| *1 The amount of PLD in samples 17C-2 to 17C-8 is 8.8 U when converted to enzyme activity for 1 g of protein in the sample. | | | | | | | | |

**[Table 38-1]**

| | sample No. | | | | | | | |
|---|---|---|---|---|---|---|---|---|
| | 17A-1 | 17A-2 | 17A-3 | 17A-4 | 17A-5 | 17A-6 | 17A-7 | 17A-8 |
| smoothness | - | 3 | 4.5 | 3.5 | 4.5 | 4 | 4.5 | 4.5 |

**[Table 38-2]**

| | sample No. | | | | | | | |
|---|---|---|---|---|---|---|---|---|
| | 17B-1 | 17B-2 | 17B-3 | 17B-4 | 17B-5 | 17B-6 | 17B-7 | 17B-8 |
| smoothness | - | 3 | 4 | 3.5 | 4 | 4 | 4 | 4 |

**[Table 38-3]**

| | sample No. | | | | | | | |
|---|---|---|---|---|---|---|---|---|
| | 17C-1 | 17C-2 | 17C-3 | 17C-4 | 17C-5 | 17C-6 | 17C-7 | 17C-8 |
| smoothness | - | 3 | 4.5 | 3.5 | 4.5 | 4 | 4.5 | 4.5 |

From the results of Table 38-1, samples 17A-2 to 17A-8, obtained by adding PLD to beef belly (lean only), were improved in smoothness compared to sample 17A-1 (control). In addition, 17A-3 to 17A-8, obtained by adding PLD and an auxiliary material (threonine, manganese-containing yeast, glutathione-containing yeast extract, alanine, cysteine hydrochloride, or cysteine) to beef belly (lean only), were improved in smoothness compared to sample 17A-2 with the addition of PLD but without addition of the above-mentioned auxiliary materials.

From the results of Table 38-2, samples 17B-2 to 17B-8, obtained by adding PLD to pork arm (lean only), were improved in smoothness compared to sample 17B-1 (control). In addition, 17B-3 to 17B-8, obtained by adding PLD and an auxiliary material (threonine, manganese-containing yeast, glutathione-containing yeast extract, alanine, cysteine hydrochloride, or cysteine) to pork arm (lean only), were improved in smoothness compared to sample 17B-2 with the addition of PLD but without addition of the above-mentioned auxiliary materials.

From the results of Table 38-3, samples 17C-2 to 17C-8, obtained by adding PLD to chicken breast, were improved in smoothness compared to sample 17C-1 (control). In addition, 17C-3 to 17C-8, obtained by adding PLD and an auxiliary material (threonine, manganese-containing yeast, glutathione-containing yeast extract, alanine, cysteine hydrochloride, or cysteine) to chicken breast, were improved in smoothness compared to sample 17C-2 with the addition of PLD but without addition of the above-mentioned auxiliary materials.

### [Experimental Example 18] Confirmation of effect of adding phospholipase D alone and effect of combined use of phospholipase D and auxiliary materials, in various protein systems

Protein gel samples 18A-1 to 18A-6 were prepared according to the sample preparation flow shown in Fig. 12 (no sitting step (generally a step of leaving a meat paste at a low temperature of around 10 to 40°C for a certain period of time)), using the mixing recipes shown in Table 39-1. The samples prepared exhibit properties of either suspension or sol or gel.

In addition, protein gel samples 18B-1 to 18B-6 were prepared according to the sample preparation flow shown in Fig. 12 (with sitting step), using the mixing recipes shown in Table 39-2.

The obtained each sample was subjected to a sensory evaluation of smoothness by three expert panelists according to the following evaluation criteria. The results are shown in Tables 40-1, 40-2.

### [Evaluation criteria (smoothness)]

smoothness: compared to control
5 points: very smooth texture
4 points: smooth texture
3 points: slightly smooth texture
2 points: the same
1 point: gritty texture

**[Table 39-1]**

| <Mixing recipe> unit: wt% | | | | | | |
|---|---|---|---|---|---|---|
| raw material | sample No. | | | | | |
| | 18A-1 (control) | 18A-2 | 18A-3 | 18A-4 | 18A-5 | 18A-6 |
| hairtail C (*1) | 70.0 | 70.0 | 70.0 | 70.0 | 70.0 | 70.0 |
| sodium chloride | 1.2 | 1.2 | 1.2 | 1.2 | 1.2 | 1.2 |
| water | 28.8 | 28.8 | 28.8 | 28.8 | 28.8 | 28.8 |
| DENAZYME PMD-P1 (*2) | | 0.003 | 0.003 | 0.003 | 0.003 | 0.003 |
| threonine | | | 0.001 | | | |
| manganese-containing yeast | | | | 0.001 | | |
| glutathione-containing yeast extract | | | | | 0.001 | |
| cysteine hydrochloride | | | | | | 0.001 |
| total | 100.0 | 100.0 | 100.0 | 100.0 | 100.0 | 100.0 |

| | | | | | | |
|---|---|---|---|---|---|---|
| *1 hairtail C: protein content: 17 wt% *2 The amount of PLD in samples 18A-2 to 18A-6 is 14.7 U when converted to enzyme activity for 1 g of protein in the sample. | | | | | | |

**[Table 39-2]**

| <Mixing recipe> unit: wt% | | | | | | |
|---|---|---|---|---|---|---|
| raw material | sample No. | | | | | |
| | 18B-1 (control) | 18B-2 | 18B-3 | 18B-4 | 18B-5 | 18B-6 |
| hairtail C (*1) | 70.0 | 70.0 | 70.0 | 70.0 | 70.0 | 70.0 |
| sodium chloride | 1.2 | 1.2 | 1.2 | 1.2 | 1.2 | 1.2 |
| water | 28.8 | 28.8 | 28.8 | 28.8 | 28.8 | 28.8 |
| DENAZYME PMD-P1 (*2) | | 0.003 | 0.003 | 0.003 | 0.003 | 0.003 |
| threonine | | | 0.001 | | | |
| manganese-containing yeast | | | | 0.001 | | |
| glutathione-containing yeast extract | | | | | 0.001 | |
| cysteine hydrochloride | | | | | | 0.001 |
| total | 100.0 | 100.0 | 100.0 | 100.0 | 100.0 | 100.0 |

| | | | | | | |
|---|---|---|---|---|---|---|
| *1 hairtail C: protein content: 17 wt% *2 The amount of PLD in samples 18B-2 to 18B-6 is 14.7 U when converted to enzyme activity for 1 g of protein in the sample. | | | | | | |

**[Table 40-1]**

| | sample No. | | | | | |
|---|---|---|---|---|---|---|
| | 18A-1 | 18A-2 | 18A-3 | 18A-4 | 18A-5 | 18A-6 |
| smoothness | - | 3 | 4 | 3.5 | 4 | 4.5 |

**[Table 40-2]**

| | sample No. | | | | | |
|---|---|---|---|---|---|---|
| | 18B-1 | 18B-2 | 18B-3 | 18B-4 | 18B-5 | 18B-6 |
| smoothness | - | 3 | 4 | 3.5 | 4 | 4.5 |

From the results of Table 40-1, samples 18A-2 to 18A-6, obtained by adding PLD to hairtail C, were improved in smoothness compared to sample 18A-1 (control). In addition, 18A-3 to 18A-6, obtained by adding PLD and an auxiliary material (threonine, manganese-containing yeast, glutathione-containing yeast extract, or cysteine hydrochloride) to hairtail C, were improved in smoothness compared to sample 18A-2 with the addition of PLD but without addition of the above-mentioned auxiliary materials.

From the results of Table 40-2, samples 18B-2 to 17B-6, obtained by adding PLD to hairtail C, were improved in smoothness compared to sample 18B-1 (control). In addition, samples 18B-3 to 18B-6, obtained by adding PLD and an auxiliary material (threonine, manganese-containing yeast, glutathione-containing yeast extract, or cysteine hydrochloride) to hairtail C, were improved in smoothness compared to sample 18B-2 with the addition of PLD but without addition of the above-mentioned auxiliary materials.

### [Experimental Example 19] Comparative verification of phospholipase D and existing materials in soy gel system

Each protein gel sample was prepared according to the sample preparation flow shown in Fig. 10, using the mixing recipes shown in Tables 41-1, 41-2. The prepared samples exhibit the properties of either a suspension or sol or gel.

The obtained each sample was subjected to a sensory evaluation of smoothness and off-taste or off-flavor by three expert panelists according to the following evaluation criteria. The results are shown in Tables 42-1, 42-2.

### [Evaluation criteria (smoothness)]

smoothness: compared to control
5 points: very smooth texture
4 points: smooth texture
3 points: slightly smooth texture
2 points: the same
1 point: gritty texture

### [Evaluation criteria (off-taste or off-flavor)]

off-taste or off-flavor: compared to control
○: no off-taste or off-flavor
×: off-taste or off-flavor

**[Table 41-1]**

| <Mixing recipe> | | | | | | | | | |
|---|---|---|---|---|---|---|---|---|---|
| | | sample No. | | | | | | | |
| | | 19-1 (control) | 19-2 | 19-3 | 19-4 | 19-5 | 19-6 | 19-7 | 19-8 |
| raw material (weight %) | soybean protein (*1) | 10.0 | 10.0 | 10.0 | 10.0 | 10.0 | 10.0 | 10.0 | 10.0 |
| | water | 90.0 | 90.0 | 90.0 | 90.0 | 90.0 | 90.0 | 90.0 | 90.0 |
| | lecithin | | 0.05 | 0.1 | 0.5 | 1.0 | | | |
| | PLA1 | | | | | | 0.00005 | 0.01 | 2.5 |
| | total | 100.0 | 100.1 | 100.1 | 100.5 | 101.0 | 100.0 | 100.0 | 102.5 |
| PLA1 activity (U) for 1 g of protein in sample | | | | | | | 0.065 | 19.5 | 3247.1 |

| | | | | | | | | | |
|---|---|---|---|---|---|---|---|---|---|
| *1 soybean protein: trade name New Fujipro SEH, protein content 87 wt% | | | | | | | | | |

**[Table 41-2]**

| <Mixing recipe> | | | | | | | |
|---|---|---|---|---|---|---|---|
| | | sample No. | | | | | |
| | | 19-9 | 19-10 | 19-11 | 19-12 | 19-13 | 19-14 |
| raw material (weight %) | soybean protein (*1) | 10.0 | 10.0 | 10.0 | 10.0 | 10.0 | 10.0 |
| | water | 90.0 | 90.0 | 90.0 | 90.0 | 90.0 | 90.0 |
| | PLA2 | 0.000005 | 0.002 | 0.3 | | | |
| | DENAZYME PMD-P1 | | | | 0.000010 | 0.003 | 0.5 |
| | total | 100.0 | 100.0 | 100.3 | 100.0 | 100.0 | 100.5 |
| PLA2 activity (U) for 1 g of protein in sample | | 0.065 | 19.5 | 3247.1 | | | |
| PLD activity (U) for 1 g of protein in sample | | | | | 0.065 | 19.5 | 3247.1 |

| | | | | | | | |
|---|---|---|---|---|---|---|---|
| *1 soybean protein: trade name New Fujipro SEH, protein content 87 wt% | | | | | | | |

**[Table 42-1]**

| | sample No. | | | | | | | |
|---|---|---|---|---|---|---|---|---|
| | 19-1 (control) | 19-2 | 19-3 | 19-4 | 19-5 | 19-6 | 19-7 | 19-8 |
| smoothness | - | 2 | 2.2 | 2.2 | 2.2 | 2.2 | reduced viscosity and not evaluable | liquid and not evaluable |
| off-taste or off-flavor | - | ○ | ○ | × | × | ○ | ○ | × |

**[Table 42-2]**

| | sample No. | | | | | |
|---|---|---|---|---|---|---|
| | 19-9 | 19-10 | 19-11 | 19-12 | 19-13 | 19-14 |
| smoothness | 2.2 | 2.2 | 1 | 3 | 3 | 3 |
| off-taste or off-flavor | ○ | ○ | × | ○ | ○ | ○ |

From the results of Tables 42-1, 42-2, samples 19-12 to 19-14, obtained by adding PLD to soy gel, were improved in smoothness compared to sample 19-1 (control). In addition, samples using high amounts of lecithin, PLA1, or PLA2 (samples 19-4, 19-5, 19-8, 19-11) had an off-taste or off-flavor, whereas samples 19-12 to 19-14 containing PLD were shown to be superior in that they had high scores of smoothness and no off-taste or off-flavor.

### [Experimental Example 20] Confirmation of effect of adding phospholipase D in soy gel system produced by one-step heating

Soy gel samples 20-1 to 20-5 were prepared according to the sample preparation flow shown in Fig. 13, using the mixing recipes shown in Table 43. The prepared samples exhibit the properties of either a suspension or sol or gel.

The obtained each sample was subjected to a sensory evaluation of smoothness by three expert panelists according to the same evaluation criteria as in Experimental Example 12, using sample 21-2 as a control. The results are shown in Table 44.

**[Table 43]**

| <Mixing recipe> | | | | | | |
|---|---|---|---|---|---|---|
| | | sample No. | | | | |
| | | 20-1 | 20-2 (control) | 20-3 | 20-4 | 20-5 |
| raw material (weight %) | soybean protein (*1) | | 10.0 | 10.0 | 10.0 | 10.0 |
| | egg white (powder) (*2) | 10.0 | | | | |
| | water | 90.0 | 90.0 | 90.0 | 90.0 | 90.0 |
| | DENAZYME PMD-P1 | | | 0.00000001 | 0.000001 | 0.003 |
| | total | 100.0 | 100.0 | 100.0 | 100.0 | 100.0 |
| PLD activity (U) for 1 g of protein in sample | | | | 0.000065 | 0.0065 | 19.5 |

| | | | | | | |
|---|---|---|---|---|---|---|
| *1 soybean protein: trade name New Fujipro SEH, protein content 87 wt% *2 egg white (powder): protein content 83 wt% | | | | | | |

**[Table 44]**

| | sample No. | | | | |
|---|---|---|---|---|---|
| | 20-1 | 20-2 (control) | 20-3 | 20-4 | 20-5 |
| smoothness | 3 | - | 3 | 3 | 3 |

From the results of Table 44, samples 20-3 to 20-5 obtained by adding PLD to soy gel were improved in smoothness compared to sample 20-2 (control). These results show that the production method of the present invention has the effect of improving smoothness not only in the case of production using two-stage heating shown in Fig. 10, but also in the case of production using one-stage heating shown in Fig. 13.

### [Experimental Example 21] Confirmation of effect of adding phospholipase D to hamburger steak

The trade names, company names, and protein contents of various proteins used are shown in Table 45.

Hamburger steak samples 21-1 to 21-9 were prepared according to the sample preparation flow shown in Fig. 14, using the mixing recipes shown in Table 45-1.

The obtained each sample was subjected to a sensory evaluation of smoothness by three expert panelists according to the same evaluation criteria as in Experimental Example 12, using sample 21-2 as a control. The results are shown in Table 46.

**[Table 45]**

| raw material name | trade name | protein content | company name |
|---|---|---|---|
| lean beef belly | beef belly (lean only) | 21 wt% | Tokyo Packer Co., Ltd. |
| lean pork arm | pork arm meat (lean only) | 22 wt% | Tokyo Packer Co., Ltd. |
| liquid whole egg | whole egg | 12 wt% | Ito-Yokado |
| fresh bread crumbs | fresh bread crumbs | 10 wt% | Ito-Yokado |
| onion | onion | 1 wt% | Ito-Yokado |

**[Table 45-1]**

| <mixing recipe> unit: wt% | | | | | | | | | |
|---|---|---|---|---|---|---|---|---|---|
| raw materials | sample No. | | | | | | | | |
| | 21-1 (control) | 21-2 | 21-3 | 21-4 | 21-5 | 21-6 | 21-7 | 21-8 | 21-9 |
| lean beef belly | 28.50 | 28.50 | 28.50 | 28.50 | 28.50 | 28.50 | 28.50 | 28.50 | 28.50 |
| beef tallow | 13.50 | 13.50 | 13.50 | 13.50 | 13.50 | 13.50 | 13.50 | 13.50 | 13.50 |
| lean pork arm | 13.30 | 13.30 | 13.30 | 13.30 | 13.30 | 13.30 | 13.30 | 13.30 | 13.30 |
| lard | 4.80 | 4.80 | 4.80 | 4.80 | 4.80 | 4.80 | 4.80 | 4.80 | 4.80 |
| liquid whole egg | 5.00 | 5.00 | 5.00 | 5.00 | 5.00 | 5.00 | 5.00 | 5.00 | 5.00 |
| fresh bread crumbs | 6.00 | 6.00 | 6.00 | 6.00 | 6.00 | 6.00 | 6.00 | 6.00 | 6.00 |
| onion | 20.90 | 20.90 | 20.90 | 20.90 | 20.90 | 20.90 | 20.90 | 20.90 | 20.90 |
| water | 6.00 | 6.00 | 6.00 | 6.00 | 6.00 | 6.00 | 6.00 | 6.00 | 6.00 |
| sodium chloride | 0.80 | 0.80 | 0.80 | 0.80 | 0.80 | 0.80 | 0.80 | 0.80 | 0.80 |
| sugar | 1.00 | 1.00 | 1.00 | 1.00 | 1.00 | 1.00 | 1.00 | 1.00 | 1.00 |
| Ajinomoto | 0.10 | 0.10 | 0.10 | 0.10 | 0.10 | 0.10 | 0.10 | 0.10 | 0.10 |
| nutmeg | 0.10 | 0.10 | 0.10 | 0.10 | 0.10 | 0.10 | 0.10 | 0.10 | 0.10 |
| DENAZYME PMD-P1 (*1) | | 0.003 | 0.003 | 0.003 | 0.003 | 0.003 | 0.003 | 0.003 | 0.003 |
| threonine | | | 0.001 | | | | | | |
| glutathione-containing yeast extract | | | | 0.001 | | | | | |
| manganese-containing yeast | | | | | 0.001 | | | | |
| cysteine hydrochloride | | | | | | 0.001 | | | |
| glycine | | | | | | | 0.001 | | |
| alanine | | | | | | | | 0.001 | |
| cysteine | | | | | | | | | 0.001 |
| total | 100.00 | 100.00 | 100.00 | 100.00 | 100.00 | 100.00 | 100.00 | 100.00 | 100.00 |

| | | | | | | | | | |
|---|---|---|---|---|---|---|---|---|---|
| *1 The amount of PLD in samples 21-2 to 21-9 is 16.2 U when converted to enzyme activity for 1 g of protein in the same sample. | | | | | | | | | |

**[Table 46]**

| | sample No. | | | | | | | |
|---|---|---|---|---|---|---|---|---|
| | 21-1 | 21-2 | 21-3 | 21-4 | 21-5 | 21-6 | 21-7 21-8 | 21-9 |
| smoothness | - | 3 | 4 | 3.5 | 3.5 | 4 | 3.5 3.5 | 4 |

From the results of Table 46, samples 21-2 to 21-9 obtained by adding PLD to hamburger steak were improved in smoothness compared to sample 21-1 (control). In addition, 21-3 to 21-9 obtained by adding PLD and an auxiliary material (threonine, manganese-containing yeast, glutathione-containing yeast extract, glycine, alanine, cysteine hydrochloride, or cysteine) to hamburger steak were improved in smoothness compared to sample 21-2 with the addition of PLD but without addition of the above-mentioned auxiliary material.

### [Experimental Example 22] Confirmation of effect of adding phospholipase D to hamburger steak

The trade names, company names, and protein contents of various proteins used are shown in Table 47.

Hamburger steak samples 22-1 to 22-9 were prepared according to the sample preparation flow shown in Fig. 14, using the mixing recipes shown in Table 47-1.

The obtained each sample was subjected to a sensory evaluation of smoothness by three expert panelists according to the same evaluation criteria as in Experimental Example 12, using sample 22-1 as a control. The results are shown in Table 48.

**[Table 47]**

| raw material name | trade name | protein content | company name |
|---|---|---|---|
| lean beef belly | beef belly (lean only) | 21 wt% | Tokyo Packer Co., Ltd. |
| lean pork arm | pork arm meat (lean only) | 22 wt% | Tokyo Packer Co., Ltd. |
| powdered plant-derived protein | New Fujipro SEH | 87 wt% | FUJI OIL CO., LTD. |
| liquid whole egg | whole egg | 12 wt% | Ito-Yokado |
| milk | Meiji Oishii Gyunyu | 3 wt% | Meiji Co., Ltd. |
| bread crumbs | soft bread crumbs | 15 wt% | Ito-Yokado |
| onion | onion | 1 wt% | Ito-Yokado |
| rehydrated granular soybean protein | New Fujinic 52 | 15 wt% | FUJI OIL CO., LTD. |

**[Table 47-1]**

| <mixing recipe> unit: wt% | | | | | | | | | |
|---|---|---|---|---|---|---|---|---|---|
| raw materials | sample No. | | | | | | | | |
| | 22-1 (control) | 22-2 | 22-3 | 22-4 | 22-5 | 22-6 | 22-7 | 22-8 | 22-9 |
| lean beef belly | 14.00 | 14.00 | 14.00 | 14.00 | 14.00 | 14.00 | 14.00 | 14.00 | 14.00 |
| beef tallow | 12.00 | 12.00 | 12.00 | 12.00 | 12.00 | 12.00 | 12.00 | 12.00 | 12.00 |
| lean pork arm | 20.00 | 20.00 | 20.00 | 20.00 | 20.00 | 20.00 | 20.00 | 20.00 | 20.00 |
| lard | 7.00 | 7.00 | 7.00 | 7.00 | 7.00 | 7.00 | 7.00 | 7.00 | 7.00 |
| powdered plant-derived protein | 1.00 | 1.00 | 1.00 | 1.00 | 1.00 | 1.00 | 1.00 | 1.00 | 1.00 |
| liquid whole egg | 4.40 | 4.40 | 4.40 | 4.40 | 4.40 | 4.40 | 4.40 | 4.40 | 4.40 |
| milk | 0.50 | 0.50 | 0.50 | 0.50 | 0.50 | 0.50 | 0.50 | 0.50 | 0.50 |
| bread crumbs | 5.50 | 5.50 | 5.50 | 5.50 | 5.50 | 5.50 | 5.50 | 5.50 | 5.50 |
| onion | 15.00 | 15.00 | 15.00 | 15.00 | 15.00 | 15.00 | 15.00 | 15.00 | 15.00 |
| rehydrated granular soybean protein | 14.00 | 14.00 | 14.00 | 14.00 | 14.00 | 14.00 | 14.00 | 14.00 | 14.00 |
| potato starch | 0.50 | 0.50 | 0.50 | 0.50 | 0.50 | 0.50 | 0.50 | 0.50 | 0.50 |
| water | 3.90 | 3.90 | 3.90 | 3.90 | 3.90 | 3.90 | 3.90 | 3.90 | 3.90 |
| sodium chloride | 0.70 | 0.70 | 0.70 | 0.70 | 0.70 | 0.70 | 0.70 | 0.70 | 0.70 |
| syrup | 1.00 | 1.00 | 1.00 | 1.00 | 1.00 | 1.00 | 1.00 | 1.00 | 1.00 |
| black pepper | 0.20 | 0.20 | 0.20 | 0.20 | 0.20 | 0.20 | 0.20 | 0.20 | 0.20 |
| nutmeg | 0.10 | 0.10 | 0.10 | 0.10 | 0.10 | 0.10 | 0.10 | 0.10 | 0.10 |
| pH adjuster | 0.20 | 0.20 | 0.20 | 0.20 | 0.20 | 0.20 | 0.20 | 0.20 | 0.20 |
| DENAZYME PMD-P1 | | 0.003 | 0.003 | 0.003 | 0.003 | 0.003 | 0.003 | 0.003 | 0.003 |
| threonine | | | 0.001 | | | | | | |
| glutathione-containing yeast extract | | | | 0.001 | | | | | |
| manganese-containing yeast | | | | | 0.001 | | | | |
| cysteine hydrochloride | | | | | | 0.001 | | | |
| glycine | | | | | | | 0.001 | | |
| alanine | | | | | | | | 0.001 | |
| cysteine | | | | | | | | | 0.001 |
| total | 100.00 | 100.00 | 100.00 | 100.00 | 100.00 | 100.00 | 100.00 | 100.00 | 100.00 |

The amount of PLD in samples 22-2 to 22-9 is 14.2 U when converted to enzyme activity for 1 g of protein in the same sample.

**[Table 48]**

| | sample No. | | | | | | | | |
|---|---|---|---|---|---|---|---|---|---|
| | 22-1 | 22-2 | 22-3 | 22-4 | 22-5 | 22-6 | 22-7 | 22-8 | 22-9 |
| smoothness | - | 3 | 4.5 | 4 | 3.5 | 4.5 | 4 | 4 | 4.5 |

From the results of Table 48, samples 22-2 to 22-9 with the addition of PLD to hamburger steak were improved in smoothness compared to sample 22-1 (control). In addition, 22-3 to 22-9 obtained by adding PLD and an auxiliary material (threonine, manganese-containing yeast, glutathione-containing yeast extract, glycine, alanine, cysteine hydrochloride, or cysteine) to hamburger steak were improved in smoothness compared to sample 22-2 with the addition of PLD but without addition of the above-mentioned auxiliary material.

### [Experimental Example 23] Confirmation of effect of adding phospholipase D in loin ham system

Pickling liquid samples 23-1 to 23-11 were prepared according to the sample preparation flow shown in Fig. 15, using the mixing recipes shown in Table 49-1, Table 49-2. Successively, ham samples 23-1 to 23-11 were prepared according to the sample preparation flow shown in Fig. 15, using the obtained pickling liquid samples 23-1 to 23-11, respectively, and raw material meat (Danish pork loin). The mixing ratio (weight ratio) of the raw material meat and the pickling liquid was 1:1.

The obtained ham samples 23-1 to 23-11 were subjected to a sensory evaluation of smoothness, hardness, elasticity, and overall evaluation by three expert panelists according to the following evaluation criteria. The results are shown in Table 50.

### [Evaluation criteria (smoothness)]

smoothness: as ham
5 points: very smooth texture and favorable
4 points: smooth texture and favorable
3 points: slightly smooth texture and favorable
2 points: average texture
1 point: texture is gritty and unfavorable

### [Evaluation criteria (hardness)]

hardness: as ham
5 points: very hard texture and favorable
4 points: hard texture and favorable
3 points: slightly hard texture and favorable
2 points: average texture
1 point: soft texture and unfavorable

### [Evaluation criteria (elasticity)]

elasticity: as ham
5 points: very flexible texture and favorable
4 points: flexible texture and favorable
3 points: slightly flexible texture and favorable
2 points: average texture
1 point: brittle texture and unfavorable

### [Evaluation criteria (overall evaluation)]

overall evaluation: as ham
⊙: very favorable texture and flavor
○: favorable texture and flavor
Δ: slightly favorable texture and flavor
×: unfavorable texture and flavor

**[Table 49-1]**

| <pickling liquid mixing recipe> | | | | | | | |
|---|---|---|---|---|---|---|---|
| raw material group | raw material name | pickling liquid sample No. | | | | | |
| | | 23-1 | 23-2 | 23-3 | 23-4 | 23-5 | 23-6 |
| | | mixing ratio (wt%) | mixing ratio (wt%) | mixing ratio (wt%) | mixing ratio (wt%) | mixing ratio (wt%) | mixing ratio (wt%) |
| B | egg white (powder) <egg white powder> (*1) | 2.5 | 1.25 | 1.25 | 1.25 | 1.25 | 1.25 |
| | soybean protein <New Fujipro IJN> (*2) | 5 | 6.25 | 6.25 | 6.25 | 6.25 | 6.25 |
| | milk protein <Super-Lact No.1> (*3) | 1 | 1 | 1 | 1 | 1 | 1 |
| | casein Na <Casein Sodium LW> (*4) | 0.2 | 0.2 | 0.2 | 0.2 | 0.2 | 0.2 |
| C | ascorbic acid Na <Sodium L-ascorbate> | 0.2 | 0.2 | 0.2 | 0.2 | 0.2 | 0.2 |
| | sodium chloride <NAKURU M> | 2.3 | 2.3 | 2.3 | 2.3 | 2.3 | 2.3 |
| | 10% sodium nitrite preparation <Highcolor HL> | 0.4 | 0.4 | 0.4 | 0.4 | 0.4 | 0.4 |
| D | "Ajinomoto" <MSG-FC> | 0.5 | 0.5 | 0.5 | 0.5 | 0.5 | 0.5 |
| | carrageenan <SATIGEL RPI730> | 0.3 | 0.3 | 0.3 | 0.3 | 0.3 | 0.3 |
| | reduced starch syrup <Amameal> | 10 | 10 | 10 | 10 | 10 | 10 |
| A | polyphosphosphate preparation <Polygon C> | 0.4 | 0.4 | 0.4 | 0.4 | 0.4 | 0.4 |
| | cochineal color <SR Red K-6> | 0.1 | 0.1 | 0.1 | 0.1 | 0.1 | 0.1 |
| water | | 77.1 | 77.1 | 77.1 | 77.1 | 77.1 | 77.1 |
| total | | 100 | 100 | 100 | 100 | 100 | 100 |
| DENAZYME PMD-P1 (*5) | | | | | 0.006 | 0.006 | 0.006 |
| threonine | | | | | | 0.002 | |
| glutathione-containing yeast extract | | | | | | | 0.002 |
| TG preparation (*6) | | | | 0.2 | 0.2 | 0.2 | 0.2 |

| | | | | | | | |
|---|---|---|---|---|---|---|---|
| *1 egg white (powder): protein content 83 wt% *2 soybean protein: protein content 71 wt% *3 milk protein: protein content 70 wt% *4 casein Na: protein content 89 wt% *5 The amount of PLD in pickling liquid samples 23-4 to 23-6 is 60.0 U when converted to enzyme activity for 1 g of protein in the same sample. *6 TG preparation (trade name Activa (registered trademark) TG-H-NF): thereof TG (transglutaminase) 0.4 wt%; the number of TG units per 1 g of TG preparation is 45 U. | | | | | | | |

The amount of TG in pickling liquid samples 23-3 to 23-6 is 1.6 U when converted to enzyme activity for 1 g of protein in the same sample.

**[Table 49-2]**

| <pickling liquid mixing recipe> | | | | | | |
|---|---|---|---|---|---|---|
| raw material group | raw material name | pickling liquid sample No. | | | | |
| | | 23-7 | 23-8 | 23-9 | 23-10 | 23-11 |
| | | mixing ratio (wt%) | mixing ratio (wt%) | mixing ratio (wt%) | mixing ratio (wt%) | mixing ratio (wt%) |
| B | egg white (powder) <egg white powder> (*1) | 1.25 | 1.25 | 1.25 | 1.25 | 1.25 |
| | soybean protein <New Fujipro IJN> (*2) | 6.25 | 6.25 | 6.25 | 6.25 | 6.25 |
| | milk protein <Super-Lact No.1> (*3) | 1 | 1 | 1 | 1 | 1 |
| | casein Na <Casein Sodium LW> (*4) | 0.2 | 0.2 | 0.2 | 0.2 | 0.2 |
| C | ascorbic acid Na <Sodium L-ascorbate> | 0.2 | 0.2 | 0.2 | 0.2 | 0.2 |
| | sodium chloride <NAKURU M> | 2.3 | 2.3 | 2.3 | 2.3 | 2.3 |
| | 10% sodium nitrite preparation <Highcolor HL> | 0.4 | 0.4 | 0.4 | 0.4 | 0.4 |
| D | "Ajinomoto" <MSG-FC> | 0.5 | 0.5 | 0.5 | 0.5 | 0.5 |
| | carrageenan <SATIGEL RPI730> | 0.3 | 0.3 | 0.3 | 0.3 | 0.3 |
| | reduced starch syrup <Amameal> | 10 | 10 | 10 | 10 | 10 |
| A | polyphosphosphate preparation <Polygon C> | 0.4 | 0.4 | 0.4 | 0.4 | 0.4 |
| | cochineal color <SR Red K-6> | 0.1 | 0.1 | 0.1 | 0.1 | 0.1 |
| water | | 77.1 | 77.1 | 77.1 | 77.1 | 77.1 |
| total | | 100 | 100 | 100 | 100 | 100 |
| DENAZYME PMD-P1 (*5) | | 0.006 | 0.006 | 0.006 | 0.006 | 0.006 |
| manganese-containing yeast | | 0.002 | | | | |
| cysteine hydrochloride | | | 0.002 | | | |
| glycine | | | | 0.002 | | |
| alanine | | | | | 0.002 | |
| cysteine | | | | | | 0.002 |
| TG preparation (*6) | | 0.2 | 0.2 | 0.2 | 0.2 | 0.2 |

| | | | | | | |
|---|---|---|---|---|---|---|
| *1 egg white (powder): protein content 83 wt% *2 soybean protein: protein content 71 wt% *3 milk protein: protein content 70 wt% *4 casein Na: protein content 89 wt% *5 The amount of PLD in pickling liquid samples 23-7 to 23-11 is 60.0 U when converted to enzyme activity for 1 g of protein in the same sample. *6 TG preparation (trade name Activa (registered trademark) TG-H-NF): thereof TG (transglutaminase) 0.4 wt%; the number of TG units per 1 g of TG preparation is 45 U. | | | | | | |

The amount of TG in pickling liquid samples 23-7 to 23-11 is 1.6 U when converted to enzyme activity for 1 g of protein in the same sample.

**[Table 50]**

| | sample No. | | | | | | | | | | |
|---|---|---|---|---|---|---|---|---|---|---|---|
| | 23-1 | 23-2 | 23-3 | 23-4 | 23-5 | 23-6 | 23-7 | 23-8 | 23-9 | 23-10 | 23-11 |
| smoothness | 3 | - | 2 | 3 | 4 | 4 | 3.5 | 4 | 3.5 | 3.5 | 4 |
| hardness | 4 | - | 4 | 4 | 4 | 4 | 4 | 4 | 4 | 4 | 4 |
| elasticity | 4 | - | 4 | 4 | 4 | 4 | 4 | 4 | 4 | 4 | 4 |
| overall evaluation | ○ | Δ | Δ | ○ | ⊙ | ⊙ | ⊙ | ⊙ | ⊙ | ⊙ | ⊙ |

From the results of Table 50, samples 23-4 to 23-11 with the further addition of PLD and a TG preparation to a mixture of egg white, soybean protein, milk protein, and casein Na were improved in smoothness, hardness, and elasticity compared to sample 23-2 (control). In addition, samples 23-5 to 23-11 with the further addition of an auxiliary material (threonine, manganese-containing yeast, glutathione-containing yeast extract, glycine, alanine, cysteine hydrochloride, or cysteine) were improved in smoothness compared to sample 23-4 without addition of the above-mentioned auxiliary material.

### [Experimental Example 24] Confirmation of effect of adding phospholipase D to tofu

Tofu samples 24-1 to 24-8 were prepared according to the sample preparation flow shown in Fig. 16, using the bittern liquid with the composition shown in Table 51 and the mixing recipes shown in Table 52.

The obtained each sample was subjected to a sensory evaluation of smoothness by three expert panelists according to the same evaluation criteria as in Experimental Example 12, using sample 24-1 as a control. The results are shown in Table 53.

**[Table 51]**

| <bittern liquid added> | |
|---|---|
| raw material | ratio (wt%) |
| magnesium chloride | 50.0 |
| water | 50.0 |
| total | 100.0 |

**[Table 52]**

| <mixing recipe> unit: wt% | | | | | | | | |
|---|---|---|---|---|---|---|---|---|
| raw materials | sample No. | | | | | | | |
| | 24-1 (control) | 24-2 | 24-3 | 24-4 | 24-5 | 24-6 | 24-7 | 24-8 |
| unsweetened soy milk (*1) | 99.45 | 99.45 | 99.45 | 99.45 | 99.45 | 99.45 | 99.45 | 99.45 |
| bittern liquid | 0.55 | 0.55 | 0.55 | 0.55 | 0.55 | 0.55 | 0.55 | 0.55 |
| DENAZYME PMD-P1 (*2) | | 0.0014 | 0.0014 | 0.0014 | 0.0014 | 0.0014 | 0.0014 | 0.0014 |
| threonine | | | 0.0005 | | | | | |
| glutathione-containing yeast extract | | | | 0.0005 | | | | |
| manganese-containing yeast | | | | | 0.0005 | | | |
| cysteine hydrochloride | | | | | | 0.0005 | | |
| alanine | | | | | | | 0.0005 | |
| cysteine | | | | | | | | 0.0005 |
| total | 100.00 | 100.00 | 100.00 | 100.00 | 100.00 | 100.00 | 100.00 | 100.00 |

| | | | | | | | | |
|---|---|---|---|---|---|---|---|---|
| *1 unsweetened soy milk: trade name Shigezo unsweetened rich soy milk (Shinozakiya Co., Ltd.), protein content 4 wt% *2 The amount of PLD in samples 24-2 to 24-8 is 18.6 U when converted to enzyme activity for 1 g of protein in the same sample. | | | | | | | | |

**[Table 53]**

| | sample No. | | | | | | | |
|---|---|---|---|---|---|---|---|---|
| | 24-1 | 24-2 | 24-3 | 24-4 | 24-5 | 24-6 | 24-7 | 24-8 |
| smoothness | - | 3 | 4.5 | 4 | 3.5 | 4.5 | 3.5 | 4.5 |

From the results of Table 53, samples 24-2 to 24-8 with the addition of PLD to tofu were improved in smoothness compared to sample 24-1 (control). In addition, samples 24-3 to 24-8 obtained by adding PLD and an auxiliary material (threonine, manganese-containing yeast, glutathione-containing yeast extract, cysteine hydrochloride, alanine, or cysteine) to tofu were improved in smoothness compared to sample 24-2 with the addition of PLD but without addition of the above-mentioned auxiliary material.

### [Experimental Example 25] Confirmation of effect of adding phospholipase D to plant based (PB) cheese

PB cheese samples 25-1 to 25-9 were prepared according to the sample preparation flow shown in Fig. 17, using the mixing recipes shown in Table 54.

The obtained each sample was subjected to a sensory evaluation of smoothness by three expert panelists according to the same evaluation criteria as in Experimental Example 12, using sample 25-1 as a control. The results are shown in Table 55.

**[Table 54]**

| <mixing recipe> unit: wt% | | | | | | | | | |
|---|---|---|---|---|---|---|---|---|---|
| raw materials | sample No. | | | | | | | | |
| | 25-1 (control) | 25-2 | 25-3 | 25-4 | 25-5 | 25-6 | 25-7 | 25-8 | 25-9 |
| Waxy Corn Starch | 10.17 | 10.17 | 10.17 | 10.17 | 10.17 | 10.17 | 10.17 | 10.17 | 10.17 |
| Tapioca Starch | 10.30 | 10.30 | 10.30 | 10.30 | 10.30 | 10.30 | 10.30 | 10.30 | 10.30 |
| Gum Arabic | 1.83 | 1.83 | 1.83 | 1.83 | 1.83 | 1.83 | 1.83 | 1.83 | 1.83 |
| Tamarind Seed Gum | 0.30 | 0.30 | 0.30 | 0.30 | 0.30 | 0.30 | 0.30 | 0.30 | 0.30 |
| Savorboost F | 0.15 | 0.15 | 0.15 | 0.15 | 0.15 | 0.15 | 0.15 | 0.15 | 0.15 |
| Yeastock Spd AT | 0.80 | 0.80 | 0.80 | 0.80 | 0.80 | 0.80 | 0.80 | 0.80 | 0.80 |
| Coconut Oil | 20.00 | 20.00 | 20.00 | 20.00 | 20.00 | 20.00 | 20.00 | 20.00 | 20.00 |
| Water | 40.50 | 40.50 | 40.50 | 40.50 | 40.50 | 40.50 | 40.50 | 40.50 | 40.50 |
| Pea Protein | 5.95 | 5.95 | 5.95 | 5.95 | 5.95 | 5.95 | 5.95 | 5.95 | 5.95 |
| Fava Protein | 8.30 | 8.30 | 8.30 | 8.30 | 8.30 | 8.30 | 8.30 | 8.30 | 8.30 |
| Salt | 1.40 | 1.40 | 1.40 | 1.40 | 1.40 | 1.40 | 1.40 | 1.40 | 1.40 |
| Lactic Acid | 0.30 | 0.30 | 0.30 | 0.30 | 0.30 | 0.30 | 0.30 | 0.30 | 0.30 |
| DENAZYME PMD-P1 (*1) | | 0.003 | 0.003 | 0.003 | 0.003 | 0.003 | 0.003 | 0.003 | 0.003 |
| threonine | | | 0.001 | | | | | | |
| glutathione-containing yeast extract | | | | 0.001 | | | | | |
| manganese-containing yeast | | | | | 0.001 | | | | |
| cysteine hydrochloride | | | | | | 0.001 | | | |
| glycine | | | | | | | 0.001 | | |
| alanine | | | | | | | | 0.001 | |
| cysteine | | | | | | | | | 0.001 |
| total | 100.00 | 100.00 | 100.00 | 100.00 | 100.00 | 100.00 | 100.00 | 100.00 | 100.00 |

| | | | | | | | | | |
|---|---|---|---|---|---|---|---|---|---|
| *1 The amount of PLD in samples 25-2 to 25-9 is 17 U when converted to enzyme activity for 1 g of protein in the same sample. | | | | | | | | | |

**[Table 55]**

| | sample No. | | | | | | | | |
|---|---|---|---|---|---|---|---|---|---|
| | 25-1 | 25-2 | 25-3 | 25-4 | 25-5 | 25-6 | 25-7 | 25-8 | 25-9 |
| smoothness | - | 3 | 4.5 | 4 | 4 | 4.5 | 4 | 4 | 4.5 |

From the results of Table 55, samples 25-2 to 25-9 with the addition of a PLD preparation to PB cheese were improved in smoothness compared to sample 25-1 (control). In addition, samples 25-3 to 25-9 obtained by adding a PLD preparation and an auxiliary material (threonine, manganese-containing yeast, glutathione-containing yeast extract, cysteine hydrochloride, glycine, alanine, or cysteine) to PB cheese were improved in smoothness compared to sample 25-2 with the addition of a PLD preparation but without addition of the above-mentioned auxiliary material.

### [Experimental Example 26] Confirmation of effect of adding phospholipase D to PB yogurt

PB yogurt samples 26-1 to 26-9 were prepared according to the sample preparation flow shown in Fig. 18, using the mixing recipes shown in Table 56.

The obtained each sample was subjected to a sensory evaluation of smoothness by three expert panelists according to the same evaluation criteria as in Experimental Example 12, using sample 26-1 as a control. The results are shown in Table 57.

**[Table 56]**

| <mixing recipe> unit: wt% | | | | | | | | | |
|---|---|---|---|---|---|---|---|---|---|
| raw materials | sample No. | | | | | | | | |
| | 26-1 (control) | 26-2 | 26-3 | 26-4 | 26-5 | 26-6 | 26-7 | 26-8 | 26-9 |
| soy milk (*1) | 99.67 | 99.67 | 99.67 | 99.67 | 99.67 | 99.67 | 99.67 | 99.67 | 99.67 |
| starter | 0.33 | 0.33 | 0.33 | 0.33 | 0.33 | 0.33 | 0.33 | 0.33 | 0.33 |
| DENAZYME PMD-P1 (*2) | | 0.002 | 0.002 | 0.002 | 0.002 | 0.002 | 0.002 | 0.002 | 0.002 |
| threonine | | | 0.001 | | | | | | |
| glutathione-containing yeast extract | | | | 0.001 | | | | | |
| manganese-containing yeast | | | | | 0.010 | | | | |
| cysteine hydrochloride | | | | | | 0.001 | | | |
| glycine | | | | | | | 0.001 | | |
| alanine | | | | | | | | 0.001 | |
| cysteine | | | | | | | | | 0.001 |
| total | 100.00 | 100.00 | 100.00 | 100.00 | 100.01 | 100.00 | 100.00 | 100.00 | 100.00 |

| | | | | | | | | | |
|---|---|---|---|---|---|---|---|---|---|
| *1 soy milk: trade name Delicious Unsweetened Soy Milk (KIKKOMAN CORPORATION), protein content 8.3 wt% *2 The amount of PLD in samples 26-2 to 26-9 is 20 U when converted to enzyme activity for 1 g of protein in the same sample. | | | | | | | | | |

**[Table 57]**

| | sample No. | | | | | | | | |
|---|---|---|---|---|---|---|---|---|---|
| | 26-1 | 26-2 | 26-3 | 26-4 | 26-5 | 26-6 | 26-7 | 26-8 | 26-9 |
| smoothness | - | 3 | 4.5 | 4 | 4 | 4.5 | 4 | 4 | 4.5 |

From the results of Table 57, samples 26-2 to 26-9 with the addition of a PLD preparation to PB yogurt were improved in smoothness compared to sample 26-1 (control). In addition, samples 26-3 to 26-9 obtained by adding a PLD preparation and an auxiliary material (threonine, manganese-containing yeast, glutathione-containing yeast extract, cysteine hydrochloride, glycine, alanine, or cysteine) to PB yogurt were improved in smoothness compared to sample 26-2 with the addition of a PLD preparation but without addition of the above-mentioned auxiliary material.

### [Experimental Example 27] Confirmation of effect of adding phospholipase D in PB egg

PB egg samples 27-1 to 27-9 were prepared according to the sample preparation flow shown in Fig. 19, using the mixing recipes shown in Table 58.

The obtained each sample was subjected to a sensory evaluation of smoothness by three expert panelists according to the same evaluation criteria as in Experimental Example 12, using sample 27-1 as a control. The results are shown in Table 59.

**[Table 58]**

| <mixing recipe> unit: wt% | | | | | | | | | |
|---|---|---|---|---|---|---|---|---|---|
| raw materials | sample No. | | | | | | | | |
| | 27-1 (control) | 27-2 | 27-3 | 27-4 | 27-5 | 27-6 | A 27-7 | 27-8 | 27-9 |
| plant-derived egg-like liquid (*1) | 100.00 | 100.00 | 100.00 | 100.00 | 100.00 | 100.00 | 100.00 | 100.00 | 100.00 |
| DENAZYME PMD-P1 (*2) | | 0.003 | 0.003 | 0.003 | 0.003 | 0.003 | 0.003 | 0.003 | 0.003 |
| threonine | | | 0.001 | | | | | | |
| glutathione-containing yeast extract | | | | 0.001 | | | | | |
| manganese-containing yeast | | | | | 0.001 | | | | |
| cysteine hydrochloride | | | | | | 0.001 | | | |
| glycine | | | | | | | 0.001 | | |
| alanine | | | | | | | | 0.001 | |
| cysteine | | | | | | | | | 0.001 |
| total | 100.00 | 100.00 | 100.00 | 100.00 | 100.00 | 100.00 | 100.00 | 100.00 | 100.00 |

| | | | | | | | | | |
|---|---|---|---|---|---|---|---|---|---|
| *1 plant-derived egg-like liquid: trade name HOBOTAMA liquid egg-like for heating (Kewpie Co., Ltd.) protein content 4.2 wt% *2 The amount of PLD in samples 27-2 to 27-9 is 40 U when converted to enzyme activity for 1 g of protein in the same sample. | | | | | | | | | |

**[Table 59]**

| | sample No. | | | | | | | | |
|---|---|---|---|---|---|---|---|---|---|
| | 27-1 | 27-2 | 27-3 | 27-4 | 27-5 | 27-6 | 27-7 | 27-8 | 27-9 |
| smoothness | - | 3 | 4.5 | 4 | 4 | 4.5 | 4 | 4 | 4.5 |

From the results of Table 59, samples 27-2 to 27-9 with the addition of a PLD preparation to PB egg were improved in smoothness compared to sample 27-1 (control). In addition, samples 27-3 to 27-9 obtained by adding a PLD preparation and an auxiliary material (threonine, manganese-containing yeast, glutathione-containing yeast extract, cysteine hydrochloride, glycine, alanine, or cysteine) to PB egg were improved in smoothness compared to sample 27-2 with the addition of a PLD preparation but without addition of the above-mentioned auxiliary material.

### [Experimental Example 28] Confirmation of effect of adding phospholipase D to PB snack (molded)

PB snack (molded) samples 28-1 to 28-9 were prepared according to the sample preparation flow shown in Fig. 20, using the mixing recipes shown in Table 60.

The obtained samples 28-1 to 28-9 were subjected to a sensory evaluation of smoothness by three expert panelists according to the same evaluation criteria as in Experimental Example 12, using sample 28-1 as a control. The results are shown in Table 61.

**[Table 60]**

| <mixing recipe> unit: wt% | | | | | | | | | |
|---|---|---|---|---|---|---|---|---|---|
| raw materials | sample No. | | | | | | | | |
| | 28-1 (control) | 28-2 | 28-3 | 28-4 | 28-5 | 28-6 | 28-7 | 28-8 | 28-9 |
| cornmeal (*1) | 24.00 | 24.00 | 24.00 | 24.00 | 24.00 | 24.00 | 24.00 | 24.00 | 24.00 |
| weak wheat-flour (*2) | 37.00 | 37.00 | 37.00 | 37.00 | 37.00 | 37.00 | 37.00 | 37.00 | 37.00 |
| soybean protein powder (*3) | 6.00 | 6.00 | 6.00 | 6.00 | 6.00 | 6.00 | 6.00 | 6.00 | 6.00 |
| water | 30.00 | 30.00 | 30.00 | 30.00 | 30.00 | 30.00 | 30.00 | 30.00 | 30.00 |
| olive oil | 2.40 | 2.40 | 2.40 | 2.40 | 2.40 | 2.40 | 2.40 | 2.40 | 2.40 |
| salt | 0.60 | 0.60 | 0.60 | 0.60 | 0.60 | 0.60 | 0.60 | 0.60 | 0.60 |
| DENAZYME PMD-P1 (*4) | | 0.030 | 0.030 | 0.030 | 0.030 | 0.030 | 0.030 | 0.030 | 0.030 |
| threonine | | | 0.001 | | | | | | |
| glutathione-containing yeast extract | | | | 0.010 | | | | | |
| manganese-containing yeast | | | | | 0.001 | | | | |
| cysteine hydrochloride | | | | | | 0.001 | | | |
| glycine | | | | | | | 0.001 | | |
| alanine | | | | | | | | 0.001 | |
| cysteine | | | | | | | | | 0.001 |
| total | 100.00 | 100.03 | 100.03 | 100.04 | 100.03 | 100.03 | 100.03 | 100.03 | 100.03 |

| | | | | | | | | | |
|---|---|---|---|---|---|---|---|---|---|
| *1 cornmeal: cornmeal (Tomizawa Shoten), protein content 6.3 wt% *2 weak wheat-flour: Nisshin Flour(R) Cake Wheat Flour (Nisshin Seifun Welna), protein content 8 wt% *3 soybean protein powder: New Fujipro SEH (Fuji Oil), protein content 87 wt% *4 The amount of PLD in samples 28-2 to 28-9 is 175 U when converted to enzyme activity for 1 g of protein in the same sample. | | | | | | | | | |

**[Table 61]**

| | sample No. | | | | | | | | |
|---|---|---|---|---|---|---|---|---|---|
| | 28-1 | 28-2 | 28-3 | 28-4 | 28-5 | 28-6 | 28-7 | 28-8 | 28-9 |
| smoothness | - | 3 | 4 | 3.5 | 3.5 | 4 | 4 | 4 | 4 |

From the results of Table 61, samples 28-2 to 28-9 with the addition of a PLD preparation to PB snack were improved in smoothness compared to sample 28-1 (control). In addition, samples 25-3 to 25-9 obtained by adding a PLD preparation and an auxiliary material (threonine, manganese-containing yeast, glutathione-containing yeast extract, cysteine hydrochloride, glycine, alanine, or cysteine) to PB snack were improved in smoothness compared to sample 28-2 with the addition of a PLD preparation but without addition of the above-mentioned auxiliary material.

### [Experimental Example 29] Confirmation of effect of adding phospholipase D to PB snack (bar)

PB snack (bar) samples 29-1 to 29-9 were prepared according to the sample preparation flow shown in Fig. 21, using the mixing recipes shown in Table 62.

The obtained each sample was subjected to a sensory evaluation of smoothness by three expert panelists according to the same evaluation criteria as in Experimental Example 12, using sample 29-1 as a control. The results are shown in Table 63.

**[Table 62]**

| <mixing recipe> unit: wt% | | | | | | | | | |
|---|---|---|---|---|---|---|---|---|---|
| raw materials | sample No. | | | | | | | | |
| | 29-1 (control) | 29-2 | 29-3 | 29-4 | 29-5 | 29-6 | 29-7 | 29-8 | 29-9 |
| soybean protein (*1) | 23.48 | 23.48 | 23.48 | 23.48 | 23.48 | 23.48 | 23.48 | 23.48 | 23.48 |
| soybean protein powder (*2) | 15.66 | 15.66 | 15.66 | 15.66 | 15.66 | 15.66 | 15.66 | 15.66 | 15.66 |
| butter (*3) | 23.48 | 23.48 | 23.48 | 23.48 | 23.48 | 23.48 | 23.48 | 23.48 | 23.48 |
| salt | 0.20 | 0.20 | 0.20 | 0.20 | 0.20 | 0.20 | 0.20 | 0.20 | 0.20 |
| coconut oil | 23.48 | 23.48 | 23.48 | 23.48 | 23.48 | 23.48 | 23.48 | 23.48 | 23.48 |
| water | 13.70 | 13.70 | 13.70 | 13.70 | 13.70 | 13.70 | 13.70 | 13.70 | 13.70 |
| DENAZYME PMD-P1 (*4) | | 0.030 | 0.030 | 0.030 | 0.030 | 0.030 | 0.030 | 0.030 | 0.030 |
| threonine | | | 0.001 | | | | | | |
| glutathione-containing yeast extract | | | | 0.010 | | | | | |
| manganese-containing yeast | | | | | 0.001 | | | | |
| cysteine hydrochloride | | | | | | 0.001 | | | |
| glycine | | | | | | | 0.001 | | |
| alanine | | | | | | | | 0.001 | |
| cysteine | | | | | | | | | 0.001 |
| total | 100.00 | 100.03 | 100.03 | 100.04 | 100.03 | 100.03 | 100.03 | 100.03 | 100.03 |

| | | | | | | | | | |
|---|---|---|---|---|---|---|---|---|---|
| *1 soybean protein: SOY PROTEIN (SAVAS), protein content 75 wt% *2 soybean protein powder: New Fujipro SHE (Fuji Oil Co., Ltd.), protein content 87 wt% *3 butter: Snow Brand Hokkaido Butter (Snow Brand Megmilk Co., Ltd.), protein content 0.6 wt% *4 The amount of PLD in samples 29-2 to 29-9 is 54 U when converted to enzyme activity for 1 g of protein in the same sample. | | | | | | | | | |

**[Table 63]**

| | sample No. | | | | | | | | |
|---|---|---|---|---|---|---|---|---|---|
| | 29-1 | 29-2 | 29-3 | 29-4 | 29-5 | 29-6 | 29-7 | 29-8 | 29-9 |
| smoothness | - | 3 | 4.5 | 4 | 4 | 4.5 | 4 | 4 | 4.5 |

From the results of Table 63, samples 29-2 to 29-9 with the addition of a PLD preparation to PB snack were improved in smoothness compared to sample 29-1 (control). In addition, samples 25-3 to 25-9 obtained by adding a PLD preparation and an auxiliary material (threonine, manganese-containing yeast, glutathione-containing yeast extract, cysteine hydrochloride, glycine, alanine, or cysteine) to PB snack were improved in smoothness compared to sample 29-2 with the addition of a PLD preparation but without addition of the above-mentioned auxiliary material.

### [Experimental Example 30] Confirmation of effect of adding phospholipase D to Chinese noodles

Frozen Chinese noodle samples 30-1 to 30-6 were prepared according to the sample preparation flow shown in Fig. 22-1, using the mixing recipes shown in Table 64.

The obtained each sample was treated according to the sensory evaluation method shown in Fig. 22-2 and subjected to a sensory evaluation of smoothness, hardness, elasticity, and off-taste or off-flavor by three expert panelists according to the following evaluation criteria. The results are shown in Table 65.

### [Sensory evaluation criteria]

smoothness, hardness, elasticity:
   sample (sample 30-6) not containing egg white (powder) or enzyme preparation as control
5 points: much better than control
4 points: better than control
3 points: slightly better than control
2 points: equivalent to control
1 point: slightly inferior to control
off-taste or off-flavor:
   O: no off-flavor, Δ: slight off-flavor, X: off-flavor

**[Table 64]**

| <mixing recipe> unit: wt% | | | | | |
|---|---|---|---|---|---|
| | raw materials | sample No. | | | |
| | | 30-1 | 30-4 | 30-5 | 30-6 |
| powder fraction | medium-strength flour | 67.0 | 67.0 | 67.0 | 67.0 |
| | processing starch | 26.8 | 26.8 | 26.8 | 26.8 |
| | wheat protein (*1) | 4.0 | 4.0 | 4.0 | 4.0 |
| | alginic acid ester | 0.5 | 0.5 | 0.5 | 0.5 |
| | gardenia dye | 0.1 | 0.1 | 0.1 | 0.1 |
| | egg white (powder) | 1.6 | 0.4 | 0.8 | - |
| | PLD preparation (*2) | - | 0.2 | 0.2 | - |
| | total powder fraction | 100.0 | 98.8 | 98.6 | 98.4 |
| water-soluble fraction (mixing ratio to powder (wt%)) | brine | 1.5 | 1.5 | 1.5 | 1.5 |
| | sodium chloride | 2.0 | 2.0 | 2.0 | 2.0 |
| | tap water | 42.0 | 42.0 | 42.0 | 42.0 |

| | | | | | |
|---|---|---|---|---|---|
| *1 wheat protein (trade name A-glu G) *2 PLD preparation: containing PLD (phospholipase D) 1.5 wt%, dextrin 98.5 wt%; the number of PLD units per 1 g of a PLD preparation is 848 U. The amount of PLD in samples 30-2 to 30-5 is 17.8 U when converted to enzyme activity for 1 g of wheat protein in the same sample. | | | | | |

**[Table 65]**

| | sample No. | | | |
|---|---|---|---|---|
| | 30-1 | 30-4 | 30-5 | 30-6 |
| smoothness | 5 | 4 | 5 | 2 |
| hardness | 5 | 3 | 4 | 2 |
| elasticity | 5 | 4 | 4 | 2 |
| off-taste or off-flavor | ○ | ○ | ○ | ○ |

From the results of Table 65, samples 30-4, 30-5 obtained by adding PLD to Chinese noodles were improved in smoothness, hardness, and elasticity compared to sample 30-6 (control).

### [Experimental Example 31] Confirmation of effect of adding phospholipase D to Chinese noodles

Frozen Chinese noodle samples 31-1 to 31-5 were prepared according to the sample preparation flow shown in Fig. 22-1, using the mixing recipes shown in Table 66.

The obtained each sample was treated according to the sensory evaluation method shown in Fig. 22-2 and subjected to a sensory evaluation of smoothness, hardness, elasticity, and off-taste or off-flavor by three expert panelists according to the following evaluation criteria. The results are shown in Table 67.

### [Sensory evaluation criteria]

smoothness, hardness, elasticity:
   sample (sample 31-5) not containing egg white (powder) or enzyme preparation as control
5 points: much better than control
4 points: better than control
3 points: slightly better than control
2 points: equivalent to control
1 point: slightly inferior to control
off-taste or off-flavor:
   ○: no off-flavor, Δ: slight off-flavor, X: off-flavor

**[Table 66]**

| <mixing recipe> unit: wt% | | | | | | |
|---|---|---|---|---|---|---|
| | raw materials | sample No. | | | | |
| | | 31-1 | 31-2 | 31-3 | 31-4 | 31-5 |
| powder fraction | medium-strength flour | 75.0 | 75.0 | 75.0 | 75.0 | 75.0 |
| | processed starch | 22.0 | 22.0 | 22.0 | 22.0 | 22.0 |
| | wheat protein (*1) | 3.0 | 3.0 | 3.0 | 3.0 | 3.0 |
| | gardenia dye | 0.1 | 0.1 | 0.1 | 0.1 | 0.1 |
| | egg white (powder) | 1.0 | - | - | - | - |
| | PLA1 preparation (*2) | - | 0.2 | - | - | - |
| | PLA2 preparation (*3) | - | - | 0.2 | - | - |
| | PLD preparation (*4) | - | - | - | 0.3 | - |
| | total powder fraction | 101.1 | 100.1 | 100.1 | 100.1 | 100.1 |
| water-soluble fraction (mixing ratio to powder (wt%)) | brine | 1.0 | 1.0 | 1.0 | 1.0 | 1.0 |
| | sodium chloride | 1.0 | 1.0 | 1.0 | 1.0 | 1.0 |
| | tap water | 40.0 | 40.0 | 40.0 | 40.0 | 40.0 |

| | | | | | | |
|---|---|---|---|---|---|---|
| *1 wheat protein (trade name A-glu G) *2 PLA1 preparation: containing PLA1 10%, dextrin 90%. The amount of PLA1 in sample 31-2 is 20.0 U when converted to enzyme activity for 1 g of wheat protein in the same sample. *3 PLA2 preparation: containing PLA2 1.0%, dextrin 99.0%. The amount of PLA2 in sample 31-3 is 19.7 U when converted to enzyme activity for 1 g of wheat protein in the same sample. *4 PLD preparation: containing PLD 1.0%, dextrin 99.0%. The amount of PLD in sample 31-4 is 20.2 U when converted to enzyme activity for 1 g of wheat protein in the same sample. | | | | | | |

**[Table 67]**

| | sample No. | | | | |
|---|---|---|---|---|---|
| | 31-1 | 31-2 | 31-3 | 31-4 | 31-5 |
| smoothness | 5 | 3 | 3 | 4 | 2 |
| hardness | 5 | 2 | 2 | 3 | 2 |
| elasticity | 5 | 3 | 3 | 4 | 2 |
| off-taste or off-flavor | ○ | Δ | Δ | ○ | ○ |
| comments | hardness and elasticity derived from egg white | brittle and breaks easily; off-flavor | brittle and breaks easily; bitter aftertaste | egg white-like hardness and elasticity | no egg white-derived hardness or elasticity |

From the results of Table 67, sample 31-4 with the addition of PLD to Chinese noodles were improved in smoothness, hardness, and elasticity compared to sample 31-5 (control).

### [Experimental Example 32] Confirmation of effect of combined addition of phospholipase D and other enzyme to Chinese noodles

Frozen Chinese noodle samples 32-1 to 32-7 were prepared according to the sample preparation flow shown in Fig. 22-1, using the mixing recipes shown in Table 68.

The obtained samples 32-1 to 32-7 were treated according to the sensory evaluation method shown in Fig. 22-2 and subjected to a sensory evaluation of smoothness, hardness, elasticity, and off-taste or off-flavor by three expert panelists according to the following evaluation criteria. The results are shown in Table 69.

### [Sensory evaluation criteria]

smoothness, hardness, elasticity:
   sample (sample 32-7) containing 0.8% of egg white (powder) and
   not containing enzyme preparation as control
5 points: much better than control
4 points: better than control
3 points: slightly better than control
2 points: equivalent to control
1 point: slightly inferior to control
off-taste or off-flavor:
   O: no off-flavor, Δ: slight off-flavor, X: off-flavor

**[Table 68]**

| <mixing recipe> unit: wt% | | | | | | | | |
|---|---|---|---|---|---|---|---|---|
| | raw materials | sample No. | | | | | | |
| | | 32-1 | 32-2 | 32-3 | 32-4 | 32-5 | 32-6 | 32-7 |
| powder fraction | medium-strength flour | 67.0 | 67.0 | 67.0 | 67.0 | 67.0 | 67.0 | 67.0 |
| | processed starch | 26.8 | 26.8 | 26.8 | 26.8 | 26.8 | 26.8 | 26.8 |
| | wheat protein (*1) | 4.0 | 4.0 | 4.0 | 4.0 | 4.0 | 4.0 | 4.0 |
| | alginic acid ester | 0.5 | 0.5 | 0.5 | 0.5 | 0.5 | 0.5 | 0.5 |
| | gardenia dye | 0.1 | 0.1 | 0.1 | 0.1 | 0.1 | 0.1 | 0.1 |
| | egg white (powder) | 1.6 | 0.8 | 0.8 | 0.8 | 0.8 | 0.8 | 0.8 |
| | PLD preparation (*2) | - | 0.2 | 0.2 | 0.2 | 0.2 | 0.2 | - |
| | TG preparation (*3) | - | - | 0.2 | - | 0.2 | - | - |
| | GO preparation (*4) | - | - | - | 0.2 | 0.2 | - | - |
| | BE preparation (*5) | - | - | - | - | - | 0.5 | - |
| | total powder fraction | 100.0 | 99.4 | 99.2 | 99.2 | 99.2 | 99.2 | 99.2 |
| water-soluble fraction (mixing ratio to powder (wt%)) | brine | 1.5 | 1.5 | 1.5 | 1.5 | 1.5 | 1.5 | 1.5 |
| | sodium chloride | 2.0 | 2.0 | 2.0 | 2.0 | 2.0 | 2.0 | 2.0 |
| | tap water | 42.0 | 42.0 | 42.0 | 42.0 | 42.0 | 42.0 | 42.0 |

| | | | | | | | | |
|---|---|---|---|---|---|---|---|---|
| *1 wheat protein (trade name A-glu G) *2 PLD preparation: containing PLD 1.5%, dextrin 98.5%. The amount of PLD in samples 32-2 to 32-6 is 17.8 U when converted to enzyme activity for 1 g of wheat protein in the same sample. *3 TG preparation: containing TG 2.4%, dextrin 97.6%. The amount of PLD in samples 32-3 and 32-5 is 0.581 U when converted to enzyme activity for 1 g of wheat protein in the same sample. *4 GO preparation: containing GO 1.2%, dextrin 98.8%. The amount of PLD in sample 32-6 is 18.2 U when converted to enzyme activity for 1 g of wheat protein in the same sample. *5 BE preparation: containing BE 0.6%, dextrin 99.4%. The amount of PLD in samples 32-4 and 32-5 is 0.543 U when converted to enzyme activity for 1 g of wheat protein in the same sample. | | | | | | | | |

**[Table 69]**

| | sample No. | | | | | | |
|---|---|---|---|---|---|---|---|
| | 32-1 | 32-2 | 32-3 | 32-4 | 32-5 | 32-6 | 32-7 |
| smoothness | 5 | 5 | 5 | 5 | 5 | 4 | 2 |
| hardness | 5 | 4 | 4 | 4 | 5 | 2 | 2 |
| elasticity | 5 | 4 | 5 | 5 | 5 | 4 | 2 |
| off-taste or off-flavor | ○ | ○ | ○ | ○ | ○ | ○ | ○ |

From the results of Table 69, samples 32-2 to 32-5 with the addition of PLD to Chinese noodles were improved in smoothness, hardness, and elasticity compared to sample 32-7 (control).

### [Industrial Applicability]

According to the present invention, a protein-containing food, in which the protein-derived unpleasant texture is improved, can be provided.

This application is based on patent application No. 2023-022040 filed in Japan, the contents of which are encompassed in full herein.

## Claims

1. A method for producing a modified protein-containing food, comprising treating a food ingredient containing a protein with phospholipase D.

2. The method according to claim 1, wherein the food ingredients comprises at least one selected from the group consisting of the following (A) to (I):
(A) alkali salt
(B) calcium salt or calcium oxide
(C) magnesium salt or magnesium oxide
(D) reducing agent
(E) metal ion
(F) non-polar amino acid or non-polar amino acid salt
(G) uncharged amino acid or uncharged amino acid salt
(H) basic amino acid or basic amino acid salt
(I) acidic amino acid or acidic amino acid salt.

3. The method according to claim 2, wherein the (A) alkali salt is at least one selected from the group consisting of sodium carbonate, trisodium phosphate, tripotassium phosphate, and trisodium citrate.

4. The method according to claim 2, wherein the (B) calcium salt or calcium oxide is at least one selected from the group consisting of calcium chloride, calcinated shell calcium, calcium lactate, and calcium carbonate.

5. The method according to claim 2, wherein the (C) magnesium salt or magnesium oxide is at least one selected from the group consisting of magnesium chloride and magnesium glutamate.

6. The method according to claim 2, wherein the (D) reducing agent is at least one selected from the group consisting of a glutathione-containing yeast extract and a cysteine-containing yeast extract.

7. The method according to claim 2, wherein the (E) metal ion is at least one selected from the group consisting of an iron-containing yeast, a copper-containing yeast, and a manganese-containing yeast.

8. The method according to claim 2, wherein the (F) non-polar amino acid or non-polar amino acid salt is at least one selected from the group consisting of glycine, cystine, alanine, valine, leucine, isoleucine, phenylalanine, proline, and methionine.

9. The method according to claim 2, wherein the (G) uncharged amino acid or uncharged amino acid salt is at least one selected from the group consisting of threonine, serine, glutamine, tyrosine, cysteine, and cysteine hydrochloride.

10. The method according to claim 2, wherein the (H) basic amino acid or basic amino acid salt is at least one selected from the group consisting of arginine, histidine, and lysine hydrochloride.

11. The method according to claim 2, wherein the (I) acidic amino acid or acidic amino acid salt is at least one selected from the group consisting of sodium aspartate and sodium glutamate.

12. The production method according to claim 1, further comprising treating with an enzyme that contributes to the formation of a cross-linked structure.

13. The production method according to any one of claims 1 to 12, wherein the protein-containing food is selected from the group consisting of a processed meat food, a processed rice food, a processed soybean food, a processed wheat food, a processed egg food, a processed milk food, a processed seafood, and a plant-based food.

14. An enzyme preparation for modifying a protein-containing food, which preparation comprises phospholipase D.

15. The enzyme preparation according to claim 14, further comprising an enzyme that contributes to the formation of a cross-linked structure.

16. The enzyme preparation according to claim 14 or 15, wherein the protein-containing food is selected from the group consisting of a processed meat food, a processed rice food, a processed soybean food, a processed wheat food, a processed egg food, a processed milk food, a processed seafood, and a plant-based food.

17. A pickling liquid for meat processing, which comprises phospholipase D, for modifying a protein in a processed meat food.

18. The pickling liquid according to claim 17, further comprising an enzyme that contributes to the formation of a cross-linked structure.

19. A method for modifying a protein-containing food, comprising treating a food ingredient containing a protein with phospholipase D.

20. The modification method according to claim 19, further comprising treating with an enzyme that contributes to the formation of a cross-linked structure.

21. The modification method according to claim 19 or 20, wherein the protein-containing food is selected from the group consisting of a processed meat food, a processed rice food, a processed soybean food, a processed wheat food, a processed egg food, a processed milk food, a processed seafood, and a plant-based food.
